# EUROPEAN PATENT SPECIFICATION

(11) **EP 3 596 211 B1**
(45) Date of publication and mention of the grant of the patent: **02.06.2021**
(21) Application number: 18712780.8
(22) Date of filing: 09.03.2018
(51) Int. Cl.: C12N 9/64, A23K 10/18

(54) **METHODS OF USING AN ARCHAEAL SERINE PROTEASE**
VERWENDUNGEN EINER ARCHAEALEN SERINPROTEASE
PROCÉDÉS D'UTILISATION D'UNE SÉRINE PROTÉASE ARCHAEAL

(30) Priority: 15.03.2017 WO PCT/CN2017/076770
(43) Date of publication of application: 22.01.2020
(73) Proprietor: DuPont Nutrition Biosciences ApS, 1411 Copenhagen K (DK)
(72) Inventor: GU, Xiaogang, Palo Alto, California 94304 (US); ZOU, Zhengzheng, Shanghai 200031 (CN); YU, Shukun, 1411 Copenhagen (DK); SHIPOVSKOV, Stepan, 8250 Egå (DK); VAN BRUSSEL-ZWIJNEN, Marco, 8148 PV Lemele (NL)
(74) Representative: Mewburn Ellis LLP
(86) International application number: PCT/US2018/021726
(87) International publication number: WO 2018/169780

(56) References cited:
- WO-A2-2004/033668
- DATABASE UniParc [Online] 14 May 2014 (2014-05-14), XP002780368, retrieved from Uniprot Database accession no. UPI00042EB195
- DATABASE UniParc [Online] 5 September 2012 (2012-09-05), XP002780369, retrieved from Uniprot Database accession no. UPI0002656C63

## Description

### Field

The field relates to a thermostable archaeal serine protease, and in particular to the identification and characterisation of the protein, termed herein TnaPro1, as a thermophilic protease enzyme suitable for use in feed or grain applications.

### Background

Proteases (also called peptidases or proteinases) are enzymes capable of cleaving peptide bonds. Proteases have evolved multiple times, and different classes of proteases can perform the same reaction by completely different catalytic mechanisms. Proteases can be found in animals, plants, fungi, bacteria, archaea and viruses.

Proteolysis can be achieved by enzymes currently classified into six broad groups: aspartyl proteases, cysteine proteases, serine proteases, threonine proteases, glutamic proteases and metalloproteases.

Serine proteases are a subgroup of carbonyl hydrolases comprising a diverse class of enzymes having a wide range of specificities and biological functions. Notwithstanding this functional diversity, the catalytic machinery of serine proteases has been approached by at least two genetically distinct families of enzymes: 1) the subtilisins; and 2) chymotrypsin-related serine proteases (e.g. trypsin).

These two families of serine proteases have very similar catalytic mechanisms. The tertiary structure of these two enzyme families brings together a conserved catalytic triad of amino acids consisting of serine, histidine and aspartate.

Much research has been conducted on the serine proteases, due largely to their useful in industrial applications. Additional work has been focused on environmental conditions which can adversely impact the functionality of these enzymes in a variety of applications (e.g. exposure to oxidative agents, chelating agents, extremes of temperature and/or pH).

In industry, protease enzymes are, for example, of particular use in food products. Such products may be for humans, but are more commonly for domestic animals, such as farm animals or pets. The addition of proteases to food products or the use of proteases in the production of food products results in partial degradation of proteins in the food products, leading to improved digestibility of the food products. The nutritional value of such food products to the animals which consume them may thus be improved.

Protease enzymes are also commonly used in industrial processes to degrade protein-containing materials in e.g. manufacturing, brewing and the processing of plant materials. For instance, during the extraction of oil from oilseeds or other plant products, proteases may be used in the degradation of cellular material. Proteases can be similarly used in the processing of grain during e.g. brewing.

In such industrial processes, uses, or products, proteases can be exposed to environmental conditions as discussed above (e.g. extremes of temperature and/or pH), which may be detrimental to enzyme stability or activity. The use of proteases in such processes and products is known from the prior art, e.g. US 2010/0081168, which discloses the use of an acid-stable protease in animal feed, and US 8772011, which discloses variants of natural protease with amended temperature activity profiles, which may be used in animal feed and detergents. However, there is a continuing need to develop applications in which proteases can be used under adverse conditions and retain or have improved activity

### Summary

In a first embodiment, there is described a recombinant construct comprising a nucleotide sequence encoding a thermostable polypeptide having serine protease activity, wherein said coding nucleotide sequence is operably linked to at least one regulatory sequence functional in a production host and the nucleotide sequence encodes a polypeptide with the amino acid sequence set forth in SEQ ID NO: 3, or a polypeptide with at least 92% amino acid sequence identity thereto;
and wherein said regulatory sequence is heterologous to the coding nucleotide sequence, or said regulatory sequence and coding sequence are not arranged as found together in nature.

In a second embodiment, the coding nucleotide sequence of the recombinant construct is a nucleotide sequence encoding a polypeptide with the amino acid sequence set forth in SEQ ID NO: 8, or a polypeptide with at least 89% amino acid sequence identity thereto.

In a third embodiment, the coding nucleotide sequence of the recombinant construct described herein elected from the group consisting of:
i) a nucleotide sequence encoding a polypeptide with the amino acid sequence set forth in SEQ ID NO: 2, or a polypeptide with at least 86 % amino acid sequence identity thereto; or
ii) a nucleotide sequence encoding a polypeptide with the amino acid sequence set forth in SEQ ID NO:5 or 14, or a polypeptide with at least 84% amino acid sequence identity thereto.

In a fourth embodiment, the regulatory sequence of recombinant construct of comprises a promoter.

In a fifth embodiment, there is described a vector comprising a recombinant construct as described herein.

In a sixth embodiment, there is described a production host or host cell comprising the recombinant construct described herein.

In a seventh embodiment, the production host or host cell of claim 6 is a cell that can be selected from the group consisting of a bacterial cell, an archaeal cell, a fungal cell or an algal cell.

In an eighth embodiment, there is described a method for producing a thermostable serine protease, said method comprising:
i) transforming a host cell with any of the recombinant constructs described herein; and
ii) culturing the transformed host cell of step (i) under conditions whereby the thermostable serine protease is produced by the method described herein is recovered.

In a ninth embodiment, recovering the thermostable serine protease from the host cell.

In a tenth embodiment, host cell can be selected from the group consisting of bacterial cell, an archaeal cell, a fungal cell or an algal cell.

In an eleventh embodiment, there is described a culture supernatant comprising a thermostable serine protease obtained by any of the methods described herein.

In a twelfth embodiment, there is described a method for hydrolyzing a material derived from corn, said method comprising:
(a) contacting the material obtained from corn with a liquid to form a mash; and
(b) hydrolyzing at least one protein in the mash to form a hydrolysate by contacting the hydrolysate with an enzyme cocktail comprising a thermostable serine protease comprises the amino acid sequence set forth in SEQ ID NO: 3, or an amino acid sequence having at least 92% sequence identity to SEQ ID NO:3 and
(c) optionally, recovering the hydrolysate of obtained in step (b).

In a thirteenth embodiment, there is described an animal feed, feedstuff, feed additive composition or premix comprising at least one polypeptide having serine protease activity and is thermostable, wherein said polypeptide comprises the amino acid sequence set forth in SEQ ID NO: 3, or an amino acid sequence with at least 92% sequence identity thereto, and wherein said animal feed, feedstuff, feed additive composition or premix optionally further comprises (a) at least one direct-fed microbial or (b) at least one other enzyme or (c) at least one direct fed microbial and at least one other enzyme.

In a fourteenth embodiment, the feed additive composition described herein further comprises at least one component selected from the group consisting of a protein, a peptide, sucrose, lactose, sorbitol, glycerol, propylene glycol, sodium chloride, sodium sulfate, sodium acetate, sodium citrate, sodium formate, sodium sorbate, potassium chloride, potassium sulfate, potassium acetate, potassium citrate, potassium formate, potassium acetate, potassium sorbate, magnesium chloride, magnesium sulfate, magnesium acetate, magnesium citrate, magnesium formate, magnesium sorbate, sodium metabisulfite, methyl paraben and propyl paraben.

In a fifteenth embodiment, any of the feed additive compositions described herein can be granulated and comprises particles produced by a process selected from the group consisting of high shear granulation, drum granulation, extrusion, spheronization, fluidized bed agglomeration, fluidized bed spray coating, spray drying, freeze drying, prilling, spray chilling, spinning disk atomization, coacervation, tableting, or any combination of the above processes.

In a sixteenth embodiment, the mean diameter of these particles is between 50 and 2000 microns.

In a seventh embodiment, any of the feed additive compositions described herein is in the form of a liquid, a dry powder, or a granule or a coating, or is in a coated or encapsulated form.

In an eighteenth embodiment, any of the feed additive compositions described herein can be in the form of a liquid which is suitable for spray drying on a feed pellet.

In a nineteenth embodiment wherein the at least one polypeptide having serine protease activity is present in an amount of 1 to 20 g/tonne of any of the animal feed described herein.

In a twentieth embodiment, there is described a method for producing fermentation products from starch-containing material comprising:
(a) liquefying the starch-containing material with an enzyme cocktail comprising a serine protease comprising the amino acid sequence set forth in SEQ ID NO: 3, or an amino acid sequence having at least 92% sequence identity to SEQ ID NO:3;
(b) saccharifying the product of step (a);
(c) fermenting with a suitable organism; and
(d) optionally, recovering the product produced in step (c).

In a twenty first embodiment, steps (b) and (c) are performed simultaneously.

In a twenty second embodiment, the addition of a nitrogen source is eliminated or reduced by at least 50% in the method described herein by using 1-20 g serine protease/MT starch-containing material wherein the serine protease comprises the amino acid sequence set forth in SEQ ID NO: 3, or an amino acid sequence having at least 92% sequence identity to SEQ ID NO:3.

In a twenty-third embodiment, the nitrogen source is urea.

In a twenty-fourth embodiment, when the liquefaction product is ethanol and no acid proteolytic enzyme is needed when using 1-20 g thermostable serine protease/MT starch-containing material wherein the serine protease comprises the amino acid sequence set forth in SEQ ID NO: 3, or an amino acid sequence having at least 92% sequence identity to SEQ ID NO:3.

### Brief Description of the Drawings and Sequences

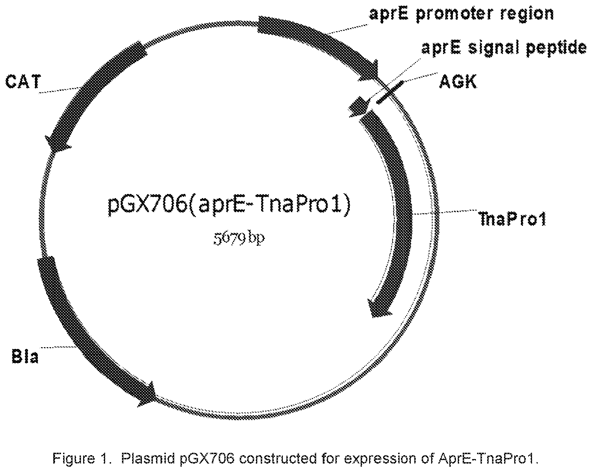

Figure 1 shows the plasmid constructed for expression of AprE-TnaPro1, namely plasmid pGX706, which has the vector backbone of plasmid p2JM 103BBI. The a*prE-TnaPro1* gene is a single open reading frame comprising the *aprE* signal peptide sequence, a 9-nucleotide linker encoding the amino acid sequence Ala-Gly-Lys (AGK) and the *TnaPro1* proenzyme sequence, in that order, as shown. The *aprE-TnaPro1* gene is under the control of the *aprE* promoter (*P_{aprE}*). CAT represents chloramphenicol acetyl transferase, conferring chloramphenicol resistance on bacteria carrying the plasmid. Bla represents β-iactamase.
Figure 2 is a dose response curve showing the proteolytic activity of increasing concentrations of TnaPro1 on the chromogenic substrate AAPF-pNA. Proteolytic activity is represented by the net A₄₁₀ of the solution. The experiments were performed at pH 8.
Figure 3 is a pH profile of purified TnaPro1, showing its proteolytic activity on AAPF-pNA at various pHs from pH 3 to pH 10. Relative activity is given as a percentage value relative to the maximum activity seen, which was observed at pH 9. Error bars represent one standard deviation either side of the mean.
Figure 4 is a temperature profile of purified TnaPro1, showing its proteolytic activity on AAPF-pNA at various temperatures from 30°C to 95°C. Relative activity is given as a percentage value relative to the maximum activity seen, which was observed at 70°C. Error bars represent one standard deviation either side of the mean.
Figure 5 presents the results of a corn soy meal hydrolysis assay using purified TnaPro1 at pH3. Hydrolysis was quantified using the OPA assay and reported as the net A340. Error bars represent one standard deviation either side of the mean.
Figure 6 presents the results of a corn soy meal hydrolysis assay using purified TnaPro1 at pH3. Hydrolysis was quantified using the BCA assay, and reported as the net A562. Error bars represent one standard deviation either side of the mean.
Figure 7 presents the results of a corn soy meal hydrolysis assay equivalent to that presented in Figure 5, with the exception that the assay was performed at pH 6.
Figure 8 presents the results of a corn soy meal hydrolysis assay equivalent to that presented in Figure 6, with the exception that the assay was performed at pH 6.
Figure 9 presents the results of a pepsin stability assay performed on TnaPro1 and ProAct proteases, showing its activity after incubation with pepsin at 37°C for 30 mins. Residual activity after the incubation demonstrates stability of the enzyme when exposed to pepsin. Residual protease activity is presented as a percentage of the original activity, which was determined as the activity of TnaPro1 and ProAct following incubation at 37°C for 30 mins with heat-inactivated pepsin.

The following sequences comply with 37 C.F.R. §§ 1.821-1.825 ("Requirements for Patent Applications Containing Nucleotide Sequences and/or Amino Acid Sequence Disclosures - the Sequence Rules") and are consistent with World Intellectual Property Organization (WIPO) Standard ST.25 (2009) and the sequence listing requirements of the European Patent Convention (EPC) and the Patent Cooperation Treaty (PCT) Rules 5.2 and 49.5(a-bis), and Section 208 and Annex C of the Administrative Instructions. The symbols and format used for nucleotide and amino acid sequence data comply with the rules set forth in 37 C.F.R. § 1.822.

SEQ ID NO: 1 sets forth the nucleotide sequence of *TnaPro1* (NCBI Reference Sequence: NZ_CP007264.1 from 1327825-1329105, complementary).

SEQ ID NO: 2 sets forth the amino acid sequence of the TnaPro1 pre-proenzyme encoded by SEQ ID NO: 1 (GenBank accession number: AHL23118.1).

SEQ ID NO: 3 sets forth the amino acid sequence of the fully processed mature enzyme TnaPro1 (i.e. the amino acid sequence encoded by SEQ ID NO: 6).

SEQ ID NO: 4 sets forth the nucleotide sequence of a synthetic gene encoding an AprE-(AGK)-TnaPro1 protein (i.e. a nucleotide sequence with three additional vector-derived codons (encoding AGK) situated between the 3' end of a nucleotide sequence encoding the *Bacillus subtilis* AprE signal sequence and the 5' end of the nucleotide sequence encoding theTnaPro1 proenzyme).

SEQ ID NO: 5 sets forth the amino acid sequence of the AprE-(AGK)- TnaPro1 protein (i.e. the amino acid sequence encoded by SEQ ID NO: 4).

SEQ ID NO: 6 sets forth the nucleotide sequence of the fully processed mature enzyme TnaPro1.

SEQ ID NO: 7 sets forth the nucleotide sequence of the TnaPro1 proenzyme.

SEQ ID NO: 8 sets forth the amino acid sequence of the TnaPro1 proenzyme (i.e. the amino acid sequence encoded by SEQ ID NO: 7).

SEQ ID NO: 9 sets forth the nucleotide sequence of the TnaPro1 signal sequence.

SEQ ID NO: 10 sets forth the amino acid sequence of the TnaPro1 signal sequence (i.e. the amino acid sequence encoded by SEQ ID NO: 9).

SEQ ID NO: 11 sets forth the nucleotide sequence of the TnaPro1 propeptide fragment (the TnaPro1 pro-domain).

SEQ ID NO: 12 sets forth the amino acid sequence of the TnaPro1 propeptide fragment/pro-domain (i.e. the amino acid sequence encoded by SEQ ID NO: 11).

SEQ ID NO: 13 sets forth the nucleotide sequence of the AprE-TnaPro1 protein (i.e. the TnaPro1 proenzyme with the AprE signal sequence).

SEQ ID NO: 14 sets forth the amino acid sequence of the AprE-TnaPro1 protein (i.e. the amino acid sequence encoded by SEQ ID NO: 13).

SEQ ID NO: 15 sets forth the nucleotide sequence of the AprE signal sequence.

SEQ ID NO: 16 sets forth the amino acid sequence of the AprE signal sequence (i.e. the amino acid sequence encoded by SEQ ID NO: 15).

SEQ ID NO: 17 sets forth the amino acid sequence present in the chromogenic protease substrate AAPF-pNA.

### Detailed Description

In this disclosure, a number of terms and abbreviations are used. The following definitions apply unless specifically stated otherwise:
The articles "a", "an", and "the" preceding an element or component are intended to be non-restrictive regarding the number of instances (i.e. occurrences) of the element or component. Therefore "a", "an", and "the" should be read to include one or at least one, and the singular word form of the element or component also includes the plural unless the number is obviously meant to be singular.

The term "comprising" means the presence of the stated features, integers, steps, or components as referred to in the claims, but that it does not preclude the presence or addition of one or more other features, integers, steps, components or groups thereof. The term "comprising" is intended to include embodiments encompassed by the terms "consisting essentially of' and "consisting of'. Similarly, the term "consisting essentially of' is intended to include embodiments encompassed by the term "consisting of".

Where present, all ranges are inclusive and combinable. For example, when a range of "1 to 5" is recited, the recited range should be construed as including ranges "1 to 4", "1 to 3", "1-2", "1-2 & 4-5", "1-3 & 5", and the like.

As used herein in connection with a numerical value, the term "about" refers to a range of +/- 0.5 of the numerical value, unless the term is otherwise specifically defined in context. For instance, the phrase a "pH value of about 6" refers to pH values of from 5.5 to 6.5, unless the pH value is specifically defined otherwise.

It is intended that every maximum numerical limitation given throughout this Specification includes every lower numerical limitation, as if such lower numerical limitations were expressly written herein. Every minimum numerical limitation given throughout this Specification will include every higher numerical limitation, as if such higher numerical limitations were expressly written herein. Every numerical range given throughout this Specification will include every narrower numerical range that falls within such broader numerical range, as if such narrower numerical ranges were all expressly written herein.

As used herein the terms "protein" and "polypeptide" are interchangeable and refer to a polymer of amino acids joined together by peptide bonds. A "protein" or "polypeptide" comprises a polymeric sequence of amino acid residues. The single and 3-letter code for amino acids as defined in conformity with the IUPAC-IUB Joint Commission on Biochemical Nomenclature (JCBN) is used throughout this disclosure. The single letter X refers to any of the twenty amino acids. It is also understood that a polypeptide may be coded for by more than one nucleotide sequence due to the degeneracy of the genetic code.

The term "protease" means a protein or polypeptide, or a domain of a protein or polypeptide, which has the ability to catalyse cleavage of peptide bonds at one or more positions of a protein backbone. A protease may be obtained from an organism, such as a microorganism (e.g. a fungus, bacterium or archaeon) or a higher organism such as a plant or animal. The terms "protease", "peptidase" and "proteinase" can be used interchangeably. Proteases can be found in all domains of life: eukaryotes (including animals, plants, fungi etc.), bacteria and archaea. Proteases are also encoded by viruses.

Proteases can be split into the two groups "exopeptidases", which cleave the terminal or penultimate peptide bond of a polypeptide chain (and thus release a single amino acid or a dipeptide from a substrate polypeptide chain), and "endopeptidases", which cleave only non-terminal peptide bonds in a polypeptide chain. Exopeptidases are thus able to cleave polypeptides into their individual constituent amino acids; endopeptidases are not able to do so.

Proteolysis (i.e. protein hydrolysis) can be achieved by enzymes currently classified into six broad groups: aspartyl proteases, cysteine proteases, serine proteases, threonine proteases, glutamic proteases, and metalloproteases. The present disclosure is directed to uses of a serine protease. Serine proteases use a catalytic triad comprising a histidine residue, a serine residue and an aspartate residue to catalyse peptide hydrolysis.

Serine protease activity can be identified by a number of methods. Bioinformatic methods are commonly used to identify serine protease sequences. Functional assays may also be used. According to the present disclosure, a protein with serine protease activity may be identified by its ability to cleave the chromogenic substrate N-Suc-Ala-Ala-Pro-Phe- p-nitroanilide (AAPF-pNA). The amino acid sequence present in AAPF-pNA is set forth in SEQ ID NO: 17. AAPF-pNA is a substrate cleaved by the majority of serine proteases, including cathepsin G, subtilisins, chymotrypsin and chymase. Not all serine proteases are able to cleave AAPF-pNA, for instance neutrophil elastase is unable to do so. The serine proteases described herein fall into the family of subtilisin-like proteases, and as such is able to cleave AAPF-pNA.

A polypeptide with serine protease activity as defined herein may thus be identified by its ability to cleave AAPF-pNA. A cleavage reaction may be performed e.g. as follows: the protease may be provided in a solution of 50 mM HEPES, pH 8. AAPF-pNA may be provided in DMSO at a concentration of 10 mM. The AAPF-pNA solution may then be diluted in 50 mM HEPES buffer at a ratio of 17 parts buffer to 1 part AAPF-pNA solution, and the mixture incubated at 40°C for 5 minutes. An appropriate amount of enzyme may then be added to the solution, which may then be incubated at 40°C for 10 minutes. An appropriate amount of enzyme may be a resultant concentration in the reaction mix of e.g. 0.1 ppm.

Enzyme activity may then be measured according to the absorbance of the reaction mixture at 410 nm (i.e. its A₄₁₀ value). A negative control reaction may be performed in which buffer or water without enzyme is added to the dilute AAPF-pNA solution in place of enzyme. A serine protease as described herein may be identified by a statistically significant increase in A₄₁₀ of the reaction mix compared to the negative control. The details of the assay presented above, in terms of buffers, temperatures, times etc., are merely exemplary and may be varied by the skilled person as appropriate. A skilled person is well able to assay the proteolysis activity of a putative protease without particular instruction, and thus while an assay may be used as described above, the protocol may be varied as appropriate. Alternatively, a different assay may be used, in accordance with the knowledge and abilities of the skilled person.

Alternative assays include those based on the release of acid-soluble peptides from casein or haemoglobin, measured as absorbance at 280 nm or calorimetrically using the Folin method, and hydrolysis of the dye-labelled azocasein, measured as absorbance at 440-450 nm. Other exemplary assays involve the solubilization of chromogenic substrates (See e.g., Ward, "Proteinases," in Fogarty (ed.)., Microbial Enzymes and Biotechnology, Applied Science, London, [1983], pp. 251-317). A protease detection assay method using highly labelled fluorescein isothiocyanate (FITC) casein as the substrate, a modified version of the procedure described by Twining [Twining, S.S., (1984) "Fluorescein Isothiocyanate-Labelled Casein Assay for Proteolytic Enzymes" Anal. Biochem. 143:30-34] may also be used.

Other exemplary assays include, but are not limited to: cleavage of casein into trichloroacetic acid-soluble peptides containing tyrosine and tryptophan residues, followed by reaction with Folin-Ciocalteu reagent and colorimetric detection of products at 660 nm, cleavage of internally quenched FRET (Fluorescence Resonance Energy Transfer) peptide substrates followed by detection of product using a fluorometer. Fluorescence Resonance Energy Transfer (FRET) is the non-radiative transfer of energy from an excited fluorophore (or donor) to a suitable quencher (or acceptor) molecule. FRET is used in a variety of applications including the measurement of protease activity with substrates, in which the fluorophore is separated from the quencher by a short peptide sequence containing the enzyme cleavage site. Proteolysis of the peptide results in fluorescence as the fluorophore and quencher are separated. Numerous additional references known to those in the art provide suitable methods (See e.g., Wells et al., Nucleic Acids Res. 11:7911-7925 [1983]; Christianson et al., Anal. Biochem. 223:119 -129 [1994]; and Hsia et al., Anal Biochem. 242:221-227 [1999]).

Preferably, however, the above-described assay using AAPF-pNA is used.

The term "thermostable serine protease" means a serine protease that is heat-stable. The terms "thermostable" and "heat-stable", as used herein, are interchangeable. Furthermore, the terms "thermostable serine protease" and "thermostable polypeptide with serine protease activity" are also interchangeable. According to the present invention a polypeptide may be defined as thermostable if it is not denatured, and retains its activity, at temperatures whereunder most polypeptides would be denatured and lose their function. In particular, a polypeptide may be defined as thermostable if it retains its activity at temperatures of at least 50°C or at least 60°C. Preferably, a polypeptide is considered thermostable only if it retains its activity at a temperature of at least 65°C, 70°C or 80°C or higher. Typically, thermostability may be determined by incubation of the enzyme at an elevated temperature (e.g. of at least 50°C, 60°C, 70°C or higher) for a given time (e.g. 5 mins, 10 mins, 20 mins, 30 mins, 1 hour, 2 hours or 3 hours or more). By this point, non-thermostable enzymes would be expected to be denatured, so a polypeptide which retains its activity following this incubation may be considered thermostable. To be considered thermostable, as defined herein, a polypeptide must retain its activity following incubation at a temperature of at least 50°C or at least 60°C, preferably at least 70°C, 75°C or 80°C, for a period of time of at least 5 mins, 10 mins, 20 mins or 30 mins, preferably at least 1 hour. It is not necessary that the polypeptide retain its activity following incubation at a temperature of at least 100°C or 110°C.

For a polypeptide to "retain its activity" as defined herein, it must retain at least 50 % of its activity, preferably at least 60 %, 70 %, 80 % or 90 % of its activity following its above-described incubation. Activity of the polypeptide following its incubation is measured against a baseline level of activity to calculate the proportion of activity retained. This baseline activity may correspond to the level of activity of the polypeptide at a temperature identified as being optimal for its activity (i.e. a temperature at which the activity of the polypeptide is highest). Such an optimal temperature may be for example 50°C, 60°C, 65°C, 70°C, 75°C, or 80°C or more, and may be identified by the skilled person. The skilled person will be able to identify suitable conditions for taking of the baseline reading without undue effort.

Herein, the relevant activity of the polypeptide is serine protease activity. Serine protease activity of a polypeptide as defined herein may be measured as detailed above, using a proteolysis assay with AAPF-pNA as a substrate, or using any other appropriate assay known in the art.

The terms "animal" and "subject" are used interchangeably herein. The term "animal" includes human and non-human animals. An animal, as referred to herein, may be a non-ruminant (such as a human) or a ruminant animal (such as a cow, sheep or goat). In a particular embodiment, the animal is a non-ruminant animal, such as a monogastric animal or a horse. Examples of monogastric animals include, but are not limited to, pigs and swine, such as piglets, growing pigs and sows; poultry such as turkeys, ducks, chickens, broiler chicks and layers (i.e. birds bred to lay eggs); fish such as salmon, trout, tilapia, catfish and carps; and crustaceans such as shrimps and prawns. Examples of ruminant animals according to the invention include, but are not limited to, cattle, calves, goats, sheep, giraffes, bison, moose, elk, yaks, water buffalo, deer, camels, alpacas, llamas, antelopes, pronghorns and nilgai.

The term "pathogen" as used herein means any causative agent of disease. Such causative agents can include, but are not limited to, bacterial, viral, fungal causative agents and the like.

A "feed" and a "food", as used herein, mean any natural or artificial diet, meal or the like or components of such meals intended or suitable for being eaten, consumed, taken in or digested by a non-human animal or a human being, respectively. The term "feed" may be used with reference to products that are fed to animals in the rearing of livestock. The terms "feed" and "animal feed" are used interchangeably.

The term "food product" also covers any component or ingredient of a food or feed, any premix or suchlike upon which a food or feed is based, and any supplement, additive or suchlike which is added to a food or feed prior to its consumption by an animal. Thus, a food product may be understood to cover any product which is eaten or consumed by an animal, both products eaten or consumed as a stand-alone food or feed and products eaten or consumed as a part of a more complex food or feed.

The terms "liquefy", "liquefaction", "liquefact" and variations thereof refer to the process or product of converting starch to soluble dextrinized substrates (e.g., smaller polysaccharides).

"Liquefact" can be referred to as "mash" or may also be called a soluble starch substrate or a liquefied substrate. In some cases, it may be a whole ground grain slurry containing a thermostable alpha amylase that has been subjected to high temperature liquefaction resulting in a soluble substrate for saccharification and fermentation or simultaneous saccharification and fermentation (SSF). High temperature is a temperature higher than the gelatinization temperature of the grain polysaccharides.

The term "milled" is used herein to refer to plant material that has been reduced in size, such as by grinding, crushing, fractionating or any other means of particle size reduction. Milling includes dry or wet milling. "Dry milling" refers to the milling of whole dry grain. "Wet milling" refers to a process whereby grain is first soaked (steeped) in water to soften the grain.

The term "hydrolysis" refers to a chemical reaction or process in which a chemical compound is broken down by reaction with water. Starch digesting enzymes hydrolyze starch into smaller units, i.e., smaller polysaccharides.

The term "lignocellulosic" refers to a composition comprising both lignin and cellulose. It may also contain hemicellulose.

The term "lignocellulosic biomass" refers to any lignocellulosic material
and includes materials comprising cellulose, hemicellulose, lignin, starch, oligosaccharides and/or monosaccharides. Biomass can also comprise additional components, such as protein and/or lipid. Biomass can be derived from a single source, or biomass can comprise a mixture derived from more than one source; 25 for example, biomass could comprise a mixture of corn cobs and corn stover, or a mixture of grass and leaves. Lignocellulosic biomass includes, but is not limited to, bioenergy crops, agricultural residues, municipal solid waste, industrial solid waste, sludge from paper manufacture, yard waste, wood and forestry waste. Examples of biomass include, but are not limited to, corn cobs, crop residues 30 such as corn husks, corn stover, grasses (including Miscanthus), wheat straw, barley straw, hay, rice straw, switchgrass, waste paper, sugar cane bagasse, sorghum material, soybean plant material, components obtained from milling of grains or from using grains in production processes (such as DDGS: dried distillers grains with solubles), trees, branches, roots, leaves, wood chips, sawdust, shrubs and bushes, vegetables, fruits, flowers, empty palm fruit bunch, and energy cane. The term "energy cane" refers to sugar cane that is bred for use in energy production. It is selected for a higher percentage of fiber than sugar.

The term "pretreated lignocellulosic biomass" refers to biomass which has been subjected to a physical, thermal and/or chemical treatment prior to saccharification. The term "ammonia pretreated lignocellulosic biomass" refers to biomass which has been subjected at least to a pretreatment process employing ammonia. In one embodiment ammonia pretreatment is a low ammonia pretreatment where biomass is contacted with an aqueous solution comprising ammonia to form a biomass-aqueous ammonia mixture where the ammonia concentration is sufficient to maintain alkaline pH of the biomass aqueous ammonia mixture but is less than about 12 weight percent relative to dry weight of biomass, and where dry weight of biomass is at least about 15 weight percent solids relative to the weight of the biomass-aqueous ammonia mixture, as disclosed in the U.S. Patent No. 7,932,063.

The term "lignocellulosic biomass hydrolysate" refers to the product
resulting from saccharification of lignocellulosic biomass. The biomass may also be pretreated or pre-processed prior to saccharification. The terms "saccharification" and "saccharifying" refer to the process of converting polysaccharides to dextrose monomers using enzymes. Saccharification can refer to the conversion of polysaccharides in a liquefact. Saccharification products are, for example, glucose and other small (low molecular weight) oligosaccharides such as disaccharides and trisaccharides.

The term "SSF" refers to simultaneous saccharification and fermentation.

The term "enzyme cocktail" refers to a mixture or combination of at least two different enzymes, which make it more efficient and effective for any catalytic reaction.

The terms "fermentation" or "fermenting" refer to the process of transforming sugars from reduced plant material to produce as fermentation product.

The term "fermentation product" means a product produced by a process including a fermentation step using a fermenting organism.

The term "by-product" refers to a secondary product derived from a manufacturing process or chemical reaction. It is not the primary product or service being produced.

The term "isolated" means a substance in a form or environment that does not occur in nature. Non-limiting examples of isolated substances include (1) any non- naturally occurring substance, (2) any substance including, but not limited to, any host cell, enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated. The terms "isolated nucleic acid molecule", "isolated polynucleotide", and "isolated nucleic acid fragment" will be used interchangeably and refer to a polymer of RNA or DNA that is single- or double-stranded, optionally containing synthetic, non-natural or altered nucleotide bases. An isolated nucleic acid molecule in the form of a polymer of DNA may be comprised of one or more segments of cDNA, genomic DNA or synthetic DNA.

The term "purified" as applied to nucleic acids or polypeptides generally denotes a nucleic acid or polypeptide that is essentially free from other components as determined by analytical techniques well known in the art (e.g., a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (e.g., percent by weight on a molar basis). In a related sense, a composition is enriched for a molecule when there is a substantial increase in the concentration of the molecule after application of a purification or enrichment technique. The term "enriched" refers to a compound, polypeptide, cell, nucleic acid, amino acid, or other specified material or component that is present in a composition at a relative or absolute concentration that is higher than a starting composition.

The term "direct-fed microbial" (DFM) as used herein is source of live (viable), naturally-occurring microorganisms. In particular, a DFM may be added to a food or feed as a source of live, naturally-occurring microorganisms for the animal for which the food or feed is intended. A DFM can comprise one or more of such live naturally-occurring microorganisms, in particular bacterial or fungal strains. Categories of DFMs include bacteria of the genus *Bacillus,* lactic acid bacteria and yeasts. Thus, the term DFM encompasses one or more of the following: direct fed bacteria, direct fed yeast and combinations thereof.

DFMs may in particular comprise bacilli in the form of spores. Bacilli form spores under certain environmental conditions, usually stress caused by e.g. lack of nutrients. Such spores are metabolically inactive and able to withstand extreme environmental conditions such as intense heat and high and low pHs. When ingested by an animal, such spores germinate into active vegetative cells. *Bacillus* spores can be used in meal and pelleted food products. Such spores should of course be of non-pathogenic species and strains of *Bacillus.*

Lactic acid bacteria are Gram-positive bacteria of a number of strains and genera which produce lactic acid as a major end-product of carbohydrate metabolism. The presence of lactic acid bacteria in food products is believed to be antagonistic to the growth of pathogenic bacterial species. Lactic acid bacteria are able to grow at low pH, but tend to be heat-sensitive, meaning they may be unsuitable for inclusion in pelleted food products. Types of lactic acid bacteria include species of the genera *Bifidobacterium, Lactobacillus, Lactococcus* and *Streptococcus.*

The term "prebiotic" means a non-digestible food ingredient that beneficially affects the host by selectively stimulating the growth and/or the activity of one or a limited number of beneficial bacteria in an animal which consumes the prebiotic. In particular, a prebiotic may selectively stimulate the growth and/or activity of beneficial species of bacteria in the gut microflora of an animal which consumes the prebiotic. By beneficial bacteria is meant species of bacteria, the growth of which in an animal, particularly in the intestinal microflora of an animal, is beneficial to that animal. In particular, such bacteria may be beneficial to the function and/or health of the animal's digestive system (e.g. preventing dysbiosis or colonisation of the gut with pathogenic species), to the animal's metabolism (e.g. they may promote healthy growth of the animal) and/or to the function of the animal's immune system. In particular, prebiotics may increase the number of activity of *Bifidobacteria* and lactic acid bacteria in the gut of an animal which consumes a prebiotic.

The term "probiotic culture" as used herein refer to a culture of live microorganisms (including bacteria or yeasts for example) which, when for example ingested or locally applied in sufficient numbers, beneficially affects the recipient organism, e.g. by conferring one or more demonstrable health benefits on the host organism. Probiotics may improve the microbial balance in one or more mucosal surfaces. For example, the mucosal surface may be the intestine, the urinary tract, the respiratory tract or the skin. The term "probiotic" as used herein also encompasses live microorganisms that can stimulate the beneficial branches of the immune system and at the same time decrease the inflammatory reactions in a mucosal surface, for example the gut. Whilst there are no lower or upper limits for probiotic intake, it has been suggested that at least 10⁶-10¹², preferably at least 10⁶-10¹⁰, preferably 10⁸-10⁹, colony-forming units (cfu) as a daily dose will be effective to achieve the beneficial health effects in a subject. Probiotics may particularly comprise bacteria of the genus *Bifidobacterium* and lactic acid bacteria, such as those of the genus *Lactobacillus.* Yeasts of the genus *Saccharomyces* may also have beneficial effects when consumed by an animal and thus be comprised in a probiotic.

The term "colony forming unit" (CFU) is a measure of viable number, in which a colony represents an aggregate of cells derived from a single progenitor cell.

A proprotein (pro-protein) or proenzyme (pro-enzyme) is an immature form of a protein or enzyme. A pro-protein does not comprise a signal peptide (either being encoded without a signal peptide or being the polypeptide sequence remaining following cleavage of a signal peptide). A pro-protein or pro-enzyme comprises an amino acid sequence at one or other terminus (i.e. its N-terminus or C-terminus) which is necessary for the proper folding and/or secretion of the protein or is used to maintain a protein or enzyme in inactive form or suchlike. This amino acid sequence is referred to herein as a proenzyme. A pro-protein or pro-enzyme is converted into its mature form by cleavage which separates the mature, active protein sequence from its pro-peptide fragment (pro-domain).. Proteases are often expressed as pro-enzymes, which are only activated when converted into their mature form by cleavage to remove the pro-peptide.

The terms "signal sequence" and "signal peptide" refer to a sequence of amino acid residues that may participate in the secretion or direct transport of the mature or precursor form of a protein, i.e. a sequence of amino acid residues which marks a polypeptide for secretion. The signal sequence is typically located N-terminal to the precursor or mature protein sequence. The signal sequence may be endogenous (a signal sequence natively encoded by the gene for the precursor protein) or exogenous (a signal sequence natively encoded as part of a different gene sequence, or an artificial signal sequence). A signal sequence is normally absent from the mature protein. A signal sequence is typically cleaved from the protein by a signal peptidase after the protein is transported. A pre-proenzyme or precursor polypeptide is a translation product that consists of a signal sequence, pro-peptide and mature sequences.

A protein with a signal sequence is known as a pre-proprotein or pre-proenzyme. In some instances, cleavage of the signal sequence can produce the mature form of the protein. A protein synthesised in the form of a pro-protein with a signal sequence is known as a pre-proprotein (or in the case of an enzyme a pre-proenzyme). Cleavage of the signal sequence from a pre-proprotein leaves a pro-protein, which can then subsequently be further processed to yield the mature protein.

The "mature" form of a protein or polypeptide, as used herein, is the functional form of a protein, polypeptide, or enzyme from which a signal sequence and/or pro-peptide fragment has been cleaved. A mature protein does not contain a signal sequence or pro-peptide fragment. Pre-proteins and pre-pro-proteins (i.e. any polypeptide comprising a signal sequence or a pro-peptide fragment) may generically be referred to as precursor proteins.

In the case of the polypeptides of the present invention, the amino acid sequence of the mature TnaPro1 enzyme (i.e. the active form of the enzyme without the signal ("pre") sequence, and without the pro-domain ("pro") sequence) is set forth in SEQ ID NO: 3; the amino acid sequence of the "pro-protein" (i.e. without the signal sequence, but with the "pro" sequence) is set forth in SEQ ID NO: 8, the amino acid sequence of the full-length precursor TnaPro1 protein expressed in this evaluation is set forth in SEQ ID NO: 5, while the amino acid sequence of the full-length precursor TnaPro1 protein identified in *Thermococcus nautili* bacteria is set forth in SEQ ID NO: 2.

The term "wild-type" in reference to an amino acid sequence or nucleic acid sequence indicates that the amino acid sequence or nucleic acid sequence is a native or naturally-occurring sequence. As used herein, the term "naturally-occurring" refers to anything (e.g. proteins, amino acids, or nucleic acid sequences) that is found in nature. Conversely, the term "non-naturally occurring" (or "non-native") refers to anything that is not found in nature (e.g., recombinant nucleic acids and protein sequences produced in the laboratory or modification of the wild-type sequence).

The terms "derived from" and "obtained from" refer to not only a protein produced or producible by a strain of the organism in question, but also a protein encoded by a DNA sequence isolated from such strain and produced in a host organism containing such DNA sequence. Additionally, the term refers to a protein which is encoded by a DNA sequence of synthetic and/or cDNA origin and which has the identifying characteristics of the protein in question.

It would be recognised by one of ordinary skill in the art that modifications of amino acid sequences disclosed herein can be made while retaining the function associated with the disclosed amino acid sequences. For example, it is well known in the art that alterations in a gene which result in the production of a chemically equivalent amino acid at a given site, but do not affect the functional properties of the encoded protein, are common. For example, any particular amino acid in an amino acid sequence disclosed herein may be substituted for another functionally-equivalent amino acid. For the purposes of this disclosure, functionally-equivalent amino acids refer to amino acids belonging to the same one of the following five groups:
1. Small aliphatic, nonpolar or slightly polar residues: Ala, Ser, Thr, Pro, Gly;
2. Polar, negatively charged residues and their amides: Asp, Asn, Glu, Gln;
3. Polar, positively charged residues: His, Arg, Lys;
4. Large aliphatic, nonpolar residues: Met, Leu, Ile, Val, Cys; and
5. Large aromatic residues: Phe, Tyr, and Trp.

In many cases, nucleotide changes which result in alteration of the N-terminal and C-terminal portions of the protein molecule would also not be expected to alter the activity of the protein.

The term "codon-optimised" refers to genes or coding regions of nucleic acid molecules for transformation of various hosts, in which the codons present in the gene or coding region of the nucleic acid molecule are altered to reflect the typical codon usage of the host organism without altering the sequence of the polypeptide for which the DNA codes. As will be recognised by one of ordinary skill in the art, such codon alterations are possible due to the degenerate nature of the nucleic acid code. Such modification of a nucleic acid sequence can be easily achieved using techniques common in the art.

The term "gene" refers to a nucleic acid molecule that expresses a specific protein, including regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different from that found in nature. "Endogenous gene" refers to a native gene in its natural location in the genome of an organism. A "foreign" gene refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

From this definition it will be appreciated that the recombinant construct as described herein can be seen to comprise, or to represent, a chimeric gene. In particular the chimeric gene, (which can also be defined as a chimeric nucleotide sequence) comprises a coding nucleotide sequence encoding the thermostable serine protease linked (more particularly operably linked) to a regulatory sequence with which, or in a manner in which, it does not occur in nature.

The term "coding sequence" refers to a nucleotide sequence which codes for a specific amino acid sequence. "Suitable regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, RNA processing site, effector binding sites, and stem-loop structures.

The term "operably linked" refers to the association of nucleic acid sequences on a single nucleic acid molecule so that the function of one is affected by the other. For example, a promoter is operably linked with a coding sequence when it is capable of affecting the expression of that coding sequence, i.e. the coding sequence is under the transcriptional control of the promoter. Coding sequences can be operably linked to regulatory sequences in sense or antisense orientation.

The terms "regulatory sequence" or "control sequence" are used interchangeably herein and refer to a segment of a nucleotide sequence which is capable of increasing or decreasing expression of specific genes within an organism. Examples of regulatory sequences include, but are not limited to, promoters, signal sequences, operators and the like. As noted above, regulatory sequences can be operably linked in sense or antisense orientation to the coding sequence of interest.

"Promoter" or "promoter sequences" refer to DNA sequences that define where transcription of a gene by RNA polymerase begins. Promoter sequences are typically located directly upstream of or at the 5' end of the transcription initiation site. Promoters may be derived in their entirety from a native or naturally occurring sequence, or be composed of different elements derived from different promoters found in nature, or even comprise synthetic DNA segments. It is understood by those skilled in the art that different promoters may direct the expression of a gene in different tissues or cell type or at different stages of development, or in response to different environmental or physiological conditions ("inducible promoters").

"3' non-coding sequences" refer to DNA sequences located downstream of a coding sequence and include sequences encoding regulatory signals capable of affecting mRNA processing or gene expression, such as termination of transcription.

The term "transformation" as used herein refers to the transfer or introduction of a nucleic acid molecule into a host organism. The nucleic acid molecule may be introduced as a linear or circular form of DNA. The nucleic acid molecule may be a plasmid that replicates autonomously, or it may integrate into the genome of a production host. Production hosts containing the transformed nucleic acid are referred to as "transformed" or "recombinant" or "transgenic" organisms or "transformants".

The term "recombinant" as used herein refers to an artificial combination of two or more otherwise separate nucleic acid sequences. The two or more nucleic acid sequences may be assembled together by e.g. chemical synthesis or the manipulation of isolated nucleic acids or segments of nucleic acids using genetic engineering techniques. The two or more nucleic acid sequences may be native sequences, artificial sequences or a combination of the two. DNA which has been artificially manipulated, e.g. to re-order sequences from within a molecule, to alter the sequence of a molecule, to combine sequences from two or more different molecules, to combine the sequences of two or more different molecules, to remove one or more sequences from a molecule or any other sequence manipulation, or any combination of the above, is a recombinant DNA molecule. Thus, a recombinant DNA sequence, which includes the recombinant construct of the invention, has a sequence not found in nature. An organism into which a recombinant DNA molecule (or recombinant construct) has been introduced is a recombinant organism. The terms "recombinant", "transgenic", "transformed", "engineered" or "modified for exogenous gene expression" are used interchangeably herein with respect to organisms.

The terms "recombinant construct", "expression construct", "recombinant expression construct" and "expression cassette" are used interchangeably herein. A recombinant construct comprises an artificial combination of nucleic acid fragments, e.g. regulatory and coding sequences, that are not all found together in nature. For example, a construct may comprise regulatory sequences and coding sequences that are derived from different sources, or regulatory sequences and coding sequences derived from the same source, but arranged in a manner different to that found in nature. Such a construct may be used by itself or may be used in conjunction with a vector. If a vector is used, then the choice of vector is dependent upon the method that will be used to transform host cells as is well known to those skilled in the art, and the purpose of this transformation. For example, a plasmid vector can be used. The skilled artisan is well aware of the genetic elements that must be present on the vector in order to successfully transform, select and propagate host cells. The skilled artisan will also recognize that different independent transformation events may result in different levels and patterns of expression (Jones et al., (1985) EMBO J 4:2411-2418; De Almeida et al., (1989) Mol Gen Genetics 218:78-86), and thus that multiple events are typically screened in order to obtain lines displaying the desired expression level and pattern. Such screening may be accomplished using standard molecular biological, biochemical, and other assays including Southern analysis of DNA, Northern analysis of mRNA expression, PCR, real time quantitative PCR (qPCR), reverse transcription PCR (RT-PCR), immunoblotting analysis of protein expression, enzyme or activity assays, and/or phenotypic analysis.

The term "vector" refers to a DNA molecule used as a vehicle to introduce foreign genetic material into a cell. A vector may for instance be a cloning vector or an expression vector. Vectors include plasmids, autonomously-replicating sequences, transposable elements, phagemids, cosmids, artificial chromosomes such as a yeast artificial chromosome (YAC), a bacterial artificial chromosome (BAC), or a PI - derived artificial chromosome (PAC), bacteriophages such as lambda phage or MI 3 phage, and animal viruses. A vector may be a genome-integrating sequence or may be extra-chromosomally maintained in a cell. It may be linear or circular, and comprise or consist of single- or double-stranded DNA or RNA.

A "transformation cassette" refers to a specific vector containing a gene and having elements in addition to the gene that facilitate transformation of a particular host cell. The terms "expression cassette" and "expression vector" are used interchangeably herein and refer to a specific vector containing a gene and having elements in addition to the gene that allow for expression of that gene in a host.

An expression vector can be one of any number of vectors or cassettes useful for the transformation of suitable production hosts known in the art. Typically, the vector or cassette will include sequences directing transcription and translation of the relevant gene, a selectable marker, and sequences allowing autonomous replication or chromosomal integration. Suitable vectors generally include a region 5' of the gene which harbours transcriptional initiation controls and a region 3' of the DNA fragment which controls transcriptional termination. Both control regions can be derived from genes homologous to those of the production host cell and/or genes native to the production host, although such control regions need not be so derived.

Possible initiation control regions or promoters that can be included in the expression vector are numerous and familiar to those skilled in the art. Virtually any promoter capable of driving gene expression is suitable, including but not limited to, *CYC1, HIS3, GAL1, GAL10, ADH1, PGK, PHO5, GAPDH, ADC1, TRP1, URA3, LEU2, ENO, TPI* (useful for expression in *Saccharomyces*); *AOX1* (useful for expression in *Pichia*); and *lac, araB, tet, trp, IP_{L}*, *IP_{R}*, *T7*, *tac,* and *trc* (useful for expression in *Escherichia coli*) as well as the *amy, apr, npr* promoters and various phage promoters useful for expression in *Bacillus.* The promoter should be suitable for driving expression of the relevant gene in the production host to be used. The promoter may be a constitutive or inducible promoter. A "constitutive promoter" is a promoter that is active under most environmental and developmental conditions. An "inducible" or "repressible" promoter is a promoter that is active under environmental or developmental regulation. In some embodiments, promoters are inducible or repressible due to changes in environmental factors including but not limited to, carbon, nitrogen or other nutrient availability, temperature, pH, osmolarity, the presence of heavy metal(s), the concentration of inhibitor(s), stress, or a combination of the foregoing, as is known in the art. In some embodiments, the inducible or repressible promoters are inducible or repressible by metabolic factors, such as the level of certain carbon sources, the level of certain energy sources, the level of certain catabolites, or a combination of the foregoing as is known in the art. In one embodiment, the promoter is one that is native to the host cell. For example, when *T. reesei* is the host, the promoter is a native *T. reesei* promoter such as the *cbh1* promoter which is deposited in GenBank under Accession Number D86235.

Suitable non-limiting examples of promoters include *cbh1, cbh2, egl1, egl2, egl3, egl4, egl5, xyn1,* and *xyn2,* repressible acid phosphatase gene (phoA) promoter of P. *chrysogenus* (see e.g., Graessle et al., (1997) Appl. Environ. Microbiol., 63 :753-756), glucose repressible PCK1 promoter (see e.g., Leuker et al., (1997), Gene, 192:235-240), maltose inducible, glucose-repressible MET3 promoter (see Liu et al., (2006), Eukary. Cell, 5:638-649), pKi promoter and cpc1 promoter. Other examples of useful promoters include promoters from *A. awamori* and *A. niger* glucoamylase genes (see e.g., Nunberg et al., (1984) Mol. Cell Biol. 15 4:2306-2315 and Boel et al., (1984) EMBO J. 3:1581-1585). Also, the promoters of the *T. reesei xln1* gene may be useful (see e.g., EPA 137280AI).

DNA fragments which control transcriptional termination may also be derived from various genes native to a preferred production host cell. In certain embodiments, the inclusion of a termination control region is optional. In certain embodiments, the expression vector includes a termination control region derived from the preferred host cell.

The terms "production host", "host" and "host cell" are used interchangeably herein and refer to prokaryotic cells such as bacterial cells, into which a recombinant construct can be stably or transiently introduced or transformed in order to express a gene. The "host cell" may be any suitable eukaryotic or prokaryotic host cell, but typically will be a microbial host cell e.g. a prokaryotic host cell or a fungal (e.g. yeast) cell, or cell line.

"Stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into a host organism, including into the nucleus or a DNA-containing organelle of a host organism, resulting in gene expression without integration or stable inheritance.

The expression vector can be introduced into the host cell, particularly the cells of microbial hosts. The host cells can be microbial hosts found within the fungal or bacterial families and which grow over a wide range of temperature, pH values, and solvent tolerances. For example, it is contemplated that any of bacteria, algae, and fungi such as filamentous fungi and yeast may suitably host the expression vector.

Following introduction of the expression vector into the host cell, the polypeptide may be expressed so that it resides intracellularly, extracellularly, or a combination of both inside and outside the cell. If the protein is a transmembrane protein, it may reside within the cell membrane. Extracellular expression renders recovery of the desired protein from a culture of the production host more facile than methods for recovery of protein produced by intracellular expression. Protein which is expressed such that it resides within the cell membrane is challenging to recover.

The term "expression", as used herein, refers to the production of an end-product of a gene (e.g. a functional RNA molecule or a protein) in either precursor or mature form. Thus, expression of a protein-encoding gene refers to transcription of the gene and translation of the resultant mRNA to yield a protein.

The terms "percent (%) identity" and "percent (%) sequence identity" refer to a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the number of matching nucleotides or amino acids between strings of such sequences. "Identity" and "similarity" can be readily calculated by known methods, including but not limited to those described in: Computational Molecular Biology (Lesk, A. M., ed.) Oxford University Press, NY (1988); Biocomputing: Informatics and Genome Projects (Smith, D. W., ed.) Academic Press, NY (1993); Computer Analysis of Sequence Data, Part I (Griffin, A. M., and Griffin, H. G., eds.) Humana Press, NJ (1994); Sequence Analysis in Molecular Biology (von Heinje, G., ed.) Academic Press (1987); and Sequence Analysis Primer (Gribskov, M. and Devereux, J., eds.) Stockton Press, NY (1991). Methods to determine identity and similarity are codified in publicly available computer programs.

As used herein, "% sequence identity" or "percent sequence identity" refer to protein or nucleic acid sequence identity. Percent identity may be determined using standard techniques known in the art. Useful algorithms include the BLAST algorithms (See, Altschul et al., J Mol Biol, 215:403-410, 1990; and Karlin and Altschul, Proc Natl Acad Sci USA, 90:5873-5787, 1993). The BLAST program uses several search parameters, most of which are set to the default values. The NCBI BLAST algorithm finds the most relevant sequences in terms of biological similarity but is not recommended for query sequences of less than 20 residues (Altschul et al., Nucleic Acids Res, 25:3389-3402, 1997; and Schaffer et al., Nucleic Acids Res, 29:2994-3005, 2001). Exemplary default BLAST parameters for a nucleic acid sequence searches include: Neighboring words threshold = 11; E-value cutoff = 10; Scoring Matrix = NUC.3.1 (match = 1, mismatch = -3); Gap Opening = 5; and Gap Extension = 2. Exemplary default BLAST parameters for amino acid sequence searches include: Word size = 3; E-value cutoff = 10; Scoring Matrix = BLOSUM62; Gap Opening = 11; and Gap extension = 1. A percent (%) amino acid sequence identity value is determined by the number of matching identical residues divided by the total number of residues of the "reference" sequence including any gaps created by the program for optimal/maximum alignment. BLAST algorithms refer to the "reference" sequence as the "query" sequence.

As used herein, "homologous proteins" or "homologous proteases" refer to proteins that have distinct similarity in primary, secondary, and/or tertiary structure. Protein homology can refer to the similarity in linear amino acid sequence when proteins are aligned. Homologous search of protein sequences can be done using BLASTP and PSI-BLAST from NCBI BLAST with threshold (E-value cut-off) at 0.001. (Altschul SF, Madde TL, Shaffer AA, Zhang J, Zhang Z, Miller W, Lipman DJ. Gapped BLAST and PSI BLAST a new generation of protein database search programs. Nucleic Acids Res 1997 Set 1;25(17):3389-402). Using this information, proteins sequences can be grouped. A phylogenetic tree can be built using the amino acid sequences.

Sequence alignments and percent identity calculations may be performed using the Megalign program of the LASERGENE bioinformatics computing suite (DNASTAR Inc., Madison, WI), the AlignX program of Vector NTI v. 7.0 (Informax, Inc., Bethesda, MD), or the EMBOSS Open Software Suite (EMBL-EBI; Rice et al., Trends in Genetics 16, (6):276-277 (2000)). Multiple alignment of the sequences can be performed using the CLUSTAL method (such as CLUSTALW; for example, version 1.83) of alignment (Higgins and Sharp, CABIOS, 5:151-153 (1989); Higgins et al., Nucleic Acids Res. 22:4673-4680 (1994); and Chenna et al., Nucleic Acids Res 31 (13):3497-500 (2003)), available from the European Molecular Biology Laboratory via the European Bioinformatics Institute) with the default parameters. Suitable parameters for CLUSTALW protein alignments include GAP Existence penalty=15, GAP extension =0.2, matrix = Gonnet (e.g., Gonnet250), protein ENDGAP = -1, protein GAPDIST=4, and KTUPLE=1. In one embodiment, a fast or slow alignment is used with the default settings where a slow alignment. Alternatively, the parameters using the CLUSTALW method (e.g., version 1.83) may be modified to also use KTUPLE =1, GAP PENALTY=10, GAP extension =1, matrix = BLOSUM (e.g., BLOSUM64), WINDOW=5, and TOP DIAGONALS SAVED=5.

Various polypeptide amino acid sequences and polynucleotide sequences are disclosed herein as features of certain aspects. Variants of these sequences that are at least about 70-85%, 85-90%, or 90%-95% identical to the sequences disclosed herein may be used in certain embodiments. Alternatively, a variant polypeptide sequence or polynucleotide sequence in certain embodiments can have at least 60%, 61%, 62%,63%,64%, 65%, 66%, 67%, 68%,69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identity with a sequence disclosed herein. The variant amino acid sequence or polynucleotide sequence has the same function of the disclosed sequence, or at least about 85%, 86%, 87%, 88%, 89%, 90%, 91%, 92%, 93%, 94%, 95%, 96%, 97%, 98%, or 99% of the function of the disclosed sequence.

The term "variant", with respect to a polypeptide, refers to a polypeptide that differs from a specified wild-type, parental, or reference polypeptide in that it includes one or more naturally-occurring or man-made substitutions, insertions, or deletions of an amino acid. Similarly, the term "variant," with respect to a polynucleotide, refers to a polynucleotide that differs in nucleotide sequence from a specified wild-type, parental, or reference polynucleotide. The identity of the wild-type, parental, or reference polypeptide or polynucleotide will be apparent from context.

Industrial uses of a serine protease from the archaeon *Thermococcus nautili* have been identified. The protease has been given the name TnaPro1. The *TnaPro1* gene has the sequence presented in SEQ ID NO: 1. The amino acid sequence encoded by SEQ ID NO: 1, which corresponds to the full-length sequence of the TnaPro1 enzyme, is presented in SEQ ID NO: 2. The DNA sequence of SEQ ID NO: 1 corresponds to NCBI Reference Sequence: NZ_CP007264.1, bases 1327825-1329105, complementary. The amino acid sequence of SEQ ID NO: 2 is that of GenBank accession number: AHL23118.1.

The full length TnaPro1 sequence presented in SEQ ID NO: 2 corresponds to a pre-pro-enzyme, i.e. it comprises both a signal sequence and a pro-enzyme fragment. The N-terminal 26 amino acids of full length TnaPro1 (i.e. amino acids 1-26 of SEQ ID NO: 2) constitute a predicted signal peptide. The amino acid sequence of the predicted TnaPro1 signal peptide is presented in SEQ ID NO: 10; the DNA sequence which natively encodes the TnaPro1 signal peptide is presented in SEQ ID NO: 9. The TnaPro1 pro-enzyme, i.e. the TnaPro1 protein following cleavage of the signal peptide, has the amino acid sequence presented in SEQ ID NO: 8, which corresponds to amino acids 27-426 of SEQ ID NO: 2. The DNA sequence which natively encodes the TnaPro1 pro-enzyme is presented in SEQ ID NO: 7.

The DNA sequence which natively encodes the TnaPro1 pro-enzyme fragment is presented in SEQ ID NO: 11. The pro-enzyme fragment is cleaved from the TnaPro1 pro-enzyme to yield the mature, active form of the enzyme. The mature form of the enzyme has the amino acid sequence presented in SEQ ID NO: 3, which corresponds to amino acids 102-426 of SEQ ID NO: 2. The DNA sequence which natively encodes the mature form of TnaPro1 is presented in SEQ ID NO: 6.

Thus, the minimal part of TnaPro1 required to provide an active serine protease can be seen to be the mature form with the sequence set forth in SEQ ID NO: 3 either alone or with the addition of about 3 amino acids to the N-terminus and/or the C-terminus and/or the deletion of about 3 amino acids to the N-terminus and/or the C-terminus

Accordingly, in one embodiment, there is described a recombinant construct comprising a nucleotide sequence encoding a thermostable polypeptide having serine protease activity, wherein said coding nucleotide sequence is operably linked to at least one regulatory sequence functional in a production host and the nucleotide sequence encodes a polypeptide with the amino acid sequence set forth in SEQ ID NO: 3, or a polypeptide with at least 92% amino acid sequence identity thereto;
and wherein said regulatory sequence is heterologous to the coding nucleotide sequence, or said regulatory sequence and coding sequence are not arranged as found together in nature.

In a second embodiment, the coding nucleotide of any of the recombinant constructs described here is a nucleotide sequence encoding a polypeptide with the amino acid sequence set forth in SEQ ID NO: 8, or a polypeptide with at least 89% amino acid sequence identity thereto.

In a third aspect, the coding nucleotide sequence is selected from the group consisting of:
i) a nucleotide sequence encoding a polypeptide with the amino acid sequence set forth in SEQ ID NO: 2, or a polypeptide with at least 86 % amino acid sequence identity thereto; or
ii) a nucleotide sequence encoding a polypeptide with the amino acid sequence set forth in SEQ ID NO:5 or 14, or a polypeptide with at least 84% amino acid sequence identity thereto.

Furthermore, the at least one regulatory sequence comprises a promoter.

By "said regulatory sequence is heterologous to the coding nucleotide sequence" is meant that the regulatory sequence does not occur with the coding sequence in nature. In other words, it is not a native regulatory sequence which occurs in the *TnaPro1* gene as it is present in a native *Thermococcus nautili* cell, or, alternatively expressed, it is not the regulatory sequence which occurs with the coding sequence in the native endogenous gene. Expressed still differently, it is not the native endogenous regulatory sequence of the *TnaPro1* gene. In a particular embodiment the regulatory sequence is obtained from a different species to the species from which the coding nucleotide sequence is obtained. The coding nucleotide sequence is the *TnaPro1* gene or a variant thereof. As mentioned above, *TnaPro1* is natively encoded by *Thermococcus nautili.* Thus, a regulatory sequence heterologous to the coding nucleotide may be a regulatory sequence which is obtained from a species other than *T. nautili,* or an artificial regulatory sequence.

For the regulatory sequence and coding sequence not to be arranged as found in nature merely means that the regulatory and coding sequences in the recombinant construct are not arranged identically to the arrangement of the *TnaPro1* gene in *T. nautili.* Thus, if the coding sequence for the serine protease is operably linked to a regulatory sequence from a species which is not *T. nautili,* this requirement will inevitably be met. However, it is not a requirement that the regulatory sequence be from a species different to *T. nautili.* It may be a regulatory sequence from *T. nautili* which natively regulates the expression of a different gene (i.e. a gene which is not *TnaPro1*), e.g. a house-keeping gene such as RNA polymerase or suchlike. Alternatively, in the recombinant construct of the invention, the serine protease coding sequence may be operably linked to a regulatory sequence which is native to the *TnaPro1* gene, e.g. the *TnaPro1* promoter, such that the arrangement of the regulatory and coding sequences is different to that found in nature. The arrangement may differ in the sequence of the regulatory sequence, e.g. the native *TnaPro1* promoter may be altered to alter, e.g. enhance, its activity. Alternatively, the spacing between the regulatory and coding sequences may be different to that found in nature.

The regulatory sequence may be any sequence which is necessary or advantageous for the expression of the serine protease polypeptide, i.e. it may be any expression control sequence. Such regulatory sequences include but are not limited to, a leader sequence, a polyadenylation sequence, a propeptide fragment sequence, a promoter sequence, a signal sequence and a transcription terminator.

In a preferred embodiment of the invention, the at least one regulatory sequence operably linked to the coding sequence which encodes the serine protease comprises a promoter. The promoter may be a constitutive promoter or an inducible promoter, as defined herein. Such a promoter may particularly be a promoter sequence recognised by a bacterial host cell, or a particular or specific bacterial host cell or group of host cells. In one particular embodiment the promoter may be derived from or recognised by a *Bacillus* host cell. The promoter region may comprise a single promoter or a combination of promoters. Where the promoter region comprises a combination of promoters, the promoters are preferably in tandem. A promoter of the promoter region is preferably a promoter that can initiate transcription of a polynucleotide encoding a polypeptide having biological activity in a *Bacillus* host cell of interest. Such a promoter can particularly be obtained from native *Bacillus* genes, particularly native *B. subtilis* genes, which direct expression of polypeptides having biological activity. The regulatory sequence may also or alternatively comprise a terminator, an operator sequence or any other regulatory sequence as defined herein.

Thus, in certain embodiments, the at least one regulatory sequence operably linked to the coding sequence which encodes the serine protease comprises a promoter obtained from a bacterial source. Possible bacterial sources include Gram-positive and Gram-negative bacteria. Gram-positive bacteria include, but are not limited to, those of the genera *Bacillus, Streptococcus, Streptomyces, Staphylococcus, Enterococcus, Lactobacillus, Lactococcus, Clostridium, Geobacillus* and *Oceanobacillus.* Gram-negative bacteria include, but are not limited to, *E. coli* and those of the genera *Pseudomonas, Salmonella, Campylobacter, Helicobacter, Flavobacterium, Fusobacterium, Ilyobacter, Neisseria* and *Ureaplasma.* The promoter region may comprise a promoter obtained from a *Bacillus* species or strain (e.g. *Bacillus agaradherens, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis* or *Bacillus thuringiensis*) or from a *Streptomyces* strain (e.g. *Streptomyces lividans* or *Streptomyces murinus*).

Examples of suitable promoters for directing transcription of a polynucleotide encoding a polypeptide having biological activity in the methods of the present disclosure are the promoters obtained from the *E*. *coli lac* operon, the *Streptomyces coelicolor* agarase gene (*dagA*), the *B. lentus* or *B. clausii* alkaline protease gene (*aprH*), the *B. licheniformis* alkaline protease gene (the subtilisin Carlsberg gene), the *B. subtilis* levansucrase gene (*sacB*), the *B. subtilis* alpha-amylase gene (*amyE*), the *B. licheniformis* alpha-amylase gene (*amyL*), the *B. stearothermophilus* maltogenic amylase gene (*amyM*), the *B. amyloliquefaciens* alpha-amylase gene (*amyQ*), the *B. licheniformis* penicillinase gene (*penP*), the *B. subtilis xylA* and *xylB* genes, the *B. thuringiensis* subsp. tenebfionis CryIIIA gene (*cryIIIA*) or portions thereof, a prokaryotic beta-lactamase gene (Villa-Kamaroff et al., 1978, Proceedings of the National Academy of Sciences USA 75:3727-3731), and the *B. megaterium xylA* gene (Rygus and Hillen, 1992, J. Bacteriol. 174: 3049-3055; Kim et al., 1996, Gene 181: 71-76). Other examples are the promoter of the spo1 bacterial phage promoter and the tac promoter (DeBoer et al., 1983, Proceedings of the National Academy of Sciences USA 80:21-25). Further promoters are described in "Useful proteins from recombinant bacteria" in Scientific American, 1980, 242:74-94; and in Sambrook, Fritsch, and Maniatis, 1989, Molecular Cloning, A Laboratory Manual, 2d edition, Cold Spring Harbor, N.Y. A preferred promoter is the wild-type *B. subtilis aprE* promoter, a mutant *aprE* promoter or a consensus *aprE* promoter as set forth in PCT International Publication No. WO 2001/51643. Others include the wild-type *B. subtilis spoVG* promoter, a mutant *spoVG* promoter, or a consensus *spoVG* promoter (Frisby and Zuber, 1991).

The promoter may be a ribosomal promoter such as a ribosomal RNA promoter or a ribosomal protein promoter. The ribosomal RNA promoter can be an *rrn* promoter derived from *B. subtilis,* more particularly the *rrn* promoter can be an *rrnB, rrnl* or *rrnE* ribosomal promoter from *B. subtilis.* In certain embodiments, the ribosomal RNA promoter is a P2 *rrnl* promoter from *B. subtilis* as set forth in PCT International Publication No. WO2013/086219.

The promoter region may comprise a promoter that is a "consensus" promoter having the sequence TTGACA for the "-35" region and TATAAT for the "-10" region. The consensus promoter may be obtained from any promoter that can function in a *Bacillus* host cell. The construction of a "consensus" promoter may be accomplished by site-directed mutagenesis using methods well known in the art to create a promoter that conforms more perfectly to the established consensus sequences for the "-10" and "-35" regions of the vegetative "sigma A-type" promoters for *B. subtilis* (Voskuil et al., 1995, Molecular Microbiology 17: 271-279).

The at least one regulatory sequence operably linked to the coding sequence which encodes the serine protease may also or alternatively comprise a suitable transcription terminator sequence, such as a sequence recognised by a bacterial host cell (e.g. a *Bacillus* host cell) to terminate transcription. A terminator sequence is operably linked to the 3' terminus of the nucleotide sequence encoding a thermostable serine protease. Preferably a terminator that is functional in a *Bacillus* host cell or an *E. coli* host cell may be used.

The regulatory sequence (which may also be referred to as a control sequence, or expression control sequence) may also be, or include, a suitable leader sequence, a non-translated region of a mRNA that is important for translation by a host cell, in particular a bacterial host cell, such as a *Bacillus* host cell or an *E. coli* host cell. The leader sequence is operably linked to the 5' terminus of the nucleotide sequence directing synthesis of the polypeptide having biological activity. Any leader sequence that is functional in a host cell of choice (e.g. a microbial, bacterial or *Bacillus* host cell) may be used in the present invention.

The at least one regulatory sequence may also or alternatively comprise an mRNA stabilising sequence. The term "mRNA stabilising sequence" is defined herein as a sequence located downstream of a promoter region and upstream of a coding sequence of a polynucleotide encoding a thermostable serine protease to which the promoter region is operably linked, such that all mRNAs synthesised from the promoter region may be processed to generate mRNA transcripts with a stabiliser sequence at the 5' end of the transcripts. For example, the presence of such a stabiliser sequence at the 5' end of the mRNA transcripts increases their half-life (Agaisse and Lereclus, 1994, supra, Hue et al., 1995, Journal of Bacteriology 177: 3465-3471). The mRNA processing/stabilising sequence is complementary to the 3' extremity of bacterial 16S ribosomal RNA. In certain embodiments, the mRNA processing/stabilising sequence generates essentially single-size transcripts with a stabilizing sequence at the 5' end of the transcripts. The mRNA processing/stabilising sequence is preferably one which is complementary to the 3' extremity of a bacterial 16S ribosomal RNA. See U.S. Patent No. 6,255,076 and U.S. Patent No. 5,955,310.

Regulatory sequences may be provided with linkers for the purpose of introducing specific restriction sites facilitating ligation or the regulatory sequences with the coding region of the nucleotide sequence encoding a thermostable serine protease. In the recombinant construct of the invention, the nucleotide sequence encoding a thermostable polypeptide having serine protease activity may be operably linked to one or more regulatory sequences capable of directing the expression of the coding sequence in a host cell, for example a *Bacillus* host cell, particularly a *B*. *subtilis* cell, under conditions compatible with the regulatory sequences.

The thermostable serine protease-encoding sequence in the recombinant construct encodes a polypeptide with the amino acid sequence of SEQ ID NO: 3 or a polypeptide with at least 92% sequence identity thereto. In particular embodiments, the nucleotide sequence which encodes the thermostable polypeptide with serine protease activity is codon-optimised for expression of the nucleotide sequence in a non-native host cell. In particular, the nucleotide sequence may be codon-optimised for expression in a prokaryote, such as *Escherichia coli* or *Bacillus subtilis.* Techniques for modifying nucleic acid sequences utilizing cloning methods are well known in the art.

In particular embodiments, the thermostable polypeptide having serine protease activity encoded by the coding sequence of the recombinant construct of the invention is encoded in the form of a pro-enzyme. The pro-enzyme fragment of the pro-enzyme may be encoded at its N-terminus or at its C-terminus. Preferably, the pro-enzyme fragment is located at the N-terminus of the pro-enzyme.

In a particular embodiment of the invention, the coding nucleotide sequence of the recombinant construct is a nucleotide sequence encoding a polypeptide with the amino acid sequence set forth in SEQ ID NO: 8, or a polypeptide with at least 70 %, 75 %, 80 %, 85 %, 89%, 90 %, 91%, 92%, 93%, 94%, 95 %, 96 %, 97 %, 98 % or 99 % amino acid sequence identity thereto.

In this particular embodiment, the pro-enzyme is the native TnaPro1 pro-enzyme or a variant thereof. It is necessary in any variant of the TnaPro1 pro-enzyme that the pro-enzyme fragment be cleavable from the mature enzyme sequence. It is thus preferred that in any variant of SEQ ID NO: 7 or a nucleotide sequence encoding a variant of SEQ ID NO: 8 the sequence encoding the pro-enzyme cleavage site is unaltered, or at least that the sequence which it encodes is unaltered, such that the cleavage site in the pro-enzyme expressed from the construct is unaltered and recognised for cleavage, so that the pro-enzyme can be correctly processed into its active, mature form.

The thermostable polypeptide having serine protease activity encoded by the coding sequence of the recombinant construct described herein may be encoded such that it comprises a signal peptide, i.e. encoded as a pre-enzyme or a pre-pro-enzyme. Preferably the thermostable polypeptide having serine protease activity is encoded as a pre-pro-enzyme, comprising from N-terminus to C-terminus a signal peptide, a pro-enzyme fragment and the mature enzyme sequence. Preferably the pro-enzyme sequence is as defined above. In one aspect of this embodiment, the signal sequence is that of the native TnaPro1 protein, i.e. it has the polypeptide sequence set forth in SEQ ID NO: 10. Thus in one embodiment, the coding nucleotide sequence in the recombinant construct of the invention is

a nucleotide sequence encoding a polypeptide with the amino acid sequence set forth in SEQ ID NO: 2, or a polypeptide with at least 70 % 75 %, 80 %, 85 %, 86%, 87%, 88%, 89%,90 %, 91%, 92%, 93%, 94%, 95 %, 96 %, 97 %, 98 % or 99 % amino acid sequence identity thereto.

The signal sequence can alternatively, however, be a signal sequence from any other gene or organism, or an artificial signal sequence. In particular, the signal sequence may be a signal sequence native to the host cell in which the thermostable polypeptide having serine protease activity is to be expressed. For instance, the signal sequence may be an *E. coli* or *B. subtilis* signal sequence. Such signal sequences are well-known and easily available to those in the art. In a particular embodiment of the invention, the thermostable polypeptide having serine protease activity is encoded as a pre-pro-enzyme, in which the pro-enzyme sequence is or is a variant of the TnaPro1 pro-enzyme sequence (i.e. the coding nucleotide sequence of the recombinant construct is a nucleotide sequence encoding a polypeptide with the amino acid sequence set forth in SEQ ID NO: 8, or a polypeptide with at least 70 %, 75 %, 80 %, 85 %, 86%, 87%, 88%, 89%90 %, 91%, 92%, 93%, 94%, 95 %, 96 %, 97 %, 98 % or 99 % amino acid sequence identity thereto), and in which the signal sequence is the *B. subtilis* AprE signal sequence. AprE is the serine protease Subtilisin E, native to *B. subtilis.* The AprE signal sequence has the polypeptide sequence presented in SEQ ID NO: 16, and is natively encoded by the nucleotide sequence presented in SEQ ID NO: 15. A TnaPro1 pre-pro-enzyme with the AprE signal sequence and the native TnaPro1 pro-enzyme sequence has the amino acid sequence presented in SEQ ID NO: 14, which is natively encoded by the nucleotide sequence presented in SEQ ID NO: 13 (in this particular instance, natively encoded indicates that the gene consists of the native coding sequence of the AprE signal sequence and the native coding sequence of the TnaPro1 pro-enzyme).

The Examples below describe the expression of a protein in which the AprE signal sequence is separated from the native TnaPro1 pro-enzyme sequence by a 3-amino acid (AGK) spacer, introduced as a result of ligating the coding sequence into the expression vector p2JM103BBI. The amino acid sequence of this protein is shown in SEQ ID NO: 5 and its coding nucleotide sequence is shown in SEQ ID NO.4.

Thus, in a particular embodiment, the coding nucleotide sequence in the recombinant construct described herein is a nucleotide sequence encoding a polypeptide with the amino acid sequence set forth in SEQ ID NO: 5 or 14, or a polypeptide with at least 70 %, 75 %, 80 %, 8'%, 82%, 83%, 84%, 85 %,86%, 87%, 88%, 89% 90 %, 91%, 92%, 93%, 94%, 95 %, 96 %, 97 %, 98 % or 99 % amino acid sequence identity thereto.

In other embodiments, the signal sequence may be any signal sequence effective in a desired host cell e.g. a *Bacillus* host cell. Examples thereof include the signal peptide coding region obtained from the maltogenic amylase gene from *Bacillus* NCIB 11837, the *B. stearothermophilus* alpha-amylase gene, the *B. licheniformis* subtilisin gene, the *B. licheniformis* beta-lactamase gene, the *B. stearothermophilus* neutral protease genes (*nprT, nprS, nprM*), and the *B.* subtilis *prsA* gene.

Thus, in certain embodiments, the coding sequence may encode a mature serine protease enzyme without a signal sequence (i.e. without a "pre" sequence), and preferably also without a "pro" sequence. In such an embodiment the coding nucleotide sequence may consist of (i) the nucleotide sequence set forth in SEQ ID NO: 6 or a nucleotide sequence with at least 92% sequence identity thereto or (ii) a nucleotide sequence encoding a polypeptide with the amino acid sequence set forth in SEQ ID NO: 3, or a polypeptide with at least 92% amino acid sequence identity thereto. Alternatively the coding sequence, or more particularly the recombinant vector, may comprise a nucleotide sequence of (i) or (ii) but will not comprise any further nucleotide sequence from SEQ ID NO: 1 which flanks, or lies immediately adjacent to the sequence of SEQ ID NO: 6, or any further nucleotide sequence which encodes any amino acid sequence from SEQ ID NO: 2 which flanks or lies immediately adjacent to the sequence of SEQ ID NO: 3. In other words, the recombinant construct may comprise only a coding sequence encoding the mature polypeptide and does not include any nucleotide sequence encoding a pre-, pro- or pre-pro- sequence from SEQ ID NO: 2, or pre-, pro- or pre-pro- sequence which has at least 86% sequence identity to the pre-, pro- or pre-pro- sequence of SEQ ID NO: 2.

In the event that the coding nucleotide sequence is a variant of SEQ ID NO: 1 or SEQ ID NO: 4, or encodes a variant of SEQ ID NO: 2, 5 or 14, or encodes a variant of a pre-pro-enzyme in which the signal sequence is neither the TnaPro1 or *B. subtilis aprE* signal sequence (e.g. the signal sequence is one of those listed above or an alternative non-native signal sequence) it is preferred that not only is the sequence encoding the pro-enzyme cleavage sit unaltered, or at least the sequence which it encodes unaltered, such that the cleavage site in the pro-enzyme expressed from the construct is unaltered and recognised for cleavage, but that also the sequence encoding the signal sequence cleavage site (i.e. the site cleaved by a signal peptidase to remove the signal sequence from the pre-protein) is unaltered, or at least the sequence which it encodes unaltered, such it is recognised by the signal peptidase to allow proper processing of the pre-protein.

The serine protease encoded in the construct described herein may preferably be encoded with an affinity tag for improved ease of purification. Such a tag is preferably located at the terminus of the protease. If the protease is encoded as a pro-enzyme or a pre-pro-enzyme, with a signal sequence and/or pro-enzyme fragment at its N-terminus, the protease is preferably encoded with an affinity tag at its C-terminus. Suitable tags are well-known in the art and include a polyhistidine tag, a strep tag, a FLAG tag, an HA tag or suchlike.

In another embodiment, there is also disclosed a vector comprising the recombinant construct described herein. The vector may be any vector as defined herein, including that it may be a cloning vector or an expression vector. The vector may be any vector that can be introduced into (e.g. transformed into) and replicated within a host cell. Preferably the vector is a plasmid. The vector of the invention is preferably suitable for use in bacteria, particularly for use in *E. coli* or *B. subtilis.* Suitable vectors for use in these species, and other specific species, are well-known in the art.

A suitable vector may comprise regulatory sequences as described above, e.g. one or more promoter, terminator or leader sequences etc. A suitable vector may further comprise a nucleic acid sequence enabling the vector to replicate in a host cell. Examples of such enabling sequences include the origins of replication of plasmids pUC19, pACYC177, pUB110, pE194, pAMB1, pIJ702, and the like.

A suitable vector may also comprise a selectable marker, e.g. a gene the product of which complements a defect in the isolated host cell, such as the *dal* genes from *B. subtilis* or *B. licheniformis;* or a gene that confers antibiotic resistance such as, e.g. ampicillin resistance, kanamycin resistance, chloramphenicol resistance, tetracycline resistance or the like.

A suitable cloning vector thus typically includes an element that permits autonomous replication of the vector in the selected host organism and one or more phenotypically detectable markers for selection purposes. Examples of general purpose cloning vectors suitable for use in bacteria, particularly *E. coli,* include pUC19, pBR322, pBluescript vectors (Stratagene Inc.) and pCR TOPO® from Invitrogen Inc., e.g. pCR2.1-TOPO.

A suitable expression vector typically includes an element that permits autonomous replication of the vector in the selected production host or host organism and one or more phenotypically detectable markers for selection purposes, in the same manner as a cloning vector. Expression vectors typically also comprise control nucleotide sequences such as, for example, a promoter, an operator, a ribosome binding site, a translation initiation signal and optionally, a repressor gene, one or more activator gene sequences, or the like.

An expression vector may also comprise a sequence for an affinity tag to be fused in-frame to the sequence of the gene of interest, such that the encoded protein is produced with such a tag at one or other terminus. Examples of affinity tags which may be used in the invention are given above. Examples of expression vectors suitable for use in bacteria include those derived from, *inter alia,* vectors of the pET family, such as pET3a, pET3b, pET3c, pET3d, pET12a, pET14b, pET15b, pET16b, pET28a and pET28c; vectors of the pBAD family, such as pBAD/HisA, pBAD/HisB and pBAD/HisC; and vectors of the pGEX family, such as pGEX-3X, pGEX-4T-1, pGEX-4T-2, pGEX-4T-3, pGEX-5X-1, pGEX-5X-2 and pGEX-5X-3. A further representative bacterial expression vector is p2JM103BBI. Expression vectors suitable for use in yeast include those derived from, *inter alia,* vectors of the pAG family, such as pAG423-type vectors, pAG424-type vectors, pAG425-type vectors, pAG426-type vectors and pAG303-type vectors; vectors of the pRG family, such as pRG206, pRG221, pRG227 and pRG236; and vectors of the pYC family, such as pYC48, pYC50, pYC55 and pYC64. If the skilled person wishes to construct a cloning or expression vector of the invention, he or she will be able to choose an appropriate vector backbone based on the considerations discussed above. Protocols used to ligate the DNA construct encoding a protein of interest, promoters, terminators and/or other elements, and to insert them into suitable vectors containing the information necessary for replication, are well known to persons skilled in the art.

There is further provided a production host or host cell comprising a recombinant construct or vector as defined herein. The host cell is preferably a non-native host cell, i.e. a host cell which is not the species from which TnaPro1 is derived, i.e. preferably not a *T. nautili* cell. The host cell may be a prokaryotic or a eukaryotic cell. Preferably it is a cell of a microorganism. The host cell may be a bacterial (by which is meant eubacterial) cell, an archaeal cell, a fungal cell, an algal cell, a mammalian cell or any other type of cell. The cell is not located within an animal. When the host cell is a bacterial cell, it may be from either a Gram-positive or a Gram-negative species. Preferably the bacterial cell is an *E. coli* cell or a *B. subtilis* cell. The host cell may be from a thermophilic prokaryote, such as a thermophilic bacterium or a thermophilic archaeon. If the host cell is a fungal cell, it may be the cell of a filamentous fungus or a yeast cell, particularly a cell of the species *Pichia pastoris.*

Methods for introducing nucleotide sequences, such as constructs and vectors, into cells are described above and are well-known in the art, as are methods of identifying cells into which such constructs have been introduced. A host cell of the invention may be obtained using any such appropriate method, e.g. transformation followed by positive selection using a marker such as antibiotic resistance. Transformation and expression methods for bacteria are disclosed in Brigidi *et al.* (1990). A general transformation and expression protocol for protease deleted *Bacillus* strains is described in Ferrari et al. (U.S. Patent No. 5, 264,366). Other methods for introducing DNA into cells include electroporation, nuclear microinjection, transduction, transfection (e.g., lipofection mediated and DEAE-Dextrin mediated transfection), incubation with calcium phosphate DNA precipitate, high velocity bombardment with DNA-coated microprojectiles, gene gun or biolistic transformation and protoplast fusion, and the like. Basic texts disclosing the general methods that can be used include Sambrook et al., Molecular Cloning, A Laboratory Manual (2nd ed. 1989); Kriegler, Gene Transfer and Expression: A Laboratory Manual (1990); and Ausubel et al., eds., Current Protocols in Molecular Biology (1994)).

Methods for transforming nucleic acids into filamentous fungi such as *Aspergillus spp., e.g., Aspergillus oryzae* or *Aspergillus niger, Hylarana grisea, Humicola insolens,* and *Trichoderma reesei.* are well known in the art. A suitable procedure for transformation of *Aspergillus* host cells is described, for example, in EP 238023. A suitable procedure for transformation of *Trichoderma* host cells is described, for example, in Steiger et al 2011, Appl. Environ. Microbiol. 77:114-121.

Many standard transfection methods can be used to produce bacterial and filamentous fungal (e.g. *Aspergillus* or *Trichoderma*) cell lines that express large quantities of the protease. Some of the published methods for the introduction of DNA constructs into cellulase-producing strains of *Trichoderma* include Lorito, Hayes, DiPietro and Harman, (1993) Curr. Genet. 24: 349-356; Goldman, VanMontagu and Herrera-Estrella, (1990) Curr. Genet. 17:169-174; and Penttila, Nevalainen, Ratto, Salminen and Knowles, (1987) Gene 6: 155-164, also see USP 6.022,725; USP 6,268,328 and Nevalainen et al., "The Molecular Biology of Trichoderma and its Application to the Expression of Both Homologous and Heterologous Genes" in Molecular Industrial Mycology, Eds, Leong and Berka, Marcel Dekker Inc., NY (1992) pp 129 - 148; for *Aspergillus* include Yelton, Hamer and Timberlake, (1984) Proc. Natl. Acad. Sci. USA 81: 1470-1474, for *Fusarium* include Bajar, Podila and Kolattukudy, (1991) Proc. Natl. Acad. Sci. USA 88: 8202-8212, for *Streptomyces* include Hopwood et al., 1985, Genetic Manipulation of Streptomyces: Laboratory Manual, The John Innes Foundation, Norwich, UK and Fernandez-Abalos et al., Microbiol 149:1623 - 1632 (2003) and for *Bacillus* include Brigidi, DeRossi, Bertarini, Riccardi and Matteuzzi, (1990) FEMS Microbiol. Lett. 55: 135-138).

However, any well-known procedure for introducing foreign nucleotide sequences into host cells may be used. These include the use of calcium phosphate transfection, polybrene, protoplast fusion, electroporation, biolistics, liposomes, microinjection, plasma vectors, viral vectors and any of the other well-known methods for introducing cloned genomic DNA, cDNA, synthetic DNA or other foreign genetic material into a host cell (see, e.g., Sambrook et al., *supra*). Also, of use is the Agrobacterium-mediated transfection method described in U.S. Patent No. 6,255,115. It is only necessary that the particular genetic engineering procedure used be capable of successfully introducing at least one gene into the host cell capable of expressing the gene.

The construct or vector of the invention comprised within the host cell described herein may be stably integrated into the chromosome of the host cell, enabling selection-free transfer of the genetic material of the construct or vector down the generations of the host. Integration of a DNA construct or vector into a host chromosome may be performed applying conventional methods, for example by homologous or heterologous recombination. For example, PCT International Publication No. WO 2002/14490 describes methods of *Bacillus* transformation, transformants thereof and libraries thereof.

Alternatively, the construct or vector may be maintained extrachromosomally in the host cell, such that it is not integrated into the chromosome. Extrachromosomal maintenance of a construct or vector is likely to be less stable than integration of the construct or vector into the chromosome of an organism, requiring constant selection to ensure the construct or vector is not lost during reproduction of the host cell. Stable integration or extrachromasomal maintenance of the plasmid may each be beneficial for certain purposes, as is well understood by the skilled person.

The invention provides a method for producing a thermostable serine protease, said method comprising:
i) introducing into a host cell a construct or vector of the invention; and
ii) culturing the host cell produced in step (i) under conditions whereby the thermostable serine protease is produced.

The host cell may be any host cell, as defined above. Preferably it is a bacterial cell, an archaeal cell, a fungal cell or an algal cell. In particular embodiments, the host cell is an *E. coli* cell or a *B. subtilis* cell.

The construct or vector may be introduced into the host cell using any method for introducing nucleotide sequences, e.g. DNA, into host cells, as described above. Preferably, the construct or vector is introduced into the host cell by transformation.

By "culturing" is meant that the host cell is grown, such that it replicates. Methods for culturing of cells are well known in the art. For instance, the host cell may be grown in a rich medium (i.e. a medium rich in nutrients) at a physiological temperature (approx. 37°C) or a temperature optimal for cell growth and division. Depending on the type of cell used as host, the medium may be shaken during culture to ensure aeration of the medium, e.g. a shaking incubator may be used. Suitable media and media components are available from commercial suppliers or may be prepared according to published recipes (e.g. as described in catalogues of the American Type Culture Collection).

By "conditions whereby the thermostable serine protease is produced" means conditions whereunder expression of the thermostable serine protease can be detected. Detection may be direct detection of the protein, e.g. Western blotting. The protease mRNA may alternatively be directly detected, e.g. by RT-PCR, but direct detection of the protein is preferred. Detection may alternatively be by its function. Assays to determine or identify serine protease activity are described above. If the proteolysis activity of the culture of host cells comprising the construct or vector described herein is statistically significantly higher than the proteolysis activity of a culture of control cells, the serine protease may be said to be expressed. Suitable control cells include wild-type cells of the strain of the production host cell, into which no construct or vector has been introduced, or cells of the strain of the production host cell into which an empty vector has been introduced. By "empty vector" is meant the backbone of a vector of the invention, into which no construct of the invention has been cloned. Thus, an empty vector may contain all the regulatory or control sequences and markers of a vector described herein, but does not encode a polypeptide with protease activity, particularly serine protease activity.

If the serine protease encoded by the construct or vector as disclosed herein is encoded with a signal peptide, such that it is secreted from the host cells, its activity may be detected in the culture supernatant. If the serine protease is encoded without a signal peptide and is therefore not secreted from the host cells, it will be necessary to harvest and lyse the host cells, and to attempt detection of serine protease activity in their lysates. Preferably, the serine protease is encoded with a signal peptide and secreted from the cells.

Expression of the serine protease may be constitutive or inducible, depending on the promoter used. If the promoter is constitutive the protease is continually expressed under essentially all growth conditions. If the promoter is inducible a stimulus is required to induce expression. In the case of inducible expression, protein production can be initiated when required by, for example, addition of an inducer substance to the culture medium, for example dexamethasone, arabinose, IPTG or sophorose, depending on the expression system used.

Depending upon the host cell used post-transcriptional and/or post-translational modifications may be made during gene expression. One non-limiting example of a post-transcriptional and/or post-translational modification is "clipping" or "truncation" of a polypeptide. For example, this may result in taking a thermostable serine protease from an inactive or substantially inactive state to an active state as in the case of a pro-peptide undergoing post-translational processing to a mature peptide having enzymatic activity. In another instance, this clipping may result in taking a mature thermostable serine protease polypeptide and further removing N or C-terminal amino acids to generate truncated forms of the thermostable serine protease that retain enzymatic activity.

Other examples of post-transcriptional or post-translational modifications include, but are not limited to, myristoylation, glycosylation, truncation, lipidation and tyrosine, serine or threonine phosphorylation. The skilled person will appreciate that the type of post-transcriptional or post-translational modifications that a protein may undergo may depend on the host organism in which the protein is expressed.

In some embodiments, the preparation of a spent whole fermentation broth of a recombinant microorganism can be achieved using any cultivation method known in the art resulting in the expression of a thermostable serine protease.

Fermentation may, therefore, be understood as comprising shake flask cultivation, small- or large-scale fermentation (including continuous, batch, fed-batch, or solid- state fermentations) in laboratory or industrial fermenters performed in a suitable medium and under conditions allowing the serine protease to be expressed or isolated. The term "spent whole fermentation broth" is defined herein as unfractionated contents of fermentation material that includes culture medium, extracellular proteins (e.g. enzymes), and cellular biomass. It is understood that the term "spent whole fermentation broth" also encompasses cellular biomass that has been lysed or permeabilised using methods well known in the art.

Any of the fermentation methods well known in the art can suitably be used to ferment the host cell into which the construct or vector has been introduced. In some embodiments, host cells, e.g. fungal cells or bacterial cells, are grown under batch or continuous fermentation conditions.

A classical batch fermentation is a closed system, where the composition of the medium is set at the beginning of the fermentation, and the composition is not altered during the fermentation. At the beginning of the fermentation, the medium is inoculated with the desired organism(s). In other words, the entire fermentation process takes place without addition of any components to the fermentation system throughout.

Alternatively, a batch fermentation qualifies as a "batch" with respect to the addition of the carbon source. Moreover, attempts are often made to control factors such as pH and oxygen concentration throughout the fermentation process. Typically, the metabolite and biomass compositions of the batch system change constantly up to the time the fermentation is stopped. Within batch cultures, cells progress through a static lag phase to a high-growth log phase and finally to a stationary phase, where the growth rate is diminished or halted. Left untreated, cells in the stationary phase would eventually die. In general, cells in log phase are responsible for the bulk of production of product. A suitable variation on the standard batch system is the "fed-batch fermentation" system. In this variation of a typical batch system, the substrate is added in increments as the fermentation progresses. Fed-batch systems are useful when it is known that catabolite repression would inhibit the metabolism of the cells, and/or where it is desirable to have limited amounts of substrates in the fermentation medium. Measurement of the actual substrate concentration in fed-batch systems is difficult and is therefore estimated on the basis of the changes of measurable factors, such as pH, dissolved oxygen and the partial pressure of waste gases, such as CO2. Batch and fed-batch fermentations are well known in the art.

Continuous fermentation is another known method of fermentation. It is an open system where a defined fermentation medium is added continuously to a bioreactor, and an equal amount of conditioned medium is removed simultaneously for processing. Continuous fermentation generally maintains the cultures at a constant density, where cells are maintained primarily in log phase growth. Continuous fermentation allows for the modulation of one or more factors that affect cell growth and/or product concentration. For example, a limiting nutrient, such as the carbon source or nitrogen source, can be maintained at a fixed rate and all other parameters are allowed to moderate. In other systems, a number of factors affecting growth can be altered continuously while the cell concentration, measured by media turbidity, is kept constant. Continuous systems strive to maintain steady state growth conditions. Thus, cell loss due to medium being drawn off should be balanced against the cell growth rate in the fermentation. Methods of modulating nutrients and growth factors for continuous fermentation processes, as well as techniques for maximizing the rate of product formation, are well known in the art of industrial microbiology.

Preferably, the method for producing a thermostable serine protease further comprises a step of recovering the thermostable protease following its production. Recovery of the thermostable protease may take the form of its isolation, e.g. purification, and/or a concentration of the protease. Thus, after production has taken place the protease may be isolated or separated, e.g. from the host cells or from the culture. In a first step the cells and medium may be separated, e.g. by centrifugation or filtration. If the protease is in the cell fraction, the cells may then be lysed and the supernatant discarded. Lysis methods are well-known in the art and include e.g. sonication, French Press and chemical lysis using a protein extraction reagent (e.g. BugBuster®, EMD Millipore (USA)). If the protease is secreted by the host cells, such that it is in the supernatant fraction, the cell fraction may then be discarded. The protease may then be purified, e.g. by chromatography. Purification techniques are well-known in the art. For instance, if the protease is expressed with an affinity tag, as described above, the protease may be purified by affinity chromatography. Size exclusion chromatograph and/or ion-exchange chromatography may also or alternatively be used, along with any other technique known in the art, e.g. electrophoretic procedures (e.g., preparative isoelectric focusing), differential solubility (e.g., ammonium sulfate precipitation), extraction microfiltration or two-phase separation. For general guidance in suitable purification techniques, see Scopes, Protein Purification (1982). The degree of purification necessary will vary depending on the use of the protein of interest. In some instances, no purification will be necessary.

The enzyme-containing solution can be concentrated using conventional concentration techniques until the desired enzyme level is obtained. Concentration of the enzyme containing solution may be achieved by any technique known in the art. Examples of methods of enrichment/concentration include but are not limited to rotary vacuum filtration and/or ultrafiltration. Concentration may alternatively be performed using e.g. a precipitation agent, such as a metal halide precipitation agent. Metal halide precipitation agents include but are not limited to alkali metal chlorides, alkali metal bromides and blends of two or more of these metal halides.

Exemplary metal halides include sodium chloride, potassium chloride, sodium bromide, potassium bromide and blends of two or more of these metal halides. The metal halide precipitation agent, sodium chloride, can also be used as a preservative. For production scale recovery, thermostable serine protease polypeptides can be enriched or partially purified as generally described above by removing cells via flocculation with polymers. Alternatively, the enzyme can be enriched or purified by microfiltration followed by concentration by ultrafiltration using available membranes and equipment. However, for some applications, the enzyme does not need to be enriched or purified, and whole broth culture can be lysed and used without further treatment. The enzyme can then be processed, for example, into granules.

In another aspect, there is described a culture supernatant comprising a thermostable polypeptide with serine protease activity obtained by the above method for producing a thermostable serine protease. The thermostable polypeptide with serine protease activity comprised within the culture supernatant of the invention preferably has or comprises the amino acid sequence set forth in SEQ ID NO: 3, or an amino acid sequence with at least 70 %, 75 %, 80 %, 85 %, 90 %, 91%, 92%, 93%, 94%, 95 %, 96%, 97 %, 98 % or 99 % sequence identity thereto. In certain embodiments, the thermostable polypeptide with serine protease activity comprised within the culture supernatant described herein has or comprises the amino acid sequence set forth in SEQ ID NO: 8, or an amino acid sequence with at least 70 %, 75 %, 80 %, 85 %, 86%, 87%, 88%, 89%, 90 %, 95 %, 96 %, 97 %, 98 % or 99 % sequence identity thereto. Such a supernatant is obtainable when the serine protease is encoded, expressed and produced with a signal peptide, which directs the protease for secretion from the host cell. The serine protease is thus secreted into the culture medium. The signal peptide is cleaved from the serine protease upon its secretion from the host cell, and so the thermostable serine protease present in the culture supernatant does not comprise a signal sequence. A culture supernatant may be obtained by separating the cells from the medium. Methods for performing this step are well known in the art and are described above, e.g. centrifugation and filtration.

Thermostable serine proteases may be isolated or purified in a variety of ways known to those skilled in the art depending on what other components are present in the sample. Standard purification methods include, but are not limited to, chromatography (e.g., ion exchange, affinity, hydrophobic, chromatofocusing, immunological and size exclusion), electrophoretic procedures (e.g., preparative isoelectric focusing), differential solubility (e.g., ammonium sulfate precipitation), extraction microfiltration, two phase separation. For example, the protein of interest may be purified using a standard anti-protein of interest antibody column. Ultrafiltration and diafiltration techniques, in conjunction with protein concentration, are also useful. For general guidance in suitable purification techniques, see Scopes, Protein Purification (1982). The degree of purification necessary will vary depending on the use of the protein of interest. In some instances, no purification will be necessary.

Assays for detecting and measuring the enzymatic activity of an enzyme, such as a thermostable serine protease polypeptide, are well known. Various assays for detecting and measuring activity of proteases (e.g., thermostable serine protease polypeptides), are also known to those of ordinary skill in the art. In particular, assays are available for measuring protease activity that are based on the release of acid-soluble peptides from casein or hemoglobin, measured as absorbance at 280 nm or colorimetrically using the Folin method, and hydrolysis of the dye-labeled azocasein, measured as absorbance at 440-450 nm.

Other exemplary assays involve the solubilization of chromogenic substrates (See e.g., Ward, "Proteinases," in Fogarty (ed.)., Microbial Enzymes and Biotechnology, Applied Science, London, [1983], pp. 251-317). A protease detection assay method using highly labelled fluorescein isothiocyanate (FITC) casein as the substrate, a modified version of the procedure described by Twining [Twining, S.S., (1984) "Fluorescein Isothiocyanate-Labelled Casein Assay for Proteolytic Enzymes" Anal. Biochem. 143:30-34] may also be used. Other exemplary assays include, but are not limited to: cleavage of casein into trichloroacetic acid-soluble peptides containing tyrosine and tryptophan residues, followed by reaction with Folin-Ciocalteu reagent and colorimetric detection of products at 660 nm, cleavage of internally quenched FRET (Fluorescence Resonance Energy Transfer) peptide substrates followed by detection of product using a fluorometer. Fluorescence Resonance Energy Transfer (FRET) is the non-radiative transfer of energy from an excited fluorophore (or donor) to a suitable quencher (or acceptor) molecule. FRET is used in a variety of applications including the measurement of protease activity with substrates, in which the fluorophore is separated from the quencher by a short peptide sequence containing the enzyme cleavage site. Proteolysis of the peptide results in fluorescence as the fluorophore and quencher are separated. Numerous additional references known to those in the art provide suitable methods (See e.g., Wells et al., Nucleic Acids Res. 11:7911-7925 [1983]; Christianson et al., Anal. Biochem. 223:119 -129 [1994]; and Hsia et al., Anal Biochem. 242:221-227 [1999]).

Also provided herein is an animal feed product comprising a polypeptide which has serine protease activity and is thermostable, wherein said polypeptide comprises the amino acid sequence set forth in SEQ ID NO: 3, or an amino acid sequence with at least 70 %, 75 %, 80 %, 85 %, 90 %, 91%, 92%, 93,%, 94% 95 %, 96 %, 97 %, 98 % or 99 % sequence identity thereto, and wherein said food product optionally further comprises (a) at least one direct-fed microbial (DFM) of (b) at least one other enzyme or (c) both a direct fed microbial and at least one other enzyme. In certain embodiments the animal feed product may comprise an amino acid sequence as set forth in SEQ ID NO: 8 or a polypeptide with at least 70 %, 75 %, 80 %, 85 %, 90 %, 95 %, 96 %, 97 %, 98 % or 99 % amino acid sequence identity thereto.

The at least one other enzyme can be selected from, but is not limited to, enzymes such as alpha-amylase, amyloglucosidase, phytase, pullulanase, beta-glucanase, cellulase, xylanase, etc.

Any of these enzymes can be used in an amount ranging from 0.5 to 500 micrograms/g feed or feedstock.

Alpha-amylases (alpha-1,4-glucan-4-glucanohydrolase, EC 3.2.1.1.) hydrolyze internal alpha-1,4-glucosidic linkages in starch, largely at random to produce smaller molecular weight dextrans. These polypeptides are used, *inter alia,* in starch processing and in alcohol production. Any alpha-amylases can be used, e.g., those described in U.S. Patent Nos. 8,927,250 and 7,354,752.

Amyloglucosidase catalyzes the hydrolysis of terminal 1,4-linked alpha-D-glucose residues successively from the non-reducing ends of maltooligo- and polysaccharides with release of beta-D-glucose. Any amyloglucosidase can be used.

Phytase refers to a protein or polypeptide which is capable of catalyzing the hydrolysis of phytate to (1) myo-inositol and/or (2) mono-, di-, tri-, tetra-, and/or penta-phosphates thereof and (3) inorganic phosphate. For example, enzymes having catalytic activity as defined in Enzyme Commission EC number 3.1.3.8 or EC number 3.1.3.26. Any phytase can be used such as described in U.S. Patent Nos. 8,144,046, 8,673,609, and 8,053,221.

Pullulanase (EC 3.2.1.41) is a specific kind of glucanase, an amylolytic exoenzyme that degrades pullan (a polysaccharide polymer consisting of maltotriose units, also known as alpha-1,4-; alpha-1,6-glucan. Thus, it is an example of a debranching enzyme. Pullulanase is also known as pullulan-6-glucanohydrolase. Pullulanases are generally secreted by a *Bacillus* species. For example, *Bacillus deramificans* (US Patent No. 5,817,498; 1998), *Bacillus acidopullulyticus* (European Patent No. 0 063 909) and *Bacillus naganoensis* (US Patent No. 5,055,403). Enzymes having pullulanase activity used commercially are produced, for example, from *Bacillus* species (trade name OPITMAX® I-100 from DuPont-Genencor and Promozyme® D2 from Novozymes). Other examples of debranching enzymes include, but are not limited to, iso-amylase from *Sulfolobus solfataricus, Pseudomonas* sp. and thermostable pullulanase from *Fervidobacterium nodosum* (e.g., WO2010/76113). The iso-amylase from *Pseudomonas* sp. is available as purified enzyme from Megazyme International. Any pullulanase can be used.

Glucanases are enzymes that break down a glucan, a polysaccharide made several glucose sub-units. As they perform hydrolysis of the glucosidic bond, they are hydrolases.

Beta-glucanase enzymes (EC 3.2.1.4) digests fiber. It helps in the breakdown of plant walls (cellulose).

Cellulases are any of several enzymes produced by fungi, bacteria and protozoans that catalyze cellulolysis, the decomposition of cellulose and of some related polysaccharides. The name is also used for any naturally-occurring mixture or complex of various such enzymes, that act serially or synergistically to decompose cellulosic material. Any cellulases can be used.

Xylanase (EC 3.2.1.8) is the name given to a class of enzymes which degrade the linear polysaccharide beta-1,4-xylan into xylose, those breaking down hemicellulose, one of the major components of plant cell walls. Any xylanases can be used.

As defined herein, animal feed or animal feed product encompasses a feed, or a feedstuff, feed additive composition, premix, by an animal, including humans.

The terms "animal feed composition", "feed", "feedstuff" and "fodder" are used interchangeably herein. A "feed" is defined above, the definition applying equally to the interchangeable terms "animal feed composition", "feedstuff' and "fodder".

A feed can comprise one or more feed materials selected from the group comprising a) cereals, such as small grains (e.g. wheat, barley, rye, oats and combinations thereof) and/or large grains such as maize or sorghum; b) by products from cereals, such as corn gluten meal, Distillers Dried Grains with Solubles (DDGS) (particularly corn-based Distillers Dried Grains with Solubles (cDDGS)), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; and/or e) minerals and vitamins.

A feed may be understood to be any food product which is provided to an animal (rather than the animal having to forage for it themselves). Feed encompasses plants that have been cut. Furthermore, feed includes silage, compressed and pelleted feeds, oils and mixed rations, and also sprouted grains and legumes.

Feed may be obtained from one or more of the plants selected from: corn (maize), alfalfa (Lucerne), barley, birdsfoot trefoil, brassicas, Chau moellier, kale, rapeseed (canola), rutabaga (swede), turnip, clover, alsike clover, red clover, subterranean clover, white clover, fescue, brome, millet, oats, sorghum, soybeans, trees (pollard tree shoots for tree-hay), wheat, and legumes. The feed may be in the form of a solution or as a solid or as a semisolid depending on the use and/or the mode of application and/or the mode of administration.

The feed may be a compound feed. The term "compound feed" means a commercial feed in the form of a meal, a pellet, nuts, cake, a crumble or suchlike. Compound feeds may be blended from various raw materials and additives. These blends are formulated according to the specific requirements of the target animal.

Compound feeds can be complete feeds that provide all the daily required nutrients, concentrates that provide a part of the ration (protein, energy) or supplements that only provide additional micronutrients, such as minerals and vitamins.

The main ingredients used in compound feed are feed grains, which include corn, wheat, canola meal, rapeseed meal, lupin, soybeans, sorghum, oats, and barley.

In one embodiment the feedstuff comprises or consists of corn, DDGS (such as cDDGS), wheat, wheat bran or any combination thereof.

In one embodiment the feed component may be corn, DDGS (e.g. cDDGS), wheat, wheat bran or a combination thereof. In one embodiment the feedstuff comprises or consists of corn, DDGS (such as cDDGS) or a combination thereof.

A feedstuff described herein may contain at least 30 %, at least 40 %, at least 50 % or at least 60 % by weight corn and soybean meal or corn and full fat soy, or wheat meal or sunflower meal.

For example, a feedstuff may contain between about 5 % to about 40 % corn DDGS. For poultry, the feedstuff on average may contain between about 7 % to 15 % corn DDGS. For swine (pigs), the feedstuff may contain on average 5 % to 40 % corn DDGS. It may also contain corn as a single grain, in which case the feedstuff may comprise between about 35 % to about 80 % corn.

In feedstuffs comprising mixed grains, e.g. comprising corn and wheat for example, the feedstuff may comprise at least 10 % corn.

In addition, or in the alternative, a feedstuff also may comprise at least one high-fibre feed material and/or at least one by-product of the at least one high-fibre feed material to provide a high-fibre feedstuff. Examples of high-fibre feed materials include: wheat, barley, rye, oats, by-products from cereals, such as corn gluten meal, corn gluten feed, wet-cake, Distillers Dried Grains (DDG), Distillers Dried Grains with Solubles (DDGS), wheat bran, wheat middlings, wheat shorts, rice bran, rice hulls, oat hulls, palm kernel, and citrus pulp. Some protein sources may also be regarded as high-fibre feed materials: protein obtained from sources such as sunflower, lupin, fava beans and cotton. In one aspect, the feedstuff as described herein comprises at least one high-fibre material and/or at least one by-product of the at least one high-fibre feed material selected from the group consisting of: Distillers Dried Grains with Solubles (DDGS), particularly cDDGS; wet-cake; Distillers Dried Grains (DDG), particularly cDDG; wheat bran; and wheat, for example. In one embodiment the feedstuff of the present invention comprises at least one high-fibre feed material and/or at least one by-product of the at least one high-fibre feed material selected from the group consisting of: Distillers Dried Grains with Solubles (DDGS), particularly cDDGS; wheat bran; and wheat.

The feed may be one or more of the following: a compound feed and premix, including pellets, nuts or (cattle) cake; a crop or crop residue: corn, soybeans, sorghum, oats, barley, copra, straw, chaff, sugar beet waste; fish meal; meat and bone meal; molasses; oil cake and press cake; oligosaccharides; conserved forage plants; silage; seaweed; seeds and grains, either whole or prepared by crushing, milling etc.; sprouted grains and legumes; yeast extract.

Suitably a premix as referred to herein may be a composition composed of microingredients such as vitamins, minerals, chemical preservatives, antibiotics, fermentation products, and other essential ingredients. Premixes are usually compositions suitable for blending into commercial rations. A feed or feed additive composition may be combined with at least one mineral and/or at least one vitamin to form a premix, as defined herein.

The term "feed" as used herein encompasses in some embodiments pet food. A pet food is plant or animal material intended for consumption by pets, such as dog food or cat food. Pet food, such as dog and cat food, may be either in a dry form, such as kibble for dogs, or wet canned form. Cat food may contain the amino acid taurine.

Animal feed also encompasses in some embodiments a fish food. A fish food normally contains macro-nutrients, trace elements and vitamins necessary to keep captive fish in good health. Fish food may be in the form of a flake, pellet or tablet. Pelleted forms, some of which sink rapidly, are often used for larger fish or bottom feeding species. Some fish foods also contain additives, such as beta-carotene or sex hormones, to artificially enhance the colour of ornamental fish.

In still another aspect, animal feed encompasses bird food. Bird food includes food that is used both in bird-feeders and to feed pet birds. Typically, bird food comprises a variety of seeds, but may also comprise suet (beef or mutton fat).

Animal feeds may include plant material such as corn, wheat, sorghum, soybean, canola, sunflower or mixtures of any of these plant materials or plant protein sources for poultry, pigs, ruminants, aquaculture and pets. It is contemplated that animal performance parameters, such as growth, feed intake and feed efficiency will be improved, and also that improved uniformity will be displayed, and a reduced ammonia concentration in the animal house will result, with the consequence of improved welfare and health status of the animals. More specifically, as used herein, "animal performance" may be determined by the feed efficiency and/or weight gain of the animal and/or by the feed conversion ratio and/or by the digestibility of a nutrient in a feed (e.g. amino acid digestibility) and/or digestible energy or metabolizable energy in a feed and/or by nitrogen retention and/or by animals' ability to avoid the negative effects of necrotic enteritis and/or by the immune response of the subject.

Preferably "animal performance" is determined by feed efficiency and/or weight gain of the animal and/or by the feed conversion ratio.

By "improved animal performance" it is meant that there is increased feed efficiency, and/or increased weight gain and/or reduced feed conversion ratio and/or improved digestibility of nutrients or energy in a feed and/or by improved nitrogen retention and/or by improved ability to avoid the negative effects of necrotic enteritis and/or by an improved immune response in the subject resulting from the use of the feed additive composition of the present invention in feed in comparison to feed which does not comprise said feed additive composition.

Preferably, by "improved animal performance" it is meant that there is increased feed efficiency and/or increased weight gain and/or reduced feed conversion ratio. As used herein, the term "feed efficiency" refers to the amount of weight gain in an animal that occurs when the animal is fed *ad libitum* or a specified amount of food during a period of time.

By "increased feed efficiency" it is meant that the use of a feed additive composition according to the present invention in feed results in an increased weight gain per unit of feed intake compared with an animal fed with the same feed but without said feed additive composition being present.

An "increased weight gain" refers to an animal having increased body weight on being fed feed comprising a feed additive composition compared with an animal being fed a feed without said feed additive composition being present.

As used herein, the term "feed conversion ratio" refers to the amount of feed fed to an animal to increase the weight of the animal by a specified amount.

An improved feed conversion ratio means a lower feed conversion ratio, i.e. that less feed must be fed to a certain animal in order for it to gain a certain amount of weight.

By "lower feed conversion ratio" or "improved feed conversion ratio" it is meant that the use of a feed additive composition in feed results in a lower amount of feed being required to be fed to an animal to increase the weight of the animal by a specified amount compared to the amount of feed required to increase the weight of the animal by the same amount when the feed does not comprise said feed additive composition.

Nutrient digestibility as used herein means the fraction of a nutrient that disappears (e.g. is taken up into the bloodstream, degraded etc.) from the gastro-intestinal tract or a specified segment of the gastro-intestinal tract, e.g. the small intestine. Nutrient digestibility may be measured as the difference between what is administered to the subject and what comes out in the faeces of the subject, or between what is administered to the subject and what remains in the digesta on a specified segment of the gastro intestinal tract, e.g. the ileum.

Nutrient digestibility as used herein may be measured by the difference between the intake of a nutrient and the excreted nutrient by means of the total collection of excreta during a period of time; or with the use of an inert marker that is not absorbed by the animal, and allows the researcher calculating the amount of nutrient that disappeared in the entire gastro-intestinal tract or a segment of the gastro-intestinal tract. Such an inert marker may be titanium dioxide, chromic oxide or acid insoluble ash. Digestibility may be expressed as a percentage of the nutrient in the feed, or as mass units of digestible nutrient per mass units of nutrient in the feed.

Nutrient digestibility as used herein encompasses starch digestibility, fat digestibility, protein digestibility, and amino acid digestibility.

"Digestible energy" as used herein means the gross energy of the feed consumed minus the gross energy of the faeces, or the gross energy of the feed consumed minus the gross energy of the remaining digesta on a specified segment of the gastro-intestinal tract of the animal, e.g. the ileum. Metabolizable energy as used herein refers to apparent metabolizable energy and means the gross energy of the feed consumed minus the gross energy contained in the faeces, urine, and gaseous products of digestion. Digestible energy and metabolizable energy may be measured as the difference between the intake of gross energy and the gross energy excreted in the faeces or the digesta present in a specified segment of the gastro-intestinal tract using the same methods to measure the digestibility of nutrients, with appropriate corrections for nitrogen excretion to calculate the metabolizable energy of a feed.

In some embodiments, the feeds and/or feed additive compositions described herein can improve the digestibility or utilization of dietary hemicellulose or fibre in a subject. In some embodiments, the subject is a pig.

Nitrogen retention as used herein means a subject's ability to retain nitrogen from the diet as body mass. A negative nitrogen balance occurs when the excretion of nitrogen exceeds the daily intake and is often seen when muscle is being lost. A positive nitrogen balance is often associated with muscle growth, particularly in growing animals.

Nitrogen retention may be measured as the difference between the intake of nitrogen and the excreted nitrogen by means of the total collection of excreta and urine during a period of time. It is understood that excreted nitrogen includes undigested protein from the feed, endogenous proteinaceous secretions, microbial protein, and urinary nitrogen.

A feed of the invention may contain additional minerals such as, for example, calcium and/or additional vitamins. In some embodiments, the feed is a corn-soybean meal mix.

Feed is typically produced in feed mills, in which raw materials are first ground to a suitable particle size and then mixed with appropriate additives. The feed may then be produced as a mash or pellets: the latter typically involves a method by which the temperature is raised to a target level and then the feed is passed through a die to produce pellets of a particular size. The pellets are allowed to cool. Subsequently, liquid additives such as fat and enzyme solutions may be added. Production of feeds may also involve an additional step that includes extrusion or expansion prior to pelleting, in particular by suitable techniques that may include at least the use of steam.

Feeds as disclosed herein may be for any animal as defined herein.

Thus, in another embodiment, there is disclosed an animal feed, feedstuff, feed additive composition or premix comprising at least one polypeptide having serine protease activity and is thermostable, wherein said polypeptide comprises the amino acid sequence set forth in SEQ ID NO: 3, or an amino acid sequence with at least 92 % sequence identity thereto, and wherein said animal feed, feedstuff, feed additive composition or premix optionally further comprises (a) at least one direct-fed microbial or (b) at least one other enzyme or (c) at least one direct fed microbial and at least one other enzyme.

The term "feed additive composition" refers to an enzyme composition for use in food. This enzyme composition may also comprise (a) at least one direct-fed microbial or (b) at least one other enzyme, i.e., more than one enzyme, or c) at least one direct fed microbial and at least one other enzyme.

A feed additive composition may be admixed with a feed component to form a feedstuff. The term "feed component" as used herein means all or part of the feedstuff. Part of the feedstuff may mean one constituent of the feedstuff or more than one constituent of the feedstuff, e.g. 2 or 3 or 4 or more constituents. In one embodiment the term "feed component" encompasses a premix or premix constituents. The feed may be a compound feed or a premix thereof. A feed additive composition may be admixed with a compound feed, a compound feed component or a premix of a compound feed, or a feed, a feed component, or a premix of a feed.

In some applications, the feed additive compositions may be mixed with feed or administered in drinking water.

The at least one DFM may comprise at least one viable microorganism such as a viable bacterial strain or a viable fungus, such as a viable yeast. Preferably, the DFM comprises at least one viable bacterium.

It is possible that the DFM may comprise a spore-forming bacterial strain and hence the DFM may comprise spores, e.g. bacterial spores. Thus, the term "viable microorganism" as used herein include microbial spores, such as endospores or conidia. Alternatively, the DFM in the feed additive composition described herein may not comprise microbial spores. Thus, the viable microorganism may be a metabolically active microorganism (e.g. a vegetative bacterial cell) or a metabolically inactive microorganism (e.g. an endospore). By "viable" is thus meant a microorganism which is able to grow and reproduce. A spore may be considered viable if it is able to germinate.

The microorganism may be a naturally-occurring microorganism, or it may be a transformed or mutated microorganism, such as a genetically engineered microorganism.

A DFM as described herein may comprise microorganisms from one or more of the following genera: *Lactobacillus, Lactococcus, Streptococcus, Bacillus, Pediococcus, Enterococcus, Leuconostoc, Carnobacterium, Propionibacterium, Bifidobacterium, Clostridium* and *Megasphaera* and combinations thereof.

Preferably, the DFM comprises one or more bacterial strains selected from the following *Bacillus* spp: *B. subtilis, B. cereus, B. licheniformis, B. pumilis* and *B. amyloliquefaciens.*

The genus *"Bacillus",* as used herein, includes all species within the genus "Bacillus," as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. gibsonii, B. pumilis* and *B. thuringiensis.* It is recognized that the genus Bacillus continues to undergo taxonomical reorganisation. Thus, it is intended that the genus include species that have been reclassified, including but not limited to such organisms as *Bacillus stearothermophilus,* which is now named *"Geobacillus stearothermophilus",* or *Bacillus polymyxa,* which is now *"Paenibacillus polymyxa".* The production of resistant endospores under stressful environmental conditions is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*
In another aspect, the DFM may further comprise one or both of the following *Lactococcus* spp: *Lactococcus cremoris* and *Lactococcus lactis.*

The DFM may further comprise one or more of the following *Lactobacillus* spp: *Lactobacillus buchneri, Lactobacillus acidophilus, Lactobacillus casei, Lactobacillus kefiri, Lactobacillus bifidus, Lactobacillus brevis, Lactobacillus helveticus, Lactobacillus paracasei, Lactobacillus rhamnosus, Lactobacillus salivarius, Lactobacillus curvatus, Lactobacillus bulgaricus, Lactobacillus sakei, Lactobacillus reuteri, Lactobacillus fermentum, Lactobacillus farciminis, Lactobacillus lactis, Lactobacillus delbreuckii, Lactobacillus plantarum, Lactobacillus paraplantarum, Lactobacillus farciminis, Lactobacillus rhamnosus, Lactobacillus crispatus, Lactobacillus gasseri, Lactobacillus johnsonii* and *Lactobacillus jensenii,* and combinations of any thereof.

In still another aspect, the DFM may further comprise one or more of the following *Bifidobacteria* spp: *Bifidobacterium lactis, Bifidobacterium bifidium, Bifidobacterium longum, Bifidobacterium animalis, Bifidobacterium breve, Bifidobacterium infantis, Bifidobacterium catenulatum, Bifidobacterium pseudocatenulatum, Bifidobacterium adolescentis,* and *Bifidobacterium angulatum,* and combinations of any thereof.

The DFM may particularly comprise one or more of the following species: *Bacillus subtilis, Bacillus licheniformis, Bacillus amyloliquefaciens, Bacillus pumilis, Enterococcus, Enterococcus* spp, and *Pediococcus* spp, *Lactobacillus* spp, *Bifidobacterium* spp, *Lactobacillus acidophilus, Pediococsus acidilactici, Lactococcus lactis, Bifidobacterium bifidum, Bacillus subtilis, Propionibacterium thoenii, Lactobacillus farciminis, Lactobacillus rhamnosus, Megasphaera elsdenii, Clostridium butyricum, Bifidobacterium animalis* ssp. *animalis, Lactobacillus reuteri, Bacillus cereus, Lactobacillus salivarius* ssp. *Salivarius, Propionibacteria spp.* and combinations thereof.

A direct-fed microbial as described herein may comprise one or more bacterial strains. The DFM may comprise one type of bacterial strain, or one type of bacterial species or one type of bacterial genus. The one type of bacterial species may consist of only a single strain, or may comprise more than one strain. The one type of bacterial genus may consist of a single species, which may comprise one or more strains, or the genus may comprise more than one bacterial species, each of which may comprise one or more strains. Alternatively, the DFM may comprise a mixture of genera, each of which may comprise one or more species and strains.

The DFM may comprise one or more of the products or the microorganisms contained in those products disclosed in WO2012110778, and summarized as follows: *Bacillus subtilis* strain 2084 Accession No. NRRI B-50013, *Bacillus subtilis* strain LSSAO1 Accession No. NRRL B-50104, and *Bacillus subtilis* strain 15A-P4 ATCC Accession No. PTA-6507 (from Enviva Pro®. (formerly known as Avicorr®); *Bacillus subtilis* Strain C3102 (from Calsporin®); *Bacillus subtilis* Strain PB6 (from Clostat®); *Bacillus pumilis* (8G-134); *Enterococcus* NCIMB 10415 (SF68) (from Cylactin®); *Bacillus subtilis* Strain C3102 (from Gallipro® & GalliproMax®); *Bacillus licheniformis* (from Gallipro®Tect®); *Enterococcus* and *Pediococcus* (from Poultry star®); *Lactobacillus, Bifidobacterium* and/or *Enterococcus* from Protexin®); *Bacillus subtilis* strain QST 713 (from Proflora®); *Bacillus amyloliquefaciens* CECT-5940 (from Ecobiol® & Ecobiol® Plus); *Enterococcus faecium* SF68 (from Fortiflora®); *Bacillus subtilis* and *Bacillus licheniformis* (from BioPlus2B®); Lactic acid bacteria 7 *Enterococcus faecium* (from Lactiferm®); *Bacillus* strain (from CSI®); *Saccharomyces cerevisiae* (from Yea-Sacc®); *Enterococcus* (from Biomin IMB52®); *Pediococcus acidilactici, Enterococcus, Bifidobacterium animalis* ssp. *animalis, Lactobacillus reuteri, Lactobacillus salivarius* ssp. *salivarius* (from Biomin C5®); *Lactobacillus farciminis* (from Biacton®); *Enterococcus* (from Oralin E1707®); *Enterococcus* (2 strains), *Lactococcus lactis* DSM 1103(from Probios-pioneer PDFM®); *Lactobacillus rhamnosus and Lactobacillus farciminis* (from Sorbiflore®); *Bacillus subtilis* (from Animavit®); *Enterococcus* (from Bonvital®); *Saccharomyces cerevisiae* (from Levucell SB 20®); *Saccharomyces cerevisiae* (from Levucell SC 0 & SC10® ME); *Pediococcus acidilacti* (from Bactocell); *Saccharomyces cerevisiae* (from ActiSaf® (formerly BioSaf®)); *Saccharomyces cerevisiae* NCYC Sc47 (from Actisaf® SC47); *Clostridium butyricum* (from Miya-Gold®); *Enterococcus* (from Fecinor and Fecinor Plus®); *Saccharomyces cerevisiae* NCYC R-625 (from InteSwine®); *Saccharomyces cerevisia* (from BioSprint®); *Enterococcus* and *Lactobacillus rhamnosus* (from Provita®); *Bacillus subtilis* and *Aspergillus oryzae* (from PepSoyGen-C®); *Bacillus cereus* (from Toyocerin®); *Bacillus cereus* var. *toyoi* NCIMB 40112/CNCM 1-1012 (from TOYOCERIN®), or other DFMs such as *Bacillus licheniformis* and *Bacillus subtilis* (from BioPlus® YC) and *Bacillus subtilis* (from GalliPro®).

The DFM may comprise Enviva® PRO which is commercially available from Danisco A/S. Enviva Pro® is a combination of *Bacillus* strain 2084 Accession No. NRRI B-50013, *Bacillus* strain LSSAO1 Accession No. NRRL B-50104 and *Bacillus* strain 15A-P4 ATCC Accession No. PTA-6507 (as taught in US 7,754,469 B).

The DFM described herein may further comprise a yeast from the genus: *Saccharomyces.*

Preferably, the DFM described herein comprises microorganisms which are generally recognised as safe (GRAS) and, preferably are GRAS-approved. Thus, the microorganisms are preferably non-pathogenic.

A person of ordinary skill in the art will readily be aware of specific species and/or strains of microorganisms from within the genera described herein which are used in the food and/or agricultural industries and which are generally considered suitable for animal consumption.

In some embodiments, it is important that the DFM be heat-tolerant, i.e. is thermotolerant. This is particularly the case when the feed is pelleted. Therefore, in another embodiment, the DFM may comprise one or more thermotolerant microorganisms, such as thermotolerant bacteria, including for example *Bacillus* spp. By thermotolerant "bacteria" is meant bacterial species able to survive at a temperature above physiological temperature (37°C). Preferably, this refers to species able to survive at a temperature of at least 50°C, 60°C, 70°C, 80°C or 90°C. By "able to survive" includes species able to survive in vegetative form at such temperatures, e.g. thermophilic microorganisms, and species which are not able to survive in vegetative form at such temperatures but which are able to sporulate to produce spores able to survive at such temperatures. Such species include *Bacillus* spp. which produce endospores able to survive at such temperatures.

It may be desirable that the DFM comprises spore-producing bacteria, such as *Bacillus* spp. Bacilli are able to form stable endospores when conditions for growth are unfavourable and are very resistant to heat, pH, moisture and disinfectants.

The DFM described herein may decrease or prevent intestinal establishment of pathogenic microorganisms (such as *Clostridium perfringens* and/or *E. coli* and/or *Salmonella* spp. and/or *Campylobacter* spp.). In other words, the DFM may be antipathogenic. The term "antipathogenic" as used herein means the DFM counters an effect (negative effect) of a pathogen.

As described above, the DFM may be any suitable DFM. For example, the "DFM assay" may be used to determine the suitability of a microorganism to be a DFM. The DFM assay as used herein is explained in more detail in US2009/0280090. For avoidance of doubt, the DFM selected as an inhibitory strain (or an antipathogenic DFM) in accordance with the "DFM assay" taught herein is a suitable DFM for use in accordance with the present disclosure, i.e. in the food product according to the present disclosure.

Tubes were seeded each with a representative pathogen (e.g. bacteria) from a representative cluster.

Supernatant from a potential DFM, grown aerobically or anaerobically, is added to the seeded tubes (except for the control to which no supernatant is added) and incubated. After incubation, the optical density (OD) of the control and supernatant treated tubes was measured for each pathogen.

Colonies of (potential DFM) strains that produced a lowered OD compared with the control (which did not contain any supernatant) can then be classified as an inhibitory strain (or an antipathogenic DFM). Thus, The DFM assay as used herein is explained in more detail in US2009/0280090.

Preferably, a representative pathogen used in this DFM assay can be one (or more) of the following: Clostridia, such as *Clostridium perfringens* and/or *Clostridium difficile, E. coli Salmonella* spp and/or *Campylobacter* spp. In one preferred embodiment the assay is conducted with one or more of *Clostridium perfringens* and/or *Clostridium difficile* and/or *E. coli,* preferably *Clostridium perfringens* and/or *Clostridium difficile,* more preferably *Clostridium perfringens.*

Antipathogenic DFMs include one or more of the following bacteria and are described in WO2013029013.:
*Bacillus subtilis* strain 3BP5 Accession No. NRRL B-50510,
*Bacillus amyloliquefaciens* strain 918 ATCC Accession No. NRRL B-50508, and
*Bacillus subtilis* strain 1013 ATCC Accession No. NRRL B-50509.

DFMs may be prepared as culture(s) and carrier(s) (where used) and can be added to a ribbon or paddle mixer and mixed for about 15 minutes, although the timing can be increased or decreased. The components are blended such that a uniform mixture of the cultures and carriers result. The final product is preferably a dry, flowable powder. The DFM(s) comprising one or more bacterial strains can then be added to animal feed or a feed premix, added to an animal's water, or administered in other ways known in the art (preferably simultaneously with the enzymes described herein).

Inclusion of the individual strains in the DFM mixture can be in proportions varying from 1 % to 99 % and, preferably, from 25 % to 75 %.

Suitable dosages of the DFM in animal feed may range from about 1x10³ CFU/g feed to about 1x10¹⁰ CFU/g feed, suitably between about 1x10⁴ CFU/g feed to about 1x10⁸ CFU/g feed, suitably between about 7.5x10⁴ CFU/g feed to about 1x10⁷ CFU/g feed.

In another aspect, the DFM may be dosed in feedstuff at more than about 1x10³ CFU/g feed, suitably more than about 1x10⁴ CFU/g feed, suitably more than about 5x10⁴ CFU/g feed, or suitably more than about 1x10⁵ CFU/g feed.

The DFM may be dosed in a feed additive composition from about 1x10³ CFU/g composition to about 1x10¹³ CFU/g composition, preferably 1x10⁵ CFU/g composition to about 1x10¹³ CFU/g composition, more preferably between about 1x10⁶ CFU/g composition to about 1x10¹² CFU/g composition, and most preferably between about 3.75x10⁷ CFU/g composition to about 1x10¹¹ CFU/g composition. In another aspect, the DFM may be dosed in a feed additive composition at more than about 1x10⁵ CFU/g composition, preferably more than about 1x10⁶ CFU/g composition, and most preferably more than about 3.75x10⁷ CFU/g composition. In one embodiment the DFM is dosed in the feed additive composition at more than about 2x10⁵ CFU/g composition, suitably more than about 2x10⁶ CFU/g composition, suitably more than about 3.75x10⁷ CFU/g composition.

A feed additive composition as described herein, comprising a thermostable serine protease, as defined herein, may be used as, in, or the preparation of a food product, such as a feed.

A feed additive composition as described herein, may also comprise (a) at least one direct-fed microbial or (b) at least one other enzyme, i.e., more than one enzyme, or c) at least one direct fed microbial and at least one other enzyme.

The feed additive composition may further comprise one or more of: a nutritionally acceptable carrier, a nutritionally acceptable diluent, a nutritionally acceptable excipient, a nutritionally acceptable adjuvant and a nutritionally active ingredient. For example, a feed additive composition described herein may comprise at least one component selected from the group consisting of a protein, a peptide, sucrose, lactose, sorbitol, glycerol, propylene glycol, sodium chloride, sodium sulfate, sodium acetate, sodium citrate, sodium formate, sodium sorbate, potassium chloride, potassium sulfate, potassium acetate, potassium citrate, potassium formate, potassium acetate, potassium sorbate, magnesium chloride, magnesium sulfate, magnesium acetate, magnesium citrate, magnesium formate, magnesium sorbate, sodium metabisulfite, methyl paraben and propyl paraben. By "a peptide" is meant a compound comprising a small number of amino acids linked by peptide bonds. For instance, a peptide may contain 30 or fewer, 25 or fewer, 20 or fewer, 15 or fewer, 10 or fewer or 5 or fewer amino acids.

This feed additive composition, may be granulated. By granulated is meant that the food product is provided in a particulate form, i.e. the food product is in the form of particles, e.g. in the form of a powder. The granulated food product may be obtained by processing of a larger bulk of the product (i.e. processing of a non-granular food product) using a process such as high shear granulation, drum granulation, extrusion, spheronization, fluidized bed agglomeration, fluidized bed spray coating, spray drying, freeze drying, prilling, spray chilling, spinning disk atomization, coacervation or tableting, or any combination of the above processes. These processes are well-known to the skilled individual and are commonly performed in the art of food product production, particularly animal feed production.

The feed additive composition described herein, may be formulated into granules as described in WO2007/044968 (referred to as TPT granules).

In certain embodiments, the particles of the granulated food product (e.g. feed additive composition) have a mean diameter of from 50 microns (µm) to 2000 microns, e.g. 100 microns to 1800 microns, 250 microns to 1500 microns, 500 microns to 1500 microns, 800 microns to 1200 microns or about 1000 microns.

When the food additive composition described herein is granulated, the food product may consist of granules comprising a hydrated barrier salt coated over a protein core. The advantage of such salt coating is improved thermo-tolerance, improved storage stability and protection against other feed additives otherwise having an adverse effect on the thermostable serine protease and/or the optional DFM comprising one or more bacterial strains. Preferably, the salt used for the salt coating has a water activity greater than 0.25 or constant humidity greater than 60 % at 20°C. Preferably, the salt coating comprises a Na₂SO₄.

In one embodiment, the feed additive composition described herein is formulated into a granule to provide feed compositions comprising: a core; an active agent; and at least one coating, the active agent of the granule retaining at least 50 %, 60 %, 70 % or 80 % activity after experiencing conditions selected from one or more of: a) a feed pelleting process, b) a steam-heated feed pretreatment process, c) storage, d) storage as an ingredient in an unpelleted mixture, and e) storage as an ingredient in a feed base mix or a feed premix comprising at least one compound selected from trace minerals, organic acids, reducing sugars, vitamins, choline chloride, and compounds which result in an acidic or a basic feed base mix or feed premix.

With regard to the granule, the at least one coating may comprise a moisture hydrating material that constitutes at least 55 % w/w of the granule; and/or the at least one coating may comprise two coatings. Said two coatings may be a moisture hydrating coating and a moisture barrier coating. In some embodiments, the moisture hydrating coating may constitute between 25 % and 60 % w/w of the granule and the moisture barrier coating may constitute between 2 % and 15 % w/w of the granule. The moisture hydrating coating may be selected from inorganic salts, sucrose, starch, and maltodextrin and the moisture barrier coating may be selected from polymers, gums, whey and starch.

The granule may be produced using a feed pelleting process, which may be conducted between 70°C and 95°C, such as between 85°C and 95°C, for up to several minutes.

The feed additive composition may be formulated into a granule for animal feed comprising: a core; an active agent, the active agent retaining at least 80 % activity after storage and after a steam-heated pelleting process where the granule is an ingredient; a moisture barrier coating; and a moisture hydrating coating that constitutes at least 25 % w/w of the granule, the granule having a water activity of less than 0.5 prior to the steam-heated pelleting process.

The granule may have a moisture barrier coating selected from polymers and gums and the moisture hydrating material may be an inorganic salt. The moisture hydrating coating may constitute between 25 % and 45 % w/w of the granule, and the moisture barrier coating may constitute between 2 % and 10 % w/w of the granule.

The granule may be produced using a steam-heated pelleting process which may be conducted at between 85°C and 95°C for up to several minutes.

The food product of the invention, e.g. a feed additive composition or a feed comprising a feed additive composition, may be used in any suitable form. Such a food product (e.g. a feed additive composition) may be used in the form of solid or liquid preparations or alternatives thereof. Examples of solid preparations include powders (including dry powders), pastes, boluses, capsules, pellets, tablets, dusts, and granules which may be wettable, spray-dried or freeze-dried. Examples of liquid preparations include, but are not limited to, aqueous, organic or aqueous-organic solutions, suspensions and emulsions. Such food products may be coated, e.g. encapsulated, for instance to protect the enzymes of the food product from heat. A coating may thus be considered a thermo-protectant.

Dry powder or granules may be prepared by any means known to those skilled in the art, such as high shear granulation, drum granulation, extrusion, spheronization, fluidized bed agglomeration and fluidized bed spray.

A feed additive composition may be powdered, as described above. The powder may further be pelleted. In the pelleting process, the powder may be mixed with other components known in the art, forced through a die and the resulting strands cut into suitable pellets of variable length.

Optionally, the pelleting step may include a steam treatment, or conditioning stage, prior to formation of the pellets. The mixture comprising the powder may be placed in a conditioner, e.g. a mixer with steam injection. The mixture is heated in the conditioner up to a specified temperature, such as from 60-100°C, typical temperatures would be 70°C, 80°C, 85°C, 90°C or 95°C. The residence time can be variable from seconds to minutes and even hours, e.g. 5 seconds, 10 seconds, 15 seconds, 30 seconds, 1 minute, 2 minutes, 5 minutes, 10 minutes, 15 minutes, 30 minutes or 1 hour. It will be understood that the thermostable serine proteases (or composition comprising the thermostable serine proteases) described herein are suitable for addition to any appropriate feed material.

Alternatively, a feed additive composition, may be formed by applying, e.g. spraying, the thermostable serine protease onto a carrier substrate, such as ground wheat for example.

When the. feed additive composition is in the form of a liquid, preferably the liquid form is suitable for spray-drying on a feed pellet.

When the feed additive composition is in the form of a liquid it may be in a formulation suitable for consumption, e.g. containing one or more of the following: a buffer, salt, sorbitol and/or glycerol.

In one embodiment, the thermostable serine protease, and optionally a DFM, are formulated with at least one physiologically acceptable carrier selected from at least one of maltodextrin, limestone (calcium carbonate), cyclodextrin, wheat or a wheat component, sucrose, starch, Na₂SO₄, Talc, PVA, sorbitol, benzoate, sorbate, glycerol, sucrose, propylene glycol, 1,3-propane diol, glucose, parabens, sodium chloride, citrate, acetate, phosphate, calcium, metabisulfite, formate and mixtures thereof.

In some embodiments, the thermostable serine protease as defined herein may be present in an animal feed product of the invention (e.g. a feed) at a concentration in the range of 1 ppb (part per billion) to 10 % (w/w) based on pure enzyme protein. In some embodiments, the protease is present in the feedstuff in the range of from 1-100 ppm (parts per million). A preferred dose can be 1-20 g of thermostable serine protease per tonne (by which is meant a metric tonne of 1000 kg) of feed product or feed composition, e.g. 1-18 g/tonne, 2-18 g/tonne, 5-15 g/tonne, 1-10 g/tonne, 10-20 g/tonne or 8-12 g/tonne, or a final dose of 1-20 ppm thermostable serine protease in final product, e.g. 1-18 ppm, 2-18 ppm, 5-15 ppm, 1-10 ppm, 10-20 ppm or 8-12 ppm.

Preferably, the thermostable serine protease is present in the feed with an activity of at least about 200 PU/kg, 300 PU/kg, 400 PU/kg, 500 PU/kg, 600 PU/kg, 700 PU/kg, 800 PU/kg, 900 PU/kg, 1000 PU/kg, 1500 PU/kg, 2000 PU/kg, 2500 PU/kg, 3000 PU/kg, 3500 PU/kg, 4000 PU/kg, 4500 PU/kg, or 5000 PU/kg feed.

In another aspect, the thermostable serine protease can be present in the feedstuff at less than about 60,000 PU/kg, 70,000 PU/kg, 80,000 PU/kg, 90,000 PU/kg, 100,000 PU/kg, 200,000 PU/kg, 300,000 PU/kg, 400,000 PU/kg, 500,000 PU/kg, 600,000 PU/kg or 700,000 PU/kg feed.

Ranges can include, but are not limited to, any combination of the lower and upper ranges discussed above.

It will be understood that one protease unit (PU) is the amount of enzyme that liberates 2.3 micrograms of phenolic compound (expressed as tyrosine equivalents) from a casein substrate per minute at pH 10.0 at 50°C. This may be referred to as the assay for determining 1 PU. The skilled person is well able to perform such an assay and to determine the number of PU present in a sample.

In another aspect, there is disclosed a method for hydrolyzing a material derived from corn, said method comprising:
(a) contacting the material obtained from corn with a liquid to form a mash; and
(b) hydrolyzing at least one protein in the mash to form a hydrolysate by contacting the hydrolysate with an enzyme cocktail comprising a thermostable serine protease comprises the amino acid sequence set forth in SEQ ID NO: 3, or an amino acid sequence having at least 92% sequence identity to SEQ ID NO:3 and
(c) optionally, recovering the hydrolysate of obtained in step (b).

Starch or amylum is a polymeric carbohydrate consisting of a large number of glucose units joined by glycosidic bonds. Starch can be hydrolyzed into simpler carbohydrates by acids, various enzymes or a combination of the two.

In industry, starch is converted into sugars, for example by malting, and fermented to produce ethanol in the manufacture of beer, whiskey and biofuel.

Any suitable starch-containing material may be used. The starting material is generally selected based on the desired fermentation product. Examples of starch-containing materials, include but are not limited to, whole grains, corn, wheat, barley, rye, milo, sago, cassava, tapioca, sorghum, rice, peas, beans, or mixtures thereof or starches derived therefrom, or cereals. The starting material can be a dry solid, such as but not limiting to a dry solid of a feed or feedstock as described herein. Contemplated are also waxy and non-waxy types or corn and barley. Starch-containing materials used for ethanol production is corn or wheat. Starch is initially collected from plant grains using either a wet milling, a dry milling or a dry grind process.

A substrate comprising plant material is reduced or milled by methods known in the art. Plant material can be obtained from: wheat, corn, rye, sorghum (milo), rice, millet, barley, triticale, cassava (tapioca), potato, sweet potato, sugar beets, sugarcane, and legumes such as soybean and peas. Preferred plant material includes corn, barley, wheat, rice, milo and combinations thereof. Plant material can include hybrid varieties and genetically modified varieties (e.g. transgenic corn, barley or soybeans comprising heterologous genes).

Any part of the plant containing starch can be used to produce the liquefact, including but not limited to, plant parts such as leaves, stems, hulls, husks, tubers, cobs, grains and the like. Preferred whole grains include corn, wheat, rye, barley, sorghum and combinations thereof. In other embodiments, starch can be obtained from fractionated cereal grains including fiber, endosperm and/or germ components. Methods for fractionating plant material, such as corn and wheat, are known in the art. In some embodiments, plant material obtained from different sources can be mixed together (e.g. corn and milo or corn and barley). Methods of milling are well known in the art and reference is made TO THE ALCOHOL TEXTBOOK: A REFERENCE FOR THE BEVERAGE, FUEL AND INDUSTRIAL ALCOHOL INDUSTRIES 3rd ED. K.A. Jacques et al., Eds, (1999) Nottingham University Press. See, Chapters 2 and 4.

In some embodiments, the plant material, whether reduced by milling or other means, will be combined with a solution resulting in a slurry comprising starch substrate. In some embodiments, the slurry can include a side stream from starch processing such as backset. In some embodiments, the slurry will comprise 15 - 55% ds (e.g., 20 - 50%, 25 - 45%, 25 - 40%, and 20 - 35%). In some embodiments the slurry can comprise 10% to 60% of backset. The slurry comprising the reduced plant material can be subject to a liquefaction process wherein an alpha amylase can be added during the liquefaction step. This results in a liquefact. To produce the liquefact, a single or split dose of an alpha amylase can be added to the slurry. One skilled in the art can readily determine the effective dosage of alpha amylase to be used in the liquefaction processes.

The amount of alpha amylase used for liquefaction is an amount effective to cause liquefaction of a majority of the starch. In other embodiments, the amount is effective to enable liquefaction of greater than 40% of the starch, including 50%, 60%, 70%, 80%, 90%, and 100%. In some embodiments, the range will be 0.05 to 50 AAU/g ds (alpha-amylase units per gram of dry solids (e.g., feed or feedstock, such as corn), also 0.1 to 20 AAU/gDS and also 1.0 to 10 AAU/gDS. In further embodiments, the alpha amylase dosage will be in the range of 0.01 to 10.0 kg/metric ton (MT)ds; also 0.05 to 5.0 kg/MT ds; and also 0.1 to 4.0 kg/MT ds.

An alpha amylase can be added at a temperature of 0 to 30°C below the starch gelatinization temperature of the granular starch of the reduced plant material. This temperature can be 0 to 25°C, 0 to 20°C, 0 to 15°C and 0 to 10°C below the starch gelatinization temperature. This specific value will vary and depends on the type of grain comprising the slurry. For example, the starch gelatinization temperature of corn is generally higher than the starch gelatinization temperature of rye or wheat. In some embodiments, the temperature will be between 45 to 80°C, also between 50 to 75°C, also between 50 to 72°C and in some embodiments the temperature will be below 68°C; below 65°C, below 62°C, below 60°C and also below 55°C. In other embodiments the temperature will be above 40°C, above 45°C, above 50°C, and above 55°C. In some preferred embodiments, the temperature of the incubation will be between 58 to 72°C and also between 60 to 68°C.

In some embodiments, the slurry will be maintained at a pH range of about 3.0 to less than 6.5, also at a pH range of 4.0 to less than 6.2, also at a pH range of about 4.5 to less than 6.0 and preferably at a pH range of about 5.0 to 6.0 (e.g. about 5.4 to 5.8), and the milled grain in the slurry will be contacted with the enzyme composition for a period of time of 2 minutes to 8 hours (e.g., 5 mins to 3 hrs; 15 mins to 2.5 hrs and 30 min to 2 hrs). In a further step the incubated substrate will be liquefied by exposing the incubated substrate to an increase in temperature such as 0 to 55°C above the starch gelatinization temperature. (*e.g.* to 65°C to 120°C, 70°C to 110°C, 70°C to 90°C) for a period of time of 2 minutes to 8 hours (e.g., 2 minutes to 6 hrs, 5 minutes to 4 hours and preferably 1hr to 2 hrs) at a pH of about 4.0 to 6.5. In some embodiments, the temperature can be raised to a temperature to between about 85-90°C and a single dose of alpha amylase can be used. If the temperature is raised above 90-105°C, a second dose of alpha amylase can be added after the temperature returns to normal. In a further embodiment, the temperature can be raised to between about 105 and 140°C and a split dose of alpha amylase can be used with one part being added before raising the temperature and the other part added after the temperature has been brought down to at least below 105°C, including below 104, 103, 102, 101, 100, 99, 98, 97, 96, 95, 94, 93, 92, and 91°C, but preferably below 90°C. In some embodiments, the resulting liquefact is cooled before saccharification.

The liquefact obtained above can be contacted with a glucoamylase, an acid stable alpha amylase, and an acid fungal protease (such as FERMGEN™, DuPont or AP1 from CTE Global) in a single dose or a split dose as long as a desired ratio of enzymes is maintained. Thus, a split dose means that the total dose in desired ratio is added in more than one portion, including two portions or three portions. In one embodiment, one portion of the total dose is added at the beginning and a second portion is added at a specified time in the process. In one embodiment, at least a portion of the dose is added at the beginning of the saccharification (or SSF) to begin the saccharification process. In one embodiment, each enzyme in the enzyme composition can be added to the liquefact separately, but simultaneously or close enough in time such that the activity ratio is maintained. Alternatively, the enzyme blend composition comprising a glucoamylase, an acid stable alpha amylase, and an acid fungal protease can be added during one or both of the saccharification and fermentation. The ratio of the glucoamylase, an acid stable alpha amylase, and an acid fungal protease is preferably about 1:1.5:0.1 to about 1:8:1, and more preferably about 1:2:0.2 to 1:5: 0.6, as measured by GAU:SSU:SAPU.

The saccharification or SSF process typically comprises the addition of urea or ammonia used as a nitrogen source for the host organism (such as but not limiting to Yeast). In commercial ethanol plants, the addition of the nitrogen source (such as urea) during saccharification or SSF typically ranges between 200-1000 ppm, such as at least 200, 300, 400, 500, 600, 700, 800, 900 up to 1000 ppm urea. Another rich nitrogen source in an ethanol plant are the corn proteins present in the corn kernel. This nitrogen source is made available for the fermenting organism when it is hydrolyzed into smaller fragments like small peptides and amino acids.

It has been found, surprisingly and unexpectedly, that when a thermostable serine protease as described herein is present during the liquefying step of a method for producing fermentation products from starch containing materials, the need for adding a nitrogen source, such as urea, during the saccharification or SSF process is reduced by at least 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, or 99% or completely eliminated (100% reduction). This indicates that a thermostable serine protease as described herein can hydrolyze corn proteins to such an extent that it enables the fermenting organism to use corn proteins as a nitrogen source. Consequently, less urea or no urea at all can be added to the SSF. The strong reduction or elimination of a nitrogen source enables the plant to run at a lower cost.

What is described is a method for producing fermentation products from starch-containing material comprising:
(a) liquefying the starch-containing material with an enzyme cocktail comprising a serine protease comprising the amino acid sequence set forth in SEQ ID NO: 3, or an amino acid sequence having at least 92% sequence identity to SEQ ID NO:3;
(b) saccharifying the product of step (a);
(c) fermenting with a suitable organism; and
(d) optionally, recovering the product produced in step (c).

Thus, as discussed above, steps (b) and (c) can be performed simultaneously.

Moreover, the addition of a nitrogen source, such as urea, is eliminated or reduced by at least 50% by using 1-20 g serine protease/MT starch-containing material wherein the serine protease comprises the amino acid sequence set forth in SEQ ID NO: 3, or an amino acid sequence having at least 92% sequence identity to SEQ ID NO:3.

In addition, when the liquefaction product is ethanol then no acid proteolytic enzyme is needed when using 1-20 g thermostable serine protease/MT starch-containing material wherein the serine protease comprises the amino acid sequence set forth in SEQ ID NO: 3, or an amino acid sequence having at least 92% sequence identity to SEQ ID NO:3.

In other words, this appears to indicate that the presence of a protease other than the thermostable serine protease described herein in the fermentation process may not be needed.

The saccharification process can last for 12 to 120 hours. However, it is common to perform a saccharification for 30 minutes to 2 hours and then complete the saccharification during fermentation. Sometimes this is referred to as simultaneous saccharification and fermentation (SSF). Saccharification is commonly carried out at temperatures of 30 to 65°C and typically at pH of 3.0 to 5.0, including 4.0 to 5.0. The saccharification can result in the production of fermentable sugars.

In some embodiments the fermentable sugars are subjected to fermentation with fermenting microorganisms. The contacting step and the fermenting step can be performed simultaneously in the same reaction vessel or sequentially. In general, fermentation processes are described in The Alcohol Textbook 3rd ED, A Reference for the Beverage, Fuel and Industrial Alcohol Industries, Eds Jacques et al., (1999) Nottingham University Press, UK.

In some embodiments, the method further comprises using the fermentable sugars (dextrin e.g. glucose) as a fermentation feedstock in microbial fermentations under suitable fermentation conditions to obtain end-products, such as alcohol (e.g., ethanol), organic acids (e.g., succinic acid, lactic acid), sugar alcohols (e.g., glycerol), ascorbic acid intermediates (e.g., gluconate, DKG, KLG) amino acids (e.g., lysine), proteins (e.g., antibodies and fragment thereof).

The fermentable sugars can be fermented with a yeast at temperatures in the range of 15 to 40°C, 20 to 38°C, and also 25 to 35°C; at a pH range of pH 3.0 to 6.5; also pH 3.0 to 6.0; pH 3.0 to 5.5, pH 3.5 to 5.0 and also pH 3.5 to 4.5 for a period of time of 5 hrs to 120 hours, preferably 12 to 120 and more preferably from 24 to 90 hours to produce an alcohol product, preferably ethanol.

Yeast cells are generally supplied in amounts of 10⁴ to 10¹², and preferably from 10⁷ to 10¹⁰ viable yeast count per ml of fermentation broth. The fermentation will include in addition to a fermenting microorganism (e.g. yeast) nutrients, optionally acid and additional enzymes. In some embodiments, in addition to the raw materials described above, fermentation media will contain supplements including but not limited to vitamins (e.g. biotin, folic acid, nicotinic acid, riboflavin), cofactors, and macro and micro-nutrients and salts (e.g. (NH4)₂SO₄; K₂HPO₄; NaCl; MgSO₄; H₃BO₃, ZnCl₂; and CaCl₂).

In some preferred embodiments, the milled plant material includes barley, milo, corn and combinations thereof, and the contacting and fermenting steps are conducted simultaneously at a pH range of 3.5 to 5.5, a temperature range of 30 - 45°C, and for a period of time of 48 to 90 hrs, wherein at least 50% of the starch is solubilized.

One end product of a fermentation process can be an alcohol product, e.g. ethanol. Other end products can be the fermentation co-products such as distillers dried grains (DDG) and distiller's dried grain plus solubles (DDGS), which can be used as an animal feed.

Use of an appropriate fermenting microorganism as known in the art, the fermentation end products can include without limitation glycerol, 1,3-propanediol, gluconate, 2-keto-D-gluconate, 2,5-diketo-D-gluconate, 2-keto-L-gulonic acid, succinic acid, lactic acid, amino acids and derivatives thereof.

Examples of fermenting organisms are ethanologenic microorganisms or ethanol producing microorganisms such as ethanologenic bacteria which express alcohol dehydrogenase and pyruvate dehydrogenase and which can be obtained from *Zymomonas moblis* (See e.g. USP 5,000,000; USP 5,028,539, USP 5,424,202; USP 5,514,583 and USP 5,554,520). In additional embodiments, the ethanologenic microorganisms express xylose reductase and xylitol dehydrogenase, enzymes that convert xylose to xylulose. In further embodiments, xylose isomerase is used to convert xylose to xylulose. In particularly preferred embodiments, a microorganism capable of fermenting both pentoses and hexoses to ethanol are utilized. For example, in some embodiments the microorganism can be a natural or non-genetically engineered microorganism or in other embodiments the microorganism can be a recombinant microorganism.

The fermenting microorganisms include, but not limited to, bacterial strains from *Bacillus, Lactobacillus, E. coli, Erwinia, Pantoea* (e.g., P. citrea), *Pseudomonas* and *Klebsiella* (e.g. *K. oxytoca*)*.* (See e.g. USP 5,028,539, USP 5,424,202 and WO 95/13362). Bacillus is a preferred fermenting microorganism. The fermenting microorganism used in the fermenting step will depend on the end product to be produced.

The ethanol-producing microorganism can be a fungal microorganism, such as Trichoderma, a yeast and specifically Saccharomyces such as strains of S. *cerevisiae* (USP 4,316,956). A variety of S. *cerevisiae* are commercially available and these include but are not limited to FALI (Fleischmann's Yeast), SUPERSTART (Alltech), FERMIOL (DSM Specialties), (Ethanol Red, Fermentis, France), (SYNERXIA® Thrive, Dupont), RED STAR (Lesaffre) and Angel alcohol yeast (Angel Yeast Company, China).

For example, when lactic acid is the desired end product, a *Lactobacillus* sp. (L. *casei*) can be used; when glycerol or 1,3-propanediol are the desired end-products, *E. coli* can be used; and when 2-keto-D-gluconate, 2,5-diketo-D-gluconate, and 2-keto-L-gulonic acid are the desired end products, *Pantoea citrea* can be used as the fermenting microorganism. The above enumerated list is only exemplary and one skilled in the art will be aware of a number of fermenting microorganisms that can be appropriately used to obtain a desired end product.

The end product produced can be separated and/or purified from the fermentation media. Methods for separation and purification are known, for example by subjecting the media to extraction, distillation and column chromatography. In some embodiments, the end product is identified directly by submitting the media to high-pressure liquid chromatography (HPLC) analysis.

### EXAMPLES

Unless defined otherwise herein, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 2D ED., John Wiley and Sons, New York (1994), and Hale & Marham, THE HARPER COLLINS DICTIONARY OF BIOLOGY, Harper Perennial, N.Y. (1991) provide one of skill with a general dictionary of many of the terms used with this disclosure.

The disclosure is further defined in the following Examples. It should be understood that the Examples, while indicating certain embodiments, is given by way of illustration only.

### EXAMPLE 1

### Analysis of TnaProl Sequence

The nucleotide sequence of the full-length *TnaPro1* gene, as identified in the NCBI database (*Thermococcus nautili,* NCBI Reference Sequence: NZ_CP007264.1 from 1327825-1329105, complementary), is provided in SEQ ID NO: 1. The corresponding full-length protein sequence encoded by the *TnaPro1* gene is shown in SEQ ID NO: 2 (GenBank accession number: AHL23118.1).

The propeptide and mature regions of the TnaPro1 protein were predicted based on a protein sequence alignment with a homologous enzyme, the *Thermococcus kodakaraensis* KOD1 TK-subtilisin protein (Pulido et al. (2006) Applied and Environmental Microbiology, 72(6): 4154-4162). Using SignalP v4.0 (Nordahl Petersen et al. (2011) Nature Methods 8:785-786) software, the TnaPro1 signal peptide was predicted to have the sequence set forth in SEQ ID NO: 10 and the pro-enzyme was predicted to have the sequence set forth in SEQ ID NO: 8. Based on the alignment with *T. kodakaraensis* KOD1, the TnaPro1 propeptide N-terminus was predicted.

The *TnaPro1* gene encoding the signal peptide is set forth in SEQ ID NO: 9 and the section encoding the pro-enzyme is set forth in SEQ ID NO: 7. The section of the *TnaPro1* gene encoding the propeptide fragment is set forth in SEQ ID NO: 11 and the section encoding the mature TnaPro1 is set forth in SEQ ID NO: 6.

### EXAMPLE 2

### Cloning of TnaPro1

The DNA sequence encoding the TnaPro1 pro-enzyme was synthesised and inserted into the *Bacillus subtilis* expression vector p2JM103BBI (Vogtentanz, Protein Expr Purif, 55: 40-52, 2007) yielding a plasmid named pGX706 (aprE-TnaPro1) (Figure 1).

Ligation of the gene encoding the TnaPro1 protein into the digested vector resulted in the addition of three codons (encoding Ala-Gly-Lys) between the 3' end of the *Bacillus subtilis* AprE signal sequence and the 5' end of the predicted TnaPro1 native pro-peptide. The gene has an alternative start codon (GTG). As shown in Figure 1, pGX706 (aprE-TnaPro1) contains an AprE promoter, an AprE signal sequence used to direct target protein secretion in *B. subtilis,* and the synthetic nucleotide sequence encoding the predicted TnaPro1 pro-enzyme. The gene sequence of the *aprE-TnaPro1* construct, including the three additional codons introduced during ligation of the fused sequences, is set forth in SEQ ID NO: 4. The amino acid sequence of the AprE-TnaPro1 fusion protein, including the three additional amino acids encoded by the three additional codons, is set forth in SEQ ID NO: 5.

### EXAMPLE 3

### Expression and Purification of TnaPro1

The pGX706 plasmid was transformed into a suitable *B. subtilis* strain and the transformed cells spread onto Luria Agar plates supplemented with 5 ppm chloramphenicol. Colonies were selected and subjected to fermentation in a 250 mL shake flask with a MOPS based defined medium.

The culture supernatant was obtained by centrifugation of the shake flask cultures, concentrated and subsequently incubated at 85°C for 30 min. For purification, the resulting solution was supplemented with ammonium sulfate to a final concentration of 1 M, and then loaded onto a HiPrep™ Phenyl FF column pre-equilibrated with 20 mM Tris (pH 8.0) supplemented with additional 1 M ammonium sulfate. The target protein was eluted from the column with 0.5 M ammonium sulfate. The resulting active protein fractions were then pooled and concentrated using 10K Amicon Ultra devices, and stored in 40 % glycerol at - 20°C until usage.

A sample of purified TnaPro1 protein was subjected to LC-mass spec and the results confirm the mature TnaPro1 protein as the sequence set forth in SEQ ID NO: 3, and the propeptide sequence as set for in SEQ ID NO: 12.

### EXAMPLE 4

### Proteolytic Activity of Purified TnaPro1

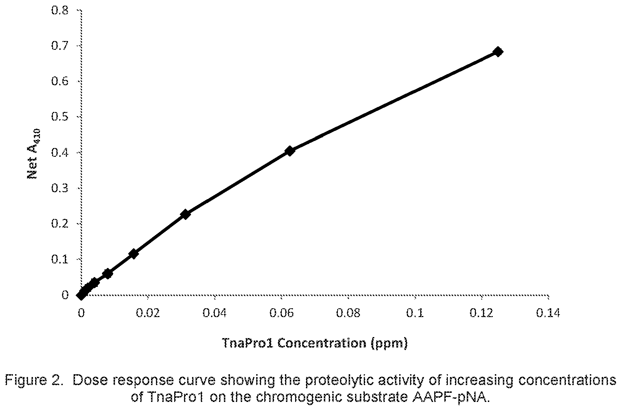

The proteolytic activity of purified TnaPro1 was measured in 50 mM HEPES buffer (pH 8), using Suc-Ala-Ala-Pro-Phe-pNA (AAPF-pNA, Cat# L-1400.0250, BACHEM) as a substrate. Prior to the reaction, the enzyme sample was serially diluted in water to 0.01-1.25 ppm. The AAPF-pNA was dissolved in dimethyl sulfoxide (DMSO, Cat# STBD2470V, Sigma) to a final concentration of 10 mM. To initiate the reaction, firstly 5 µL of AAPF-pNA was mixed with 85 µL of HEPES buffer in a non-binding 96-well microtiter plate (96-MTP) (Cat#3641, Corning Life Sciences) and incubated at 40°C for 5 min at 600 rpm in a Thermomixer (Eppendorf), then 10 µL of the diluted enzyme (or water alone as the blank control) was added. After 10 min incubation in a Thermomixer at 40°C and 600 rpm, the reaction plate was directly read at 410 nm using a SpectraMax 190. Net A₄₁₀ was calculated by subtracting the A₄₁₀ of the blank control from that of the wells containing enzyme, and then plotted against different protein concentrations (from 0.001 ppm to 0.125 ppm). The results are shown in Figure 2. Each value was the mean of triplicate assays. The proteolytic activity is shown as Net A₄₁₀. The proteolytic assay with AAPF-pNA as the substrate indicates that TnaPro1 is an active protease.

### EXAMPLE 5

### pH Profile of Purified TnaPro1

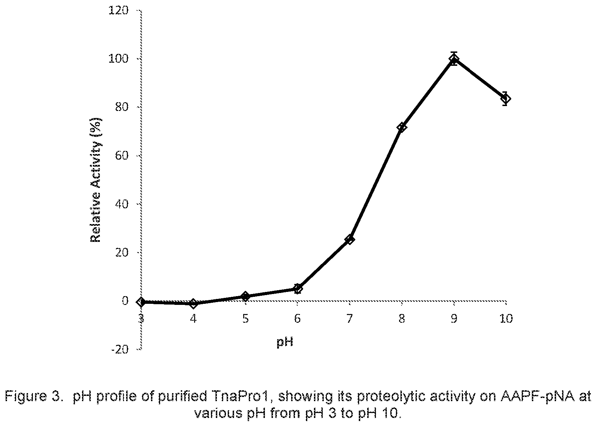

With AAPF-pNA as the substrate, the pH profile of TnaPro1 was studied in 25 mM glycine/sodium acetate/HEPES buffer with different pH values (ranging from pH 3.0 to 10.0). Prior to the assay, 85 µL of 25 mM glycine/sodium acetate/HEPES buffer with a specific pH value was first mixed with 5 µL of 10 mM AAPF-pNA (dissolved in DMSO) in a 96-MTP, and then 10 µL of diluted enzyme (0.5 ppm in water or water alone as the blank control) was added. The reaction was performed and analysed as described above. Enzyme activity at each pH was reported as the relative activity, where the activity at the optimal pH was set to be 100 %. The pH values tested were 3, 4, 5, 6, 7, 8, 9 and 10. Each value was the mean of triplicate assays. As shown in Figure 3, under the conditions of this test, the optimal pH of TnaPro1 is 9.0, with greater than 70 % of maximal activity retained between pH 8 and 10.

### EXAMPLE 6

### Temperature Profile of Purified TnaPro1

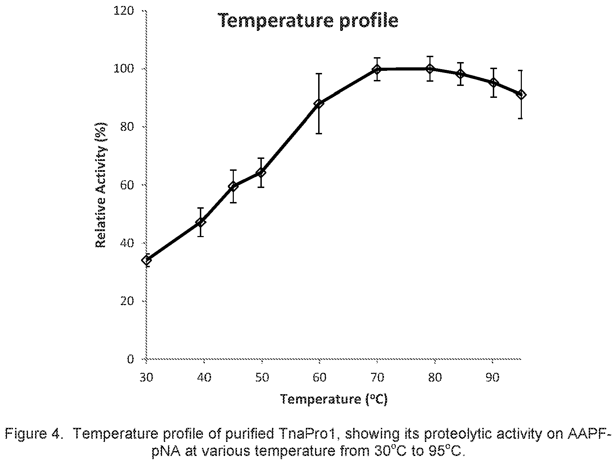

The temperature profile of purified TnaPro1 was analyzed in 50 mM HEPES buffer (pH 8) using the AAPF-pNA assay. Prior to the reaction, 85 µL of 50 mM pH 8.0 HEPES buffer and 5 µL of 10 mM AAPF-pNA were added to 200 µL PCR tubes, which were subsequently incubated in Peltier Thermal Cyclers (BioRad) at desired temperatures (i.e. 30∼95°C) for 5 min. After the incubation, 10 µL of diluted enzyme (0.4 ppm in water, or water alone as the blank control) was added to the substrate to initiate the reaction. Following 10 min incubation in the thermocyclers at different temperatures, 80 µL of the reaction mixture was transferred to a new 96-MTP and the absorbance was read at 410 nm. The activity was reported as relative activity where the activity at the optimal temperature was set to be 100%. The tested temperatures were 30.0°C, 39.4°C, 45.0°C, 49.8°C, 59.9°C, 70.0°C, 79.1°C, 84.4°C, 90.2°C and 95.0°C, and the results are presented in Figure 4. Each value was the mean of triplicate assays.

The data in Figure 4 shows that 70°C was the optimal temperature of TnaPro1, and that it retained more than 80% of its maximal activity between 60°C and 95°C.

### EXAMPLE 7

### Thermostability of Purified TnaPro1

Thermostability analyses of purified TnaPro1 and the commercially available RONOZYME® ProAct (RONOZYME® ProAct, DSM) proteases were performed using 20 mM Tris buffer (pH 8, with 150 mM NaCl) and 50 mM sodium acetate buffer (pH 3) as incubation buffers, respectively, and with AAPF-pNA as the substrate for measurement of remaining activity. The purified TnaPro1 was diluted in 1 mL incubation buffer to a final concentration of 24 ppm and subsequently incubated at 90°C or 95°C for 0, 5, 15, 30 or 60 min, while ProAct protease was diluted to 500 ppm and incubated at 90°C for 30 sec or 60 sec. At the end of each incubation period, a 50 µL aliquot of each enzyme-buffer mixture was transferred to a 96-MTP and placed on ice. To initiate the enzymatic reaction, 85 µL of 50 mM pH 8.0 HEPES buffer was mixed with 5 µL of 10 mM AAPF-pNA and 10 µL of each diluted incubation solution (40 times diluted in water for TnaPro1 and 250 times diluted in water for ProAct, respectively) in a 96-microtiter plate (MTP). Following a 10 min incubation in a thermomixer at 40°C and 600 rpm, the absorbance was measured at 410 nm on a 96 well spectrophotometer. The enzyme activity of each sample was reported as the percent residual activity, where the activity at 0 min incubation time was set to 100%. The thermostability results for TnaPro1 are summarized in Table 1. ProAct was completely deactivated after 1 min incubation at 90°C (data not shown).

| **Table 1.** Thermostability of TnaPro1 protease at 90 and 95°C measured with AAPF-pNA substrate | | | | | | | |
|---|---|---|---|---|---|---|---|
| **Residual activity (%)** | | | | | | | |
| **90 °C** | | | | **95 °C** | | | |
| 5 min | 15 min | 30 min | 60 min | 5 min | 15 min | 30 min | 60 min |
| 89 | 83 | 80 | 78 | 71 | 53 | 40 | 21 |

### EXAMPLE 8

### Corn Soy Meal Hydrolysis Analyses of Purified TnaPro1

The extent of corn soy meal (having 60% corn flour and 40% soybean meal) hydrolysis by purified TnaPro1 was evaluated using the OPA (o-Phthalaldehyde) or the BCA (bicinchoninic acid) detection assays described below, to measure the amount of newly produced N-terminal amine groups or soluble peptides, respectively, released into the supernatant after the enzymatic hydrolysis. To conduct the assays, 140 µL of corn soy meal substrate (10 % w/w in 50 mM sodium acetate pH 3 buffer or MES pH 6 buffer) (Yu S, Cowieson A, Gilbert C, Plumstead P, Dalsgaard S., Interactions of phytate and myo-inositol phosphate esters (IP1-5) including IP5 isomers with dietary protein and iron and inhibition of pepsin. J. Anim. Sci. 2012, 90:1824-1832) was mixed with 20 µL of a diluted TnaPro1 enzyme sample (4.0 mg/mL) in a 96-MTP. After incubation for 2 hrs at 40°C in an incubator, the plates were centrifuged at 3700 rpm for 15 min at 4°C. The resulting supernatant was diluted 10 times in water to prepare for subsequent reaction product detection using the OPA and BCA assays. A sample of ProAct protease (RONOZYME® ProAct, DSM) was included for comparison. Water was used as the (no enzyme) blank control.

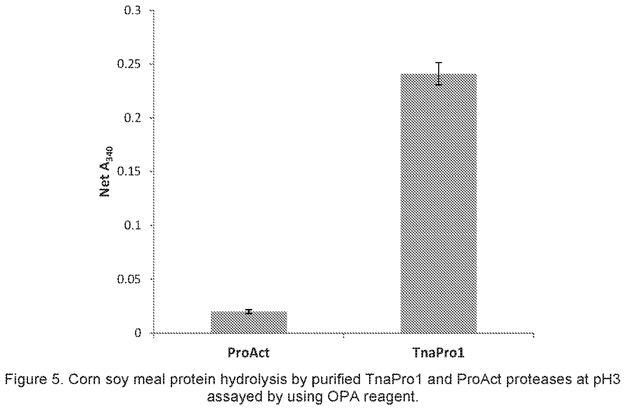

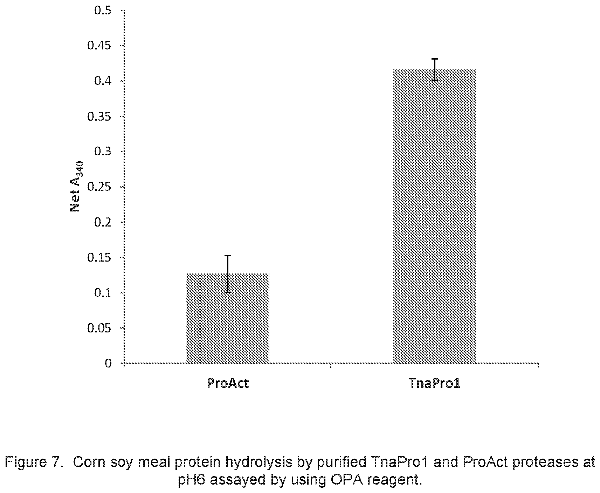

OPA detection: the OPA reagent was prepared by mixing 30 mL of 2 % tri-sodium phosphate buffer (pH 11), 800 µL of 4 % OPA (Cat# P1378, Sigma, dissolved in 96 % ethanol), 1 mL of 3.52 % Dithiothreitol (Cat# D0632, Sigma), and 8.2 mL of water. The reaction was initiated by adding 10 µL of the 10X diluted protease reaction to 175 µL OPA reagent in a 96-MTP (Cat# 3635, Corning Life Sciences). After 2 min incubation, the absorbance of the resultant solution was measured at 340 nm (A₃₄₀) using a spectrophotometer. Net A₃₄₀ (Figures 5 and 7) was calculated by subtracting the A₃₄₀ of the blank (no enzyme) control from that of each protease reaction, to measure the extent of corn soy meal hydrolysis achieved by each protease sample.

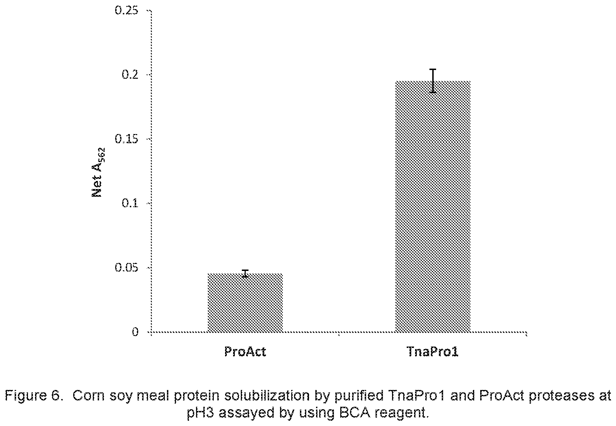

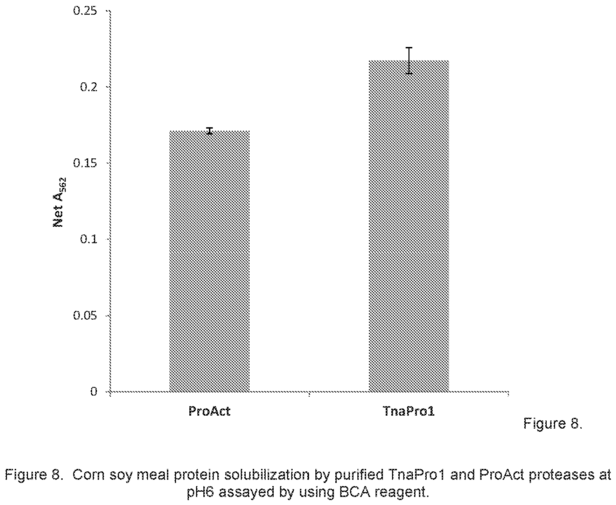

BCA detection: BCA colour reaction is proportional to the number of peptide bonds in polypeptides at least 3 residues long. The BCA reaction was conducted by mixing 10 µL of the 10X diluted protease reaction with 200 µL BCA reagent. The mixture was incubated in a thermomixer at 37°C for 30 min. The absorbance was subsequently measured at 562 nm (A₅₆₂) using a spectrophotometer. Net A₅₆₂ (Figures 6 and 8) was calculated by subtracting the A₅₆₂ of the blank control (no enzyme) from that of each protease reaction, to determine the extent of corn soy meal hydrolysis observed for each protease sample.

As shown in Figures 5, 6, 7 and 8, purified TnaPro1 can effectively hydrolyze the corn soy meal substrate.

### EXAMPLE 9

### Hydrolysis and Solubilization of Corn Soy Protein with TnaPro1 Protease

To a 96 well MTP was added 140µL 10% (w/w) corn soy feed slurry prepared as described in (Yu S, Cowieson A, Gilbert C, Plumstead P, Dalsgaard S., Interactions of phytate and myo-inositol phosphate esters (IP1-5) including IP5 isomers with dietary protein and iron and inhibition of pepsin. J. Anim. Sci. 2012, 90:1824-1832) with pH adjusted to pH 3.0, 20µL the TnaPro1 protease prepared in 50mM Na-acetate pH3.0 to obtain a final concentration of 0, 500, 1000, and 1500ppm, and 10µL pepsin (Sigma P7000, dissolved in water to a final concentration of 1.69mg/mL).

Pepsin and pancreatin enzymes were added for the control only, for the Blank, pepsin and pancreatin were omitted. The commercial feed protease, ProAct (RONOZYME® ProAct, DSM), was used as a reference. The plate was incubated at 40°C for 45 min in an iEMS Incubator/Shaker (Thermo Scientific) at 1150rpm. At the end of the incubation, 34µL of porcine pancreatin solution (Sigma P7545, 0.4636 mg/mL prepared in 1M sodium bicarbonate) was added and the samples were further incubated at 40°C for 60 min in an iEMS set at 1150rpm. After the incubation, the plate was centrifuged for 15min at 5°C and 4000 rpm. A 10µL aliquot of supernatant was transferred to a new 96 well MTP containing 90µL water (10x dilution). This 10-time diluted supernatant was used to determine protein hydrolysis by the OPA method.

Protein hydrolysis, using o-phthaldialdehyde (OPA) reagent, was done basically as described before (P.M. Nielsen, D. Petersen, and C. Dambmann, Improved method for determining food protein degree of hydrolysis, J. Food Sci. 66:642-646, 2001). The OPA reagent was prepared freshly by mixing 30mL tri-sodium phosphate (Na3PO4.12H2O, 2% w/v in water with pH adjusted pH11), 0.8mL OPA (0.4g o-phthaldialdehyde 97% in 10mL 96% ethanol1ml DTT solution (0.352g DL-dithiothreitol 99% in 10mL water) and water to a final volume of 40mL), and saved at -20°C). The reagent was kept in the dark and used right after the preparation. A 20µL aliquot of the 10x diluted supernatant was mixed with 175µL of the OPA reagent for 5 seconds and the absorbance was measured at 340nm exactly after 2min.

Table 2 shows the protein hydrolysis in the corn soy feed increasing with TnaPro1 and ProAct proteases at doses from 0 to 1500 ppm in the presence of both pepsin and pancreatin.

| **Table 2. Protein hydrolysis of corn soy feed by TnaPro1 and ProAct proteases.** | | | | | |
|---|---|---|---|---|---|
| Protease concentration (ppm) | Blank | Control | Control+ 500ppm | Control+ 1000ppm | Control+ 1500ppm |
| Protein hydrolysis (OPA value) by TnaPro1 | 0.306 | 0.542 | 0.927 | 1.044 | 1.119 |
| % of OPA valued with control as 100% by TnaPro1 | 56 | 100 | 171 | 193 | 207 |
| Protein hydrolysis (OPA value) by ProAct | 0.306 | 0.542 | 0.838 | 0.898 | 0.949 |
| % of OPA value with control as 100% by ProAct | 56 | 100 | 155 | 166 | 175 |

### EXAMPLE 10

### Pepsin Stability of TnaPro1

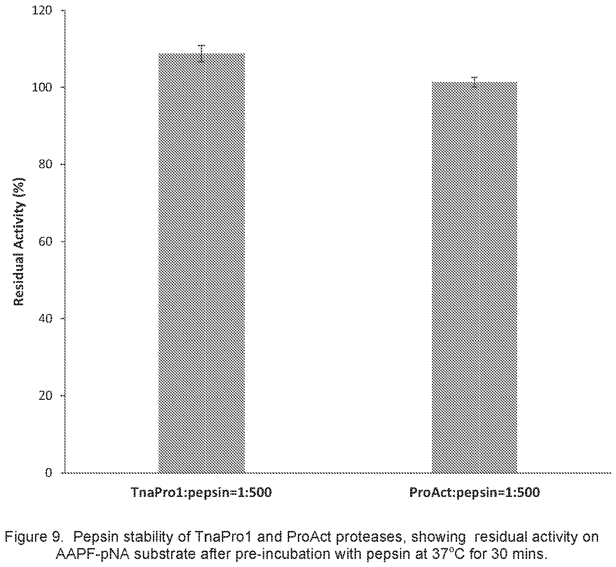

The pepsin stability of purified TnaPro1 and ProAct enzymes was analysed by incubating the enzymes with pepsin (Sigma, Cat. No. P7000), heat-deactivated and active, in 50 mM sodium acetate buffer (pH 3.0) and using AAPF-pNA to measure remaining activity . A 50 mg/mL stock solution of pepsin dissolved in 50 mM sodium acetate buffer (pH 3.0) was heated at 90°C for 15 min in a PCR machine to heat deactivate. The TnaPro1 and ProAct enzyme samples were mixed with the heat-deactivated pepsin or active pepsin at a ratio of 1:500, where the purified TnaPro1 and ProAct were dosed at 100 ppm. After 30 min incubation at 37°C, the samples were diluted 160 times in water. To measure the remaining activity,5 µL of 10 mM AAPF-pNA was mixed with 85 µL of HEPES buffer (50 mM, pH 8.0) in 96-MTP wells, then 10 µL of the diluted incubation mixtures were added. The AAPF-pNA assay was performed and results analysed as described above. Sodium acetate buffer containing 50 mg/mL pepsin was used as the blank control. The remaining enzyme activity was reported as the residual activity, where the activity of the sample in heat-deactivated pepsin was set to be 100%. The results are shown in Figure 9, and indicate that TnaPro1 activity is not affected by incubation with exogenous pepsin wherease ProAct activity was somewhat affected by incubation with exogenous pepsin

### EXAMPLE 11

### Evaluation of TnaPro1 Protease in Starch Liquefaction

The effect of TnaPro1 protease was tested in a lab scale corn liquefaction protocol as described below. Corn kernels (Arie Blok Animal Nutrition, NL-3440 AA Woerden, Artnr: 3777) were milled using a Retsch ZM200 grinding machine and settings: 3mm screen, 10k rpm. The milled corn flour was used to generate a 0.5 kG slurry at 32.0% dry solids by adding a 1:1 mixture of tap water/demineralized water to the flour. The pH was adjusted to 5.5 with H₂SO4 and afterwards Spezyme® RSL (DuPont commercial product containing a bacterial alpha amylase) was dosed at a relevant commercial dose. Subsequently, the *TnaPro1*protease was added to a final concentration of 4ug/gDS and the 0.5kG mixture was incubated at 85°C for two hours under constantly stirring conditions using an overhead mixer.

A control sample was generated by adding no protease to the liquefaction. Following the incubation, the treated corn sample (Liquefact) was collected and analyzed by HPLC size exclusion to determine the extent of corn protein solubilization during corn liquefaction. The end-of-liquefaction samples were spun down at 13,000 rpm for 5 minutes and 200 uL of supernatant was filtrated through a 0.22um filter. A 5uL aliquot of this filtrate, was injected on an HPLC (Agilent Technologies 1200 series), on a Waters BEH125Å, 1.7um/300mm column; and run at 0.3mL/min. Running buffer: 25mM NaPO4 pH6.8, 0.1M NaCl. Total area between retention time 4.8 min to 23min was integrated, Detection: OD220nm.

Results of the HPLC detection of peptides in liquefaction samples are shown in Table 3, as the integrated area under the curve with retention time between 4.8-23 minutes for samples treated with and without TnaPro1 protease. The control is set to 100% area units. The protein hydrolysis results shown on Table 3 indicate that when TnaPro1 protease was added in liquefaction an increase in the levels of soluble peptides was observed.

| Table 3. HPLC detection of peptides in liquefaction samples. Integrated area with retention time between 4.8-23 minutes is reported. | | |
|---|---|---|
| | Integrated Area units | Relative area under the curve |
| Spezyme RSL | 89957 | 100% |
| Spezyme RSL + TnaPro1 | 250815 | 279% |

### EXAMPLE 12

### Evaluation of TnaPro1 Protease as a Liquefaction Protease

Liquefaction-SSF studies were performed in which the protease, TnaPro1 was tested in lab scale corn liquefaction and simultaneous saccharification and fermentation (SSF). Corn kernels (Arie Blok Animal Nutrition, NL-3440 AA Woerden, Artnr: 3777) were milled using a Retsch ZM200 grinding machine and settings: 3mm screen, 10k rpm A 32% dry solids slurry was made by adding a 6:4 w/w mixture of tap water:backset from a commercial ethanol plant to the flour. The pH was adjusted to 5.3 with H₂SO₄ and afterwards SPEZYME® RSL (DuPont commercial product containing a bacterial alpha amylase) was dosed at a relevant commercial dose. Subsequently, the TnaPro1 protease was added to a final concentration of 3.5ug/gDS and the 0.5kG mixture was incubated at 85°C for two hours under constantly stirring conditions using an overhead mixer. A control sample was generated by adding no protease to the liquefaction. Following the incubation, the treated corn sample (Liquefact) was collected and used in a subsequent SSF experiment.

The pH of the liquefact samples (with and without *TnaPro1* protease added to liquefaction reaction) was adjusted to 4.8 and Synerxia Thrive LC® (DuPont commercial product containing a fungal alpha amylase and a fungal glucoamylase) was added at a commercial relevant dose to each SSF vessel containing 49 grams of liquefact. Afterwards, 1mL of a propagated yeast culture (Synerxia® Thrive, Dupont) was added and subsequently urea and FERMGEN™ 2.5x (DuPont commercial product containing an acid fungal protease) were added at different concentrations as indicated in Table 4. The experiments were performed in triplicate.

The fermentation vessels were incubated at 32°C in a forced air incubator at 150rpm. Samples were collected at five different time points (17h, 23h, 41h, 48h and 65hours). These samples were analyzed for ethanol concentration using HPLC separation and quantitation as described below.

Fermentation samples were centrifuged for 10 minutes 15,000g. 50ul of supernatant was added to a vial containing 500ul of 0.01 N H₂SO₄ Afterwards the sample was heated for 7 minutes at 95°C and filtered through a 0.2µm filter before HPLC analysis. The HPLC (Agilent Technologies 1200 series) run conditions were as follows: Phenomenex Rezex™ RFQ-Fast Acid H+ column held at 80°C, run at 1.0mL/min isocratic flow of 0.01 N H₂SO₄ solvent, a 10 µL injection volume, and 5.3 min elution runtime. Refractive index detection was used for quantification of ethanol, and the results are shown in Table 4.

| **Table 4. SSF ethanol yields (% v/v) obtained with various urea and FERMGEN™ 2.5X quantities, with and without TnaPro1 protease during corn liquefaction step.** | | |
|---|---|---|
| **Condition** | **hrs** | **% v/v Ethanol** |
| 600 ppm urea + 0.1 SAPU/gDS FERMGEN™ 2.5X | 17 | 8.5 (+/-0.08) |
| 150 ppm urea | 17 | 6.22 (+/-0.06) |
| 300 ppm urea | 17 | 6.97 (+/-0.1) |
| 3.5 ug/gDS *TnaPro1*, 150ppm Urea | 17 | 8.61 (+/-0.05) |
| 3.5 ug/gDS *TnaPro1*, 300ppm Urea | 17 | 8.57 (+/-0.19) |
| 3.5 ug/gDS *TnaPro1*, 600ppm Urea + 0.1 SAPU/gDS FERMGEN™ 2.5X | 17 | 8.63 (+/-0.05) |
| | | |
| 600 ppm urea + 0.1 SAPU/gDS FERMGEN™ 2.5X | 23 | 9.88 (+/-0.08) |
| 150 ppm urea | 23 | 7.61 (+/-0.11) |
| 300 ppm urea | 23 | 8.39 (+/-0.2) |
| 3.5 ug/gDS *TnaPro1*, 150ppm Urea | 23 | 9.93 (+/-0.02) |
| 3.5 ug/gDS *TnaPro1*, 300ppm Urea | 23 | 9.84 (+/-0.18) |
| 3.5 ug/gDS *TnaPro1*, 600ppm Urea + 0.1 SAPU/gDS FERMGEN™ 2.5X | 23 | 9.98 (+/-0.08) |
| | | |
| 600 ppm urea + 0.1 SAPU/gDS FERMGEN™ 2.5X | 41 | 14.11 (+/-0.28) |
| 150 ppm urea | 41 | 11.49 (+/-0.13) |
| 300 ppm urea | 41 | 12.73 (+/-0.03) |
| 3.5 ug/gDS *TnaPro1*, 150ppm Urea | 41 | 14.04 (+/-0.21) |
| 3.5 ug/gDS *TnaPro1*, 300ppm Urea | 41 | 13.69 (+/-0.15) |
| 3.5 ug/gDS *TnaPro1*, 600ppm Urea + 0.1 SAPU/gDS FERMGEN™ 2.5X | 41 | 14.21 (+/-0.01) |
| | | |
| 600 ppm urea + 0.1 SAPU/gDS FERMGEN™ 2.5X | 47 | 15.01 (+/-0.22) |
| 150 ppm urea | 47 | 12.3 (+/-0.15) |
| 300 ppm urea | 47 | 13.58 (+/-0.09) |
| 3.5 ug/gDS *TnaPro1*, 150ppm Urea | 47 | 14.91 (+/-0.2) |
| 3.5 ug/gDS *TnaPro1*, 300ppm Urea | 47 | 14.84 (+/-0.19) |
| 3.5 ug/gDS *TnaPro1*, 600ppm Urea + 0.1 SAPU/gDS FERMGEN™ 2.5X | 47 | 14.77 (+/-0.07) |
| | | |
| 600 ppm urea + 0.1 SAPU/gDS FERMGEN™ 2.5X | 65 | 15.56 (+/-0.34) |
| 150 ppm urea | 65 | 14.02 (+/-0.19) |
| 300 ppm urea | 65 | 14.88 (+/-0.04) |
| 3.5 ug/gDS *TnaPro1*, 150ppm Urea | 65 | 15.49 (+/-0.35) |
| 3.5 ug/gDS *TnaPro1*, 300ppm Urea | 65 | 15.93 (+/-0.05) |
| 3.5 ug/gDS *TnaPro1* 600ppm Urea + 0.1 SAPU/gDS FERMGEN™ 2.5X | 65 | 15.57 (+/-0.12) |

Results show that when TnaPro1 was added to the liquefaction, the Urea dose in SSF could be reduced by 75% without having a negative effect on ethanol formation.

## Claims

1. A recombinant construct comprising a nucleotide sequence encoding a thermostable polypeptide having serine protease activity, wherein said coding nucleotide sequence is operably linked to at least one regulatory sequence functional in a production host and the nucleotide sequence encodes a polypeptide with the amino acid sequence set forth in SEQ ID NO: 3, or a polypeptide with at least 92% amino acid sequence identity thereto;
and wherein said regulatory sequence is heterologous to the coding nucleotide sequence, or said regulatory sequence and coding sequence are not arranged as found together in nature.

2. The recombinant construct of claim 1, wherein:
(a) said coding nucleotide sequence is a nucleotide sequence encoding a polypeptide with the amino acid sequence set forth in SEQ ID NO: 8, or a polypeptide with at least 89% amino acid sequence identity thereto; and/or
(b) said coding nucleotide sequence is selected from the group consisting of:
i) a nucleotide sequence encoding a polypeptide with the amino acid sequence set forth in SEQ ID NO: 2, or a polypeptide with at least 86 % amino acid sequence identity thereto; or
ii) a nucleotide sequence encoding a polypeptide with the amino acid sequence set forth in SEQ ID NO:5 or 14, or a polypeptide with at least 84% amino acid sequence identity thereto;
and/or
(c) said at least one regulatory sequence comprises a promoter.

3. A vector comprising a recombinant construct as defined in claim 1 or claim 2.

4. A production host or host cell comprising the recombinant construct of claim 1 or claim 2, optionally wherein said cell is a bacterial cell, an archaeal cell, a fungal cell or an algal cell.

5. A method for producing a thermostable serine protease, said method comprising:
i) transforming a host cell with a construct as defined in claim 1 or claim 2; and
ii) culturing the transformed host cell of step (i) under conditions whereby the thermostable serine protease is produced,
optionally wherein the method further comprises recovering the thermostable serine protease from the host cell, and/or wherein said host cell is a bacterial cell, an archaeal cell, a fungal cell or an algal cell.

6. A culture supernatant comprising a thermostable serine protease obtained by the method of claim 5.

7. A method for hydrolyzing a material derived from corn, said method comprising:
(a) contacting the material obtained from corn with a liquid to form a mash; and
(b) hydrolyzing at least one protein in the mash to form a hydrolysate by contacting the mash with an enzyme cocktail comprising a thermostable serine protease comprises the amino acid sequence set forth in SEQ ID NO: 3, or an amino acid sequence having at least 92% sequence identity to SEQ ID NO:3 and
(c) optionally, recovering the hydrolysate of obtained in step (b).

8. An animal feed, feedstuff, feed additive composition or premix comprising at least one polypeptide having serine protease activity and is thermostable, wherein said polypeptide comprises the amino acid sequence set forth in SEQ ID NO: 3, or an amino acid sequence with at least 92% sequence identity thereto, and wherein said animal feed, feedstuff, feed additive composition or premix optionally further comprises (a) at least one direct-fed microbial or (b) at least one other enzyme or (c) at least one direct fed microbial and at least one other enzyme.

9. The feed additive composition of claim 8, wherein said feed additive composition further comprises at least one component selected from the group consisting of a protein, a peptide, sucrose, lactose, sorbitol, glycerol, propylene glycol, sodium chloride, sodium sulfate, sodium acetate, sodium citrate, sodium formate, sodium sorbate, potassium chloride, potassium sulfate, potassium acetate, potassium citrate, potassium formate, potassium acetate, potassium sorbate, magnesium chloride, magnesium sulfate, magnesium acetate, magnesium citrate, magnesium formate, magnesium sorbate, sodium metabisulfite, methyl paraben and propyl paraben.

10. The feed additive composition of claim 8 or 9, wherein
(a) said feed additive composition is granulated and comprises particles produced by a process selected from the group consisting of high shear granulation, drum granulation, extrusion, spheronization, fluidized bed agglomeration, fluidized bed spray coating, spray drying, freeze drying, prilling, spray chilling, spinning disk atomization, coacervation, tableting, or any combination of the above processes, optionally wherein the mean diameter of the particles is between 50 and 2000 microns; and/or
(b) said feed additive composition is in the form of a liquid, a dry powder, or a granule or a coating, or is in a coated or encapsulated form, optionally wherein said feed additive composition is in the form of a liquid which is suitable for spray drying on a feed pellet.

11. The animal feed of claim 8, wherein the at least one polypeptide having serine protease activity is present in an amount of 1 to 20 g/tonne.

12. A method for producing fermentation products from starch-containing material comprising:
(a) liquefying the starch-containing material with an enzyme cocktail comprising a serine protease comprising the amino acid sequence set forth in SEQ ID NO: 3, or an amino acid sequence having at least 92% sequence identity to SEQ ID NO:3;
(b) saccharifying the product of step (a);
(c) fermenting with a suitable organism; and
(d) optionally, recovering the product produced in step (c).

13. The method of claim 12 wherein steps (b) and (c) are performed simultaneously.

14. The method of claim 12 or 13 wherein addition of a nitrogen source is eliminated or reduced by at least 50% by using 1-20 g serine protease/MT starch-containing material wherein the serine protease comprises the amino acid sequence set forth in SEQ ID NO: 3, or an amino acid sequence having at least 92% sequence identity to SEQ ID NO:3, optionally wherein the nitrogen source is urea.

15. The method of claim 14 wherein the liquefaction product is ethanol and no acid proteolytic enzyme is needed when using 1-20 g thermostable serine protease/MT starch-containing material wherein the serine protease comprises the amino acid sequence set forth in SEQ ID NO: 3, or an amino acid sequence having at least 92% sequence identity to SEQ ID NO:3.

## Patentansprüche

1. Rekombinantes Konstrukt umfassend eine Nukleotidsequenz, die für ein wärmestabiles Polypeptid codiert, das Serinproteaseaktivität aufweist, wobei die kodierende Nukleotidsequenz funktionsfähig mit mindestens einer regulatorischen Sequenz verknüpft ist, die in einem Produktionswirt funktional ist, und die Nukleotidsequenz für ein Polypeptid mit der Aminosäuresequenz, die in SEQ ID NO: 3 aufgeführt ist, oder ein Polypeptid mit mindestens 92 % Aminosäuresequenzidentität dazu codiert;
und wobei die regulatorische Sequenz zu der kodierenden Nukleotidsequenz heterolog ist oder die regulatorische Sequenz und die kodierende Sequenz nicht wie in der Natur zusammen aufgefunden angeordnet sind.

2. Rekombinantes Konstrukt nach Anspruch 1, wobei:
(a) die kodierende Nukleotidsequenz eine Nukleotidsequenz ist, die für ein Polypeptid mit der Aminosäuresequenz, die in SEQ ID NO: 8 aufgeführt ist, oder ein Polypeptid mit mindestens 89 % Aminosäuresequenzidentität dazu codiert; und/oder
(b) die kodierende Nukleotidsequenz aus der Gruppe ausgewählt ist bestehend aus:
i) einer Nukleotidsequenz, die für ein Polypeptid mit der Aminosäuresequenz, die in SEQ ID NO: 2 aufgeführt ist, oder einem Polypeptid mit mindestens 86 % Aminosäuresequenzidentität dazu codiert; oder
ii) einer Nukleotidsequenz, die für ein Polypeptid mit der Aminosäuresequenz, die in SEQ ID NO: 5 oder 14 aufgeführt ist, oder ein Polypeptid mit mindestens 84 % Aminosäuresequenzidentität dazu codiert;
und/oder
(c) die mindestens eine regulatorische Sequenz einen Promotor umfasst

3. Vektor umfassend ein rekombinantes Konstrukt wie in Anspruch 1 oder Anspruch 2 definiert.

4. Herstellungswirt oder -wirtszelle umfassend das rekombinante Konstrukt nach Anspruch 1 oder Anspruch 2, wobei wahlweise die Zelle eine Bakterienzelle, eine archaeale Zelle, eine Pilzzelle oder eine Algenzelle ist.

5. Verfahren zum Herstellen einer wärmestabilen Serinprotease, wobei das Verfahren Folgendes umfasst:
i) Umwandeln einer Wirtszelle mit einem Konstrukt wie in Anspruch 1 oder Anspruch 2 definiert; und
ii) Züchten der umgewandelten Wirtszelle aus Schritt (i) unter Bedingungen, wobei die wärmestabile Serinprotease hergestellt wird,
wobei wahlweise das Verfahren ferner das Gewinnen der wärmestabilen Serinprotease aus der Wirtszelle umfasst und/oder wobei die Wirtszelle eine Bakterienzelle, eine archaeale Zelle, eine Pilzzelle oder eine Algenzelle ist.

6. Kulturüberstand umfassend eine wärmestabile Serinprotease, die durch das Verfahren nach Anspruch 5 erhalten wird.

7. Verfahren zum Hydrolysieren eines Materials, das von Mais abgeleitet ist, wobei das Verfahren Folgendes umfasst:
(a) In-Kontakt-Bringen des Materials, das von Mais erhalten worden ist, mit einer Flüssigkeit, um einen Brei zu bilden; und
(b) Hydrolysieren mindestens eines Proteins in dem Brei, um ein Hydrolysat durch In-Kontakt-Bringen des Breis mit einem Enzymcocktail zu bilden, der eine wärmestabile Serinprotease umfasst, die die Aminosäuresequenz, die in SEQ ID NO: 3 aufgeführt ist, oder eine Aminosäuresequenz umfasst, die mindestens 92 % Sequenzidentität mit SEQ ID NO: 3 aufweist und
(c) wahlweise Gewinnen des Hydrolysats, das in Schritt (b) erhalten wird.

8. Tierfutter, Futtermittel, Futterzusatzmittelzusammensetzung oder Vormischung umfassend mindestens ein Polypeptid, das Serinproteaseaktivität aufweist und wärmestabil ist, wobei das Polypeptid die Aminosäuresequenz, die in SEQ ID NO: 3 aufgeführt ist, oder eine Aminosäuresequenz mit mindestens 92 % Sequenzidentität dazu umfasst, und wobei das Tierfutter, Futtermittel, die Futterzusatzmittelzusammensetzung oder Vormischung ferner wahlweise (a) mindestens ein direkt gefüttertes mikrobielles Mittel oder (b) mindestens ein anderes Enzym oder (c) mindestens ein direkt gefüttertes mikrobielles Mittel und mindestens ein anderes Enzym umfasst.

9. Futterzusatzmittelzusammensetzung nach Anspruch 8, wobei die Futterzusatzmittelzusammensetzung ferner mindestens eine Komponente umfasst ausgewählt aus der Gruppe bestehend aus einem Protein, einem Peptid, Sucrose, Lactose, Sorbit, Glycerin, Propylenglykol, Natriumchlorid, Natriumsulfat, Natriumacetat,, Natriumcitrat, Natriumformiat, Natriumsorbat, Kaliumchlorid, Kaliumsulfat, Kaliumacetat, Kaliumcitrat, Kaliumformiat, Kaliumacetat, Kaliumsorbat, Magnesiumchlorid, Magnesiumsulfat, Magnesiumacetat, Magnesiumcitrat, Magnesiumformiat, Magnesiumsorbat, Natriummetabisulfit, Methylparaben und Propylparaben.

10. Futterzusatzmittelzusammensetzung nach Anspruch 8 oder 9, wobei
(a) die Futterzusatzmittelzusammensetzung granuliert ist und Teilchen umfasst, die durch ein Verfahren hergestellt werden ausgewählt aus der Gruppe bestehend aus Hochschergranulierung, Trommelgranulierung, Extrusion, Sphäronisierung, Wirbelbettagglomeration, Wirbelbettsprühbeschichtung, Sprühtrocknung, Gefriertrocknung, Sprühkristallisation, Spritzkühlen, Schleuderscheibenzerstäubung, Coazervation, Tablettieren oder eine beliebige Kombination der obigen Verfahren, wobei wahlweise der mittlere Durchmesser der Teilchen zwischen 50 und 2000 Mikron beträgt; und/oder
(b) die Futterzusatzmittelzusammensetzung in Form einer Flüssigkeit, eines trockenen Pulvers oder eines Granulats oder einer Beschichtung vorliegt oder in einer schichtförmig aufgebrachten oder eingekapselten Form vorliegt, wobei wahlweise die Futterzusatzmittelzusammensetzung in Form einer Flüssigkeit vorliegt, die zum Sprühtrocknen auf einem Futtermittelkügelchen geeignet ist.

11. Tierfutter nach Anspruch 8, wobei das mindestens eine Polypeptid, das Serinproteaseaktivität aufweist, in einer Menge von 1 bis 20 g/Tonne vorliegt.

12. Verfahren zum Herstellen von Fermentationsprodukten aus stärkehaltigem Material, umfassend:
(a) Verflüssigen des stärkehaltigen Materials mit einem Enzymcocktail umfassend eine Serinprotease umfassend die Aminosäuresequenz, die in SEQ ID NO: 3 aufgeführt ist, oder eine Aminosäuresequenz, mit mindestens 92 % Sequenzidentität mit SEQ ID NO:3 aufweist;
(b) Sacharifizieren des Produkts aus Schritt (a);
(c) Fermentieren mit einem geeigneten Organismus; und
(d) wahlweise Gewinnen des in Schritt (c) hergestellten Produkts.

13. Verfahren nach Anspruch 12, wobei die Schritte (b) und (c) gleichzeitig ausgeführt werden.

14. Verfahren nach Anspruch 12 oder 13, wobei die Zugabe einer Stickstoffquelle eliminiert oder mindestens 50 % durch Verwenden von 1-20 g Serinprotease/Tonne stärkehaltigem Material reduziert wird, wobei die Serinprotease die Aminosäuresequenz, die in SEQ ID NO: 3e aufgeführt ist, oder eine Aminosäuresequenz, die mindestens 92 % Sequenzidentität mit SEQ ID NO: 3 aufweist, umfasst, wobei wahlweise die Stickstoffquelle Harnstoff ist.

15. Verfahren nach Anspruch 14, wobei das Verflüssigungsprodukt Ethanol ist und kein saures proteolytisches Enzym benötigt wird, wenn 1-20 g wärmestabile Serinprotease/MT stärkehaltiges Material verwendet werden, wobei die Serinprotease die Aminosäuresequenz, die in SEQ ID NO: 3 aufgeführt ist, oder eine Aminosäuresequenz, die mindestens 92 % Sequenzidentität mit SEQ ID NO: 3 aufweist, umfasst.

## Revendications

1. Construction recombinante comprenant une séquence de nucléotides codant un polypeptide thermostable qui présente une activité de sérine protéase, dans laquelle ladite séquence de nucléotides de codage est liée de manière opérationnelle à au moins une séquence de régulation qui est fonctionnelle au niveau d'un hôte de production et la séquence de nucléotides code un polypeptide avec la séquence d'acides aminés telle que mise en exergue selon SEQ ID NO : 3, ou un polypeptide qui présente une identité d'au moins 92 % de séquence d'acides aminés par rapport à cette séquence ;
et dans laquelle ladite séquence de régulation est hétérologue par rapport à la séquence de nucléotides de codage, ou ladite séquence de régulation et ladite séquence de codage ne sont pas agencées tel qu'on les trouve ensemble dans l'état naturel.

2. Construction recombinante selon la revendication 1, dans laquelle :
(a) ladite séquence de nucléotides de codage est une séquence de nucléotides codant un polypeptide avec la séquence d'acides aminés telle que mise en exergue selon SEQ ID NO : 8, ou un polypeptide qui présente une identité d'au moins 89 % de séquence d'acides aminés par rapport à cette séquence ; et/ou
(b) ladite séquence de nucléotides de codage est sélectionnée parmi le groupe qui est constitué par :
i) une séquence de nucléotides codant un polypeptide avec la séquence d'acides aminés telle que mise en exergue selon SEQ ID NO : 2, ou un polypeptide qui présente une identité d'au moins 86 % de séquence d'acides aminés par rapport à cette séquence ; ou
ii) une séquence de nucléotides codant un polypeptide avec la séquence d'acides aminés telle que mise en exergue selon SEQ ID NO : 5 ou 14, ou un polypeptide qui présente une identité d'au moins 84 % de séquence d'acides aminés par rapport à cette séquence ;
et/ou
(c) ladite au moins une séquence de régulation comprend un promoteur.

3. Vecteur comprenant une construction recombinante telle que définie selon la revendication 1 ou la revendication 2.

4. Hôte de production ou cellule hôte comprenant la construction recombinante selon la revendication 1 ou la revendication 2, en option dans lequel ou laquelle ladite cellule est une cellule bactérienne, une cellule archéenne, une cellule fongique ou une cellule algale.

5. Procédé pour produire une sérine protéase thermostable, ledit procédé comprenant :
i) la transformation d'une cellule hôte à l'aide d'une construction telle que définie selon la revendication 1 ou la revendication 2 ; et
ii) la mise en culture de la cellule hôte transformée de l'étape i) sous des conditions desquelles il résulte que la sérine protéase thermostable est produite ;
en option, dans lequel le procédé comprend en outre la récupération de la sérine protéase thermostable à partir de la cellule hôte, et/ou dans lequel ladite cellule hôte est une cellule bactérienne, une cellule archéenne, une cellule fongique ou une cellule algale.

6. Surnageant de culture comprenant une sérine protéase thermostable obtenue au moyen du procédé selon la revendication 5.

7. Procédé pour hydrolyser une matière dérivée du maïs, ledit procédé comprenant :
(a) la mise en contact de la matière qui est obtenue à partir du maïs avec un liquide pour former une purée ; et
(b) l'hydrolyse d'au moins une protéine dans la purée pour former un hydrolysat par mise en contact de la purée avec un cocktail d'enzymes qui comprend une sérine protéase thermostable comprend la séquence d'acides aminés telle que mise en exergue selon SEQ ID NO : 3, ou une séquence d'acides aminés qui présente une identité d'au moins 92 % de séquence par rapport à SEQ ID NO : 3; et
(c) en option, la récupération de l'hydrolysat qui est obtenu au niveau de l'étape (b).

8. Aliment pour animaux, produit alimentaire, composition d'additif alimentaire ou prémélange comprenant au moins un polypeptide qui présente une activité de sérine protéase et qui est thermostable, dans lequel ou laquelle ledit polypeptide comprend la séquence d'acides aminés telle que mise en exergue selon SEQ ID NO : 3, ou une séquence d'acides aminés qui présente une identité d'au moins 92 % de séquence par rapport à cette séquence, et dans lequel ou laquelle ledit aliment pour animaux, ledit produit élémentaire, ladite composition d'additif alimentaire ou ledit prémélange comprend en option en outre (a) au moins un élément microbien à alimentation directe ou (b) au moins un autre enzyme ou (c) au moins un élément microbien à alimentation directe et au moins un autre enzyme.

9. Composition d'additif alimentaire selon la revendication 8, dans laquelle ladite composition d'additif alimentaire comprend en outre au moins un composant qui est sélectionné parmi le groupe qui est constitué par une protéine, un peptide, le sucrose, le lactose, le sorbitol, le glycérol, le propylène glycol, le chlorure de sodium, le sulfate de sodium, l'acétate de sodium, le citrate de sodium, le formate de sodium, le sorbate de sodium, le chlorure de potassium, le sulfate de potassium, l'acétate de potassium, le citrate de potassium, le formate de potassium, l'acétate de potassium, le sorbate de potassium, le chlorure de magnésium, le sulfate de magnésium, l'acétate de magnésium, le citrate de magnésium, le formate de magnésium, le sorbate de magnésium, le métabisulfite de sodium, le méthylparabène et le propylparabène.

10. Composition d'additif alimentaire selon la revendication 8 ou 9, dans laquelle :
(a) ladite composition d'additif alimentaire est granulée et comprend des particules qui sont produites au moyen d'un processus qui est sélectionné parmi le groupe qui est constitué par une granulation à cisaillement élevé, une granulation en tambour, une extrusion, une sphéronisation, une agglomération à lit fluidisé, un enrobage par pulvérisation à lit fluidisé, un séchage par pulvérisation, une lyophilisation, un grelonage, un refroidissement par aspersion, une atomisation par disque rotatif, une coacervation, une fabrication de comprimés ou une quelconque combinaison des processus mentionnés ci-avant, en option dans laquelle le diamètre moyen des particules se situe entre 50 et 2 000 micromètres ; et/ou
(b) ladite composition d'additif alimentaire se présente sous la forme d'un liquide, d'une poudre sèche ou de granulés ou d'un enrobage, ou se présente selon une forme enrobée ou encapsulée sous gélules, en option dans laquelle ladite composition d'additif alimentaire se présente sous la forme d'un liquide qui convient pour un séchage par pulvérisation sur un granule alimentaire.

11. Aliment pour animaux selon la revendication 8, dans lequel l'au moins un polypeptide qui présente une activité de sérine protéase est présent selon une quantité qui va de 1 à 20 g/tonne.

12. Procédé pour produire des produits de fermentation à partir d'une matière contenant de l'amidon, comprenant :
(a) la liquéfaction de la matière contenant de l'amidon à l'aide d'un cocktail d'enzymes qui comprend une sérine protéase qui comprend la séquence d'acides aminés telle que mise en exergue selon SEQ ID NO : 3, ou une séquence d'acides aminés qui présente une identité d'au moins 92 % de séquence par rapport à SEQ ID NO : 3 ;
(b) la saccharification du produit de l'étape (a) ;
(c) la fermentation à l'aide d'un organisme approprié ; et
(d) en option, la récupération du produit qui est produit au niveau de l'étape (c).

13. Procédé selon la revendication 12, dans lequel les étapes (b) et (c) sont réalisées simultanément.

14. Procédé selon la revendication 12 ou 13, dans lequel l'ajout d'une source d'azote est éliminé ou réduit d'au moins 50 % en utilisant 1 à 20 g de sérine protéase/TM de matière contenant de l'amidon, dans lequel la sérine protéase comprend la séquence d'acides aminés telle que mise en exergue selon SEQ ID NO : 3, ou une séquence d'acides aminés qui présente une identité d'au moins 92 % de séquence par rapport à SEQ ID NO : 3, en option dans lequel la source d'azote est l'urée.

15. Procédé selon la revendication 14, dans lequel le produit de liquéfaction est l'éthanol et aucun enzyme protéolytique acide n'est nécessaire lors de l'utilisation de 1 à 20 g de sérine protéase/TM de matière contenant de l'amidon, dans lequel la sérine protéase comprend la séquence d'acides aminés telle que mise en exergue selon SEQ ID NO : 3, ou une séquence d'acides aminés qui présente une identité d'au moins 92 % de séquence par rapport à SEQ ID NO : 3.


### SEQUENCE LISTING

<110> DUPONT NUTRITION BIOSCIENCES APS
   Gu, Xiaogang
   van Brussel, Marco
   Zou, Zhengzheng
   Yu, Shukun
   Shipovskov, Stepan
<120> NB41217
<130> Methods of using an Archaeal Serine Protease
<160> 17
<170> PatentIn version 3.5
<210> 1
   <211> 1281
   <212> DNA
   <213> Thermococcus nautili
<400> 1
   atggagcgga gggtagtagt tggcggactg ctcgtcctgg cactggttct gtccctcttt      60
   gctgtgccaa ccgctgccaa gggcagcgac acggttaggg tggttgccct tatagacagg     120
   gacgacttca ggggagatgc agtaaggggc ataggcggta aaatcgtcta cgagttcaag     180
   ctgattgatg ccgtcgtcat tgacattcct cccaacgcgg ttggaaagct cgccaagctt     240
   gagggagtta agaatgtcga gttcgacgcc caggcccagc tctacagggg agggccgagg     300
   tggctcgttc cagagcccac acagcccgcc caggagattc cctggggaat agagcgcgtt     360
   aaggctcccg acacatggag tataaccacc ggcgccagca acggcgtcat cgaggttgcc     420
   atactcgaca ccggaataga ctgggaccac cccgacctca aggacaacat cgcctggggc      480
   gtcagcacca taggcggcga ggttagcacc gaccccgctg actggtacga cggcaacggc     540
   cacggaaccc acgtcgccgg aaccatcgcg gcgcttaaca acgacatcgg cgtcgtcgga     600
   gtcgccccga acgttgaaat ctacgccata aaggttcttg acgacagggg aagtggagcg     660
   tacaccgaca tagcgatagg aatcgagcag gcccttctcg gtccggacgg aatacttgac     720
   aaggacgggg acggaataat cgttggagac ccggatgatg acgccgccga ggtcataagc     780
   atgtccctcg gaggaccgag cgacgatgag tacctccacg acatgataat caaggcctac     840
   aactacggcg tcgtcatcgt cgccgcgagc ggtaacgagg ccgccgacca gccgagctac     900
   ccggccatct acccggaggt catagcggtt ggagccaccg actcgagcga cgccgtagct     960
   tacttcagca acctccagcc cgaagttagc gccccgggcg ttgacgtgct cagcacctac    1020
   ccggacgaca cctacgagag cctcagcgga acctcaatgg ccaccccgca cgtcagcggt    1080
   gtcgttgcgc tcatccaggc ggcctactac aacaagtacg gcaaggttct cccggtcgga    1140
   accttcgatg acatgaacac caacaccgtc aggggaatcc tccacgttac cgccgacgac    1200
   ctcggagacc ccggatggga catctactac ggctacggaa tagtcagggc cgacctggca    1260
   gtccaggcgg ctcttggctg a                                               1281
<210> 2
   <211> 426
   <212> PRT
   <213> Thermococcus nautili
<400> 2
   Met Glu Arg Arg Val Val Val Gly Gly Leu Leu Val Leu Ala Leu Val
   1               5                   10                  15
   Leu Ser Leu Phe Ala Val Pro Thr Ala Ala Lys Gly Ser Asp Thr Val
               20                  25                  30
   Arg Val Val Ala Leu Ile Asp Arg Asp Asp Phe Arg Gly Asp Ala Val
           35                  40                  45
   Arg Gly Ile Gly Gly Lys Ile Val Tyr Glu Phe Lys Leu Ile Asp Ala
       50                  55                  60
   Val Val Ile Asp Ile Pro Pro Asn Ala Val Gly Lys Leu Ala Lys Leu
   65                  70                  75                  80
   Glu Gly Val Lys Asn Val Glu Phe Asp Ala Gln Ala Gln Leu Tyr Arg
                   85                  90                  95
   Gly Gly Pro Arg Trp Leu Val Pro Glu Pro Thr Gln Pro Ala Gln Glu
               100                 105                 110
   Ile Pro Trp Gly Ile Glu Arg Val Lys Ala Pro Asp Thr Trp Ser Ile
           115                 120                 125
   Thr Thr Gly Ala Ser Asn Gly Val Ile Glu Val Ala Ile Leu Asp Thr
       130                 135                 140
   Gly Ile Asp Trp Asp His Pro Asp Leu Lys Asp Asn Ile Ala Trp Gly
   145                 150                 155                 160
   Val Ser Thr Ile Gly Gly Glu Val Ser Thr Asp Pro Ala Asp Trp Tyr
                   165                 170                 175
   Asp Gly Asn Gly His Gly Thr His Val Ala Gly Thr Ile Ala Ala Leu
               180                 185                 190
   Asn Asn Asp Ile Gly Val Val Gly Val Ala Pro Asn Val Glu Ile Tyr
           195                 200                 205
   Ala Ile Lys Val Leu Asp Asp Arg Gly Ser Gly Ala Tyr Thr Asp Ile
       210                 215                 220
   Ala Ile Gly Ile Glu Gln Ala Leu Leu Gly Pro Asp Gly Ile Leu Asp
   225                 230                 235                 240
   Lys Asp Gly Asp Gly Ile Ile Val Gly Asp Pro Asp Asp Asp Ala Ala
                   245                 250                 255
   Glu Val Ile Ser Met Ser Leu Gly Gly Pro Ser Asp Asp Glu Tyr Leu
               260                 265                 270
   His Asp Met Ile Ile Lys Ala Tyr Asn Tyr Gly Val Val Ile Val Ala
           275                 280                 285
   Ala Ser Gly Asn Glu Ala Ala Asp Gln Pro Ser Tyr Pro Ala Ile Tyr
       290                 295                 300
   Pro Glu Val Ile Ala Val Gly Ala Thr Asp Ser Ser Asp Ala Val Ala
   305                 310                 315                 320
   Tyr Phe Ser Asn Leu Gln Pro Glu Val Ser Ala Pro Gly Val Asp Val
                   325                 330                 335
   Leu Ser Thr Tyr Pro Asp Asp Thr Tyr Glu Ser Leu Ser Gly Thr Ser
               340                 345                 350
   Met Ala Thr Pro His Val Ser Gly Val Val Ala Leu Ile Gln Ala Ala
           355                 360                 365
   Tyr Tyr Asn Lys Tyr Gly Lys Val Leu Pro Val Gly Thr Phe Asp Asp
       370                 375                 380
   Met Asn Thr Asn Thr Val Arg Gly Ile Leu His Val Thr Ala Asp Asp
   385                 390                 395                 400
   Leu Gly Asp Pro Gly Trp Asp Ile Tyr Tyr Gly Tyr Gly Ile Val Arg
                   405                 410                 415
   Ala Asp Leu Ala Val Gln Ala Ala Leu Gly
               420                 425
<210> 3
   <211> 325
   <212> PRT
   <213> Thermococcus nautili
<400> 3
   Leu Val Pro Glu Pro Thr Gln Pro Ala Gln Glu Ile Pro Trp Gly Ile
   1               5                   10                  15
   Glu Arg Val Lys Ala Pro Asp Thr Trp Ser Ile Thr Thr Gly Ala Ser
               20                  25                  30
   Asn Gly Val Ile Glu Val Ala Ile Leu Asp Thr Gly Ile Asp Trp Asp
           35                  40                  45
   His Pro Asp Leu Lys Asp Asn Ile Ala Trp Gly Val Ser Thr Ile Gly
       50                  55                  60
   Gly Glu Val Ser Thr Asp Pro Ala Asp Trp Tyr Asp Gly Asn Gly His
   65                  70                  75                  80
   Gly Thr His Val Ala Gly Thr Ile Ala Ala Leu Asn Asn Asp Ile Gly
                   85                  90                  95
   Val Val Gly Val Ala Pro Asn Val Glu Ile Tyr Ala Ile Lys Val Leu
               100                 105                 110
   Asp Asp Arg Gly Ser Gly Ala Tyr Thr Asp Ile Ala Ile Gly Ile Glu
           115                 120                 125
   Gln Ala Leu Leu Gly Pro Asp Gly Ile Leu Asp Lys Asp Gly Asp Gly
       130                 135                 140
   Ile Ile Val Gly Asp Pro Asp Asp Asp Ala Ala Glu Val Ile Ser Met
   145                 150                 155                 160
   Ser Leu Gly Gly Pro Ser Asp Asp Glu Tyr Leu His Asp Met Ile Ile
                   165                 170                 175
   Lys Ala Tyr Asn Tyr Gly Val Val Ile Val Ala Ala Ser Gly Asn Glu
               180                 185                 190
   Ala Ala Asp Gln Pro Ser Tyr Pro Ala Ile Tyr Pro Glu Val Ile Ala
           195                 200                 205
   Val Gly Ala Thr Asp Ser Ser Asp Ala Val Ala Tyr Phe Ser Asn Leu
       210                 215                 220
   Gln Pro Glu Val Ser Ala Pro Gly Val Asp Val Leu Ser Thr Tyr Pro
   225                 230                 235                 240
   Asp Asp Thr Tyr Glu Ser Leu Ser Gly Thr Ser Met Ala Thr Pro His
                   245                 250                 255
   Val Ser Gly Val Val Ala Leu Ile Gln Ala Ala Tyr Tyr Asn Lys Tyr
               260                 265                 270
   Gly Lys Val Leu Pro Val Gly Thr Phe Asp Asp Met Asn Thr Asn Thr
           275                 280                 285
   Val Arg Gly Ile Leu His Val Thr Ala Asp Asp Leu Gly Asp Pro Gly
       290                 295                 300
   Trp Asp Ile Tyr Tyr Gly Tyr Gly Ile Val Arg Ala Asp Leu Ala Val
   305                 310                 315                 320
   Gln Ala Ala Leu Gly
                   325
<210> 4
   <211> 1299
   <212> DNA
   <213> Artificial Sequence
<220>
   <223> AprE-TnaProl gene with 3 AA insertion
<400> 4
   gtgagaagca aaaaattgtg gatcagcttg ttgtttgcgt taacgttaat ctttacgatg      60
   gcgttcagca acatgagcgc gcaggctgct ggaaaaaaag gctcagatac ggttcgcgtt     120
   gttgcactta tcgatagaga tgattttcgc ggcgatgcag ttagaggaat cggcggcaaa     180
   atcgtttatg agttcaaact tatcgatgca gttgttatcg atattccgcc taatgcagtt     240
   ggaaaacttg caaaacttga aggtgttaaa aatgttgaat ttgatgcaca agcacaactg     300
   tatagaggcg gccctcgctg gctggttcct gaaccgacac aacctgcaca agaaattccg     360
   tggggcatcg aacgcgttaa agcaccggat acgtggtcaa tcacgacggg cgcatctaat     420
   ggcgttatcg aagttgcaat tctggataca ggcatcgatt gggatcatcc ggatcttaaa     480
   gataatatcg catggggagt ttctacaatc ggcggagaag tttctacaga tcctgcagat     540
   tggtatgatg gaaatggcca tggaacacat gttgcaggca cgatcgcagc acttaataac     600
   gatattggcg ttgttggcgt tgcacctaat gttgaaatct atgcaattaa agttttagat     660
   gatagaggct caggcgcata tacagatatc gcaatcggca ttgaacaagc attactgggc     720
   ccggatggta tccttgataa agatggagat ggcatcatcg ttggcgatcc ggatgatgat     780
   gcagcagaag ttatctctat gtctctggga ggacctagcg atgatgaata tcttcatgat     840
   atgatcatta aagcatataa ttatggagtt gttattgttg cagcaagcgg aaacgaggca     900
   gcagatcaac cgagctatcc ggcaatctat cctgaagtta ttgcagttgg cgcaacggat     960
   tctagcgatg cagttgcata tttttcaaat cttcaacctg aagttagcgc accgggagtt 1020
   gatgttctgt ctacgtatcc tgatgatacg tatgaaagcc tgtcaggaac gtcaatggca 1080
   acacctcatg tttcaggcgt tgttgcactt attcaagcag catattataa taagtatggc 1140
   aaagttttac cggttggaac atttgatgat atgaatacga atacggttcg cggaatctta 1200
   catgttacgg cagatgatct aggcgatccg ggctgggata tctattatgg ctatggaatc 1260
   gttcgcgcag atctagcagt tcaagcagca ttaggataa 1299
<210> 5
   <211> 432
   <212> PRT
   <213> Artificial Sequence
<220>
   <223> AprE-TnaProl precursor with 3 AA insertion
<400> 5
   Met Arg Ser Lys Lys Leu Trp Ile Ser Leu Leu Phe Ala Leu Thr Leu
   1 5 10 15
   Ile Phe Thr Met Ala Phe Ser Asn Met Ser Ala Gln Ala Ala Gly Lys
   20 25 30
   Lys Gly Ser Asp Thr Val Arg Val Val Ala Leu Ile Asp Arg Asp Asp
   35 40 45
   Phe Arg Gly Asp Ala Val Arg Gly Ile Gly Gly Lys Ile Val Tyr Glu
   50 55 60
   Phe Lys Leu Ile Asp Ala Val Val Ile Asp Ile Pro Pro Asn Ala Val
   65 70 75 80
   Gly Lys Leu Ala Lys Leu Glu Gly Val Lys Asn Val Glu Phe Asp Ala
   85 90 95
   Gln Ala Gln Leu Tyr Arg Gly Gly Pro Arg Trp Leu Val Pro Glu Pro
   100 105 110
   Thr Gln Pro Ala Gln Glu Ile Pro Trp Gly Ile Glu Arg Val Lys Ala
   115 120 125
   Pro Asp Thr Trp Ser Ile Thr Thr Gly Ala Ser Asn Gly Val Ile Glu
   130 135 140
   Val Ala Ile Leu Asp Thr Gly Ile Asp Trp Asp His Pro Asp Leu Lys
   145 150 155 160
   Asp Asn Ile Ala Trp Gly Val Ser Thr Ile Gly Gly Glu Val Ser Thr
                   165                 170                 175
   Asp Pro Ala Asp Trp Tyr Asp Gly Asn Gly His Gly Thr His Val Ala
               180                 185                 190
   Gly Thr Ile Ala Ala Leu Asn Asn Asp Ile Gly Val Val Gly Val Ala
           195                 200                 205
   Pro Asn Val Glu Ile Tyr Ala Ile Lys Val Leu Asp Asp Arg Gly Ser
       210                 215                 220
   Gly Ala Tyr Thr Asp Ile Ala Ile Gly Ile Glu Gln Ala Leu Leu Gly
   225                 230                 235                 240
   Pro Asp Gly Ile Leu Asp Lys Asp Gly Asp Gly Ile Ile Val Gly Asp
                   245                 250                 255
   Pro Asp Asp Asp Ala Ala Glu Val Ile Ser Met Ser Leu Gly Gly Pro
               260                 265                 270
   Ser Asp Asp Glu Tyr Leu His Asp Met Ile Ile Lys Ala Tyr Asn Tyr
           275                 280                 285
   Gly Val Val Ile Val Ala Ala Ser Gly Asn Glu Ala Ala Asp Gln Pro
       290                 295                 300
   Ser Tyr Pro Ala Ile Tyr Pro Glu Val Ile Ala Val Gly Ala Thr Asp
   305                 310                 315                 320
   Ser Ser Asp Ala Val Ala Tyr Phe Ser Asn Leu Gln Pro Glu Val Ser
                   325                 330                 335
   Ala Pro Gly Val Asp Val Leu Ser Thr Tyr Pro Asp Asp Thr Tyr Glu
               340                 345                 350
   Ser Leu Ser Gly Thr Ser Met Ala Thr Pro His Val Ser Gly Val Val
           355                 360                 365
   Ala Leu Ile Gln Ala Ala Tyr Tyr Asn Lys Tyr Gly Lys Val Leu Pro
       370                 375                 380
   Val Gly Thr Phe Asp Asp Met Asn Thr Asn Thr Val Arg Gly Ile Leu
   385                 390                 395                 400
   His Val Thr Ala Asp Asp Leu Gly Asp Pro Gly Trp Asp Ile Tyr Tyr
                   405                 410                 415
   Gly Tyr Gly Ile Val Arg Ala Asp Leu Ala Val Gln Ala Ala Leu Gly
   420 425 430
<210> 6
   <211> 978
   <212> DNA
   <213> Thermococcus nautili
<400> 6
   ctcgttccag agcccacaca gcccgcccag gagattccct ggggaataga gcgcgttaag 60
   gctcccgaca catggagtat aaccaccggc gccagcaacg gcgtcatcga ggttgccata 120
   ctcgacaccg gaatagactg ggaccacccc gacctcaagg acaacatcgc ctggggcgtc 180
   agcaccatag gcggcgaggt tagcaccgac cccgctgact ggtacgacgg caacggccac 240
   ggaacccacg tcgccggaac catcgcggcg cttaacaacg acatcggcgt cgtcggagtc 300
   gccccgaacg ttgaaatcta cgccataaag gttcttgacg acaggggaag tggagcgtac 360
   accgacatag cgataggaat cgagcaggcc cttctcggtc cggacggaat acttgacaag 420
   gacggggacg gaataatcgt tggagacccg gatgatgacg ccgccgaggt cataagcatg 480
   tccctcggag gaccgagcga cgatgagtac ctccacgaca tgataatcaa ggcctacaac 540
   tacggcgtcg tcatcgtcgc cgcgagcggt aacgaggccg ccgaccagcc gagctacccg 600
   gccatctacc cggaggtcat agcggttgga gccaccgact cgagcgacgc cgtagcttac 660
   ttcagcaacc tccagcccga agttagcgcc ccgggcgttg acgtgctcag cacctacccg 720
   gacgacacct acgagagcct cagcggaacc tcaatggcca ccccgcacgt cagcggtgtc 780
   gttgcgctca tccaggcggc ctactacaac aagtacggca aggttctccc ggtcggaacc 840
   ttcgatgaca tgaacaccaa caccgtcagg ggaatcctcc acgttaccgc cgacgacctc 900
   ggagaccccg gatgggacat ctactacggc tacggaatag tcagggccga cctggcagtc 960
   caggcggctc ttggctga 978
<210> 7
   <211> 1203
   <212> DNA
   <213> Thermococcus nautili
<400> 7
   aagggcagcg acacggttag ggtggttgcc cttatagaca gggacgactt caggggagat      60
   gcagtaaggg gcataggcgg taaaatcgtc tacgagttca agctgattga tgccgtcgtc     120
   attgacattc ctcccaacgc ggttggaaag ctcgccaagc ttgagggagt taagaatgtc     180
   gagttcgacg cccaggccca gctctacagg ggagggccga ggtggctcgt tccagagccc     240
   acacagcccg cccaggagat tccctgggga atagagcgcg ttaaggctcc cgacacatgg     300
   agtataacca ccggcgccag caacggcgtc atcgaggttg ccatactcga caccggaata     360
   gactgggacc accccgacct caaggacaac atcgcctggg gcgtcagcac cataggcggc     420
   gaggttagca ccgaccccgc tgactggtac gacggcaacg gccacggaac ccacgtcgcc      480
   ggaaccatcg cggcgcttaa caacgacatc ggcgtcgtcg gagtcgcccc gaacgttgaa     540
   atctacgcca taaaggttct tgacgacagg ggaagtggag cgtacaccga catagcgata     600
   ggaatcgagc aggcccttct cggtccggac ggaatacttg acaaggacgg ggacggaata     660
   atcgttggag acccggatga tgacgccgcc gaggtcataa gcatgtccct cggaggaccg     720
   agcgacgatg agtacctcca cgacatgata atcaaggcct acaactacgg cgtcgtcatc     780
   gtcgccgcga gcggtaacga ggccgccgac cagccgagct acccggccat ctacccggag     840
   gtcatagcgg ttggagccac cgactcgagc gacgccgtag cttacttcag caacctccag     900
   cccgaagtta gcgccccggg cgttgacgtg ctcagcacct acccggacga cacctacgag     960
   agcctcagcg gaacctcaat ggccaccccg cacgtcagcg gtgtcgttgc gctcatccag    1020
   gcggcctact acaacaagta cggcaaggtt ctcccggtcg gaaccttcga tgacatgaac    1080
   accaacaccg tcaggggaat cctccacgtt accgccgacg acctcggaga ccccggatgg    1140
   gacatctact acggctacgg aatagtcagg gccgacctgg cagtccaggc ggctcttggc    1200
   tga                                                                  1203
<210> 8
   <211> 400
   <212> PRT
   <213> Thermococcus nautili
<400> 8
   Lys Gly Ser Asp Thr Val Arg Val Val Ala Leu Ile Asp Arg Asp Asp
   1               5                   10                  15
   Phe Arg Gly Asp Ala Val Arg Gly Ile Gly Gly Lys Ile Val Tyr Glu
               20                  25                  30
   Phe Lys Leu Ile Asp Ala Val Val Ile Asp Ile Pro Pro Asn Ala Val
           35                  40                  45
   Gly Lys Leu Ala Lys Leu Glu Gly Val Lys Asn Val Glu Phe Asp Ala
       50                  55                  60
   Gln Ala Gln Leu Tyr Arg Gly Gly Pro Arg Trp Leu Val Pro Glu Pro
   65                  70                  75                  80
   Thr Gln Pro Ala Gln Glu Ile Pro Trp Gly Ile Glu Arg Val Lys Ala
                   85                  90                  95
   Pro Asp Thr Trp Ser Ile Thr Thr Gly Ala Ser Asn Gly Val Ile Glu
               100                 105                 110
   Val Ala Ile Leu Asp Thr Gly Ile Asp Trp Asp His Pro Asp Leu Lys
           115                 120                 125
   Asp Asn Ile Ala Trp Gly Val Ser Thr Ile Gly Gly Glu Val Ser Thr
       130                 135                 140
   Asp Pro Ala Asp Trp Tyr Asp Gly Asn Gly His Gly Thr His Val Ala
   145                 150                 155                 160
   Gly Thr Ile Ala Ala Leu Asn Asn Asp Ile Gly Val Val Gly Val Ala
                   165                 170                 175
   Pro Asn Val Glu Ile Tyr Ala Ile Lys Val Leu Asp Asp Arg Gly Ser
               180                 185                 190
   Gly Ala Tyr Thr Asp Ile Ala Ile Gly Ile Glu Gln Ala Leu Leu Gly
           195                 200                 205
   Pro Asp Gly Ile Leu Asp Lys Asp Gly Asp Gly Ile Ile Val Gly Asp
       210                 215                 220
   Pro Asp Asp Asp Ala Ala Glu Val Ile Ser Met Ser Leu Gly Gly Pro
   225                 230                 235                 240
   Ser Asp Asp Glu Tyr Leu His Asp Met Ile Ile Lys Ala Tyr Asn Tyr
                   245                 250                 255
   Gly Val Val Ile Val Ala Ala Ser Gly Asn Glu Ala Ala Asp Gln Pro
               260                 265                 270
   Ser Tyr Pro Ala Ile Tyr Pro Glu Val Ile Ala Val Gly Ala Thr Asp
           275                 280                 285
   Ser Ser Asp Ala Val Ala Tyr Phe Ser Asn Leu Gln Pro Glu Val Ser
       290                 295                 300
   Ala Pro Gly Val Asp Val Leu Ser Thr Tyr Pro Asp Asp Thr Tyr Glu
   305                 310                 315                 320
   Ser Leu Ser Gly Thr Ser Met Ala Thr Pro His Val Ser Gly Val Val
                   325                 330                 335
   Ala Leu Ile Gln Ala Ala Tyr Tyr Asn Lys Tyr Gly Lys Val Leu Pro
               340                 345                 350
   Val Gly Thr Phe Asp Asp Met Asn Thr Asn Thr Val Arg Gly Ile Leu
   355 360 365
   His Val Thr Ala Asp Asp Leu Gly Asp Pro Gly Trp Asp Ile Tyr Tyr
   370 375 380
   Gly Tyr Gly Ile Val Arg Ala Asp Leu Ala Val Gln Ala Ala Leu Gly
   385 390 395 400
<210> 9
   <211> 78
   <212> DNA
   <213> Thermococcus nautili
<400> 9
   atggagcgga gggtagtagt tggcggactg ctcgtcctgg cactggttct gtccctcttt 60
   gctgtgccaa ccgctgcc 78
<210> 10
   <211> 26
   <212> PRT
   <213> Thermococcus nautili
<400> 10
   Met Glu Arg Arg Val Val Val Gly Gly Leu Leu Val Leu Ala Leu Val
   1 5 10 15
   Leu Ser Leu Phe Ala Val Pro Thr Ala Ala
   20 25
<210> 11
   <211> 225
   <212> DNA
   <213> Thermococcus nautili
<400> 11
   aagggcagcg acacggttag ggtggttgcc cttatagaca gggacgactt caggggagat 60
   gcagtaaggg gcataggcgg taaaatcgtc tacgagttca agctgattga tgccgtcgtc 120
   attgacattc ctcccaacgc ggttggaaag ctcgccaagc ttgagggagt taagaatgtc 180
   gagttcgacg cccaggccca gctctacagg ggagggccga ggtgg 225
<210> 12
   <211> 75
   <212> PRT
   <213> Thermococcus nautili
<400> 12
   Lys Gly Ser Asp Thr Val Arg Val Val Ala Leu Ile Asp Arg Asp Asp
   1               5                   10                  15
   Phe Arg Gly Asp Ala Val Arg Gly Ile Gly Gly Lys Ile Val Tyr Glu
               20                  25                  30
   Phe Lys Leu Ile Asp Ala Val Val Ile Asp Ile Pro Pro Asn Ala Val
           35                  40                  45
   Gly Lys Leu Ala Lys Leu Glu Gly Val Lys Asn Val Glu Phe Asp Ala
       50                  55                  60
   Gln Ala Gln Leu Tyr Arg Gly Gly Pro Arg Trp
   65                  70                  75
<210> 13
   <211> 1290
   <212> DNA
   <213> Artificial Sequence
<220>
   <223> AprE-TnaProl gene
<400> 13
   gtgagaagca aaaaattgtg gatcagcttg ttgtttgcgt taacgttaat ctttacgatg      60
   gcgttcagca acatgagcgc gcaggctaaa ggctcagata cggttcgcgt tgttgcactt     120
   atcgatagag atgattttcg cggcgatgca gttagaggaa tcggcggcaa aatcgtttat     180
   gagttcaaac ttatcgatgc agttgttatc gatattccgc ctaatgcagt tggaaaactt     240
   gcaaaacttg aaggtgttaa aaatgttgaa tttgatgcac aagcacaact gtatagaggc     300
   ggccctcgct ggctggttcc tgaaccgaca caacctgcac aagaaattcc gtggggcatc     360
   gaacgcgtta aagcaccgga tacgtggtca atcacgacgg gcgcatctaa tggcgttatc     420
   gaagttgcaa ttctggatac aggcatcgat tgggatcatc cggatcttaa agataatatc     480
   gcatggggag tttctacaat cggcggagaa gtttctacag atcctgcaga ttggtatgat     540
   ggaaatggcc atggaacaca tgttgcaggc acgatcgcag cacttaataa cgatattggc     600
   gttgttggcg ttgcacctaa tgttgaaatc tatgcaatta aagttttaga tgatagaggc     660
   tcaggcgcat atacagatat cgcaatcggc attgaacaag cattactggg cccggatggt     720
   atccttgata aagatggaga tggcatcatc gttggcgatc cggatgatga tgcagcagaa     780
   gttatctcta tgtctctggg aggacctagc gatgatgaat atcttcatga tatgatcatt     840
   aaagcatata attatggagt tgttattgtt gcagcaagcg gaaacgaggc agcagatcaa     900
   ccgagctatc cggcaatcta tcctgaagtt attgcagttg gcgcaacgga ttctagcgat     960
   gcagttgcat atttttcaaa tcttcaacct gaagttagcg caccgggagt tgatgttctg    1020
   tctacgtatc ctgatgatac gtatgaaagc ctgtcaggaa cgtcaatggc aacacctcat    1080
   gtttcaggcg ttgttgcact tattcaagca gcatattata ataagtatgg caaagtttta    1140
   ccggttggaa catttgatga tatgaatacg aatacggttc gcggaatctt acatgttacg    1200
   gcagatgatc taggcgatcc gggctgggat atctattatg gctatggaat cgttcgcgca    1260
   gatctagcag ttcaagcagc attaggataa                                      1290
<210> 14
   <211> 429
   <212> PRT
   <213> Artificial Sequence
<220>
   <223> AprE-TnaProl precursor
<400> 14
   Met Arg Ser Lys Lys Leu Trp Ile Ser Leu Leu Phe Ala Leu Thr Leu
   1               5                   10                  15
   Ile Phe Thr Met Ala Phe Ser Asn Met Ser Ala Gln Ala Lys Gly Ser
               20                  25                  30
   Asp Thr Val Arg Val Val Ala Leu Ile Asp Arg Asp Asp Phe Arg Gly
           35                  40                  45
   Asp Ala Val Arg Gly Ile Gly Gly Lys Ile Val Tyr Glu Phe Lys Leu
       50                  55                  60
   Ile Asp Ala Val Val Ile Asp Ile Pro Pro Asn Ala Val Gly Lys Leu
   65                  70                  75                  80
   Ala Lys Leu Glu Gly Val Lys Asn Val Glu Phe Asp Ala Gln Ala Gln
                   85                  90                  95
   Leu Tyr Arg Gly Gly Pro Arg Trp Leu Val Pro Glu Pro Thr Gln Pro
               100                 105                 110
   Ala Gln Glu Ile Pro Trp Gly Ile Glu Arg Val Lys Ala Pro Asp Thr
           115                 120                 125
   Trp Ser Ile Thr Thr Gly Ala Ser Asn Gly Val Ile Glu Val Ala Ile
       130                 135                 140
   Leu Asp Thr Gly Ile Asp Trp Asp His Pro Asp Leu Lys Asp Asn Ile
   145                 150                 155                 160
   Ala Trp Gly Val Ser Thr Ile Gly Gly Glu Val Ser Thr Asp Pro Ala
                   165                 170                 175
   Asp Trp Tyr Asp Gly Asn Gly His Gly Thr His Val Ala Gly Thr Ile
               180                 185                 190
   Ala Ala Leu Asn Asn Asp Ile Gly Val Val Gly Val Ala Pro Asn Val
           195                 200                 205
   Glu Ile Tyr Ala Ile Lys Val Leu Asp Asp Arg Gly Ser Gly Ala Tyr
       210                 215                 220
   Thr Asp Ile Ala Ile Gly Ile Glu Gln Ala Leu Leu Gly Pro Asp Gly
   225                 230                 235                 240
   Ile Leu Asp Lys Asp Gly Asp Gly Ile Ile Val Gly Asp Pro Asp Asp
                   245                 250                 255
   Asp Ala Ala Glu Val Ile Ser Met Ser Leu Gly Gly Pro Ser Asp Asp
               260                 265                 270
   Glu Tyr Leu His Asp Met Ile Ile Lys Ala Tyr Asn Tyr Gly Val Val
           275                 280                 285
   Ile Val Ala Ala Ser Gly Asn Glu Ala Ala Asp Gln Pro Ser Tyr Pro
       290                 295                 300
   Ala Ile Tyr Pro Glu Val Ile Ala Val Gly Ala Thr Asp Ser Ser Asp
   305                 310                 315                 320
   Ala Val Ala Tyr Phe Ser Asn Leu Gln Pro Glu Val Ser Ala Pro Gly
                   325                 330                 335
   Val Asp Val Leu Ser Thr Tyr Pro Asp Asp Thr Tyr Glu Ser Leu Ser
               340                 345                 350
   Gly Thr Ser Met Ala Thr Pro His Val Ser Gly Val Val Ala Leu Ile
           355                 360                 365
   Gln Ala Ala Tyr Tyr Asn Lys Tyr Gly Lys Val Leu Pro Val Gly Thr
       370                 375                 380
   Phe Asp Asp Met Asn Thr Asn Thr Val Arg Gly Ile Leu His Val Thr
   385                 390                 395                 400
   Ala Asp Asp Leu Gly Asp Pro Gly Trp Asp Ile Tyr Tyr Gly Tyr Gly
                   405                 410                 415
   Ile Val Arg Ala Asp Leu Ala Val Gln Ala Ala Leu Gly
               420                 425
<210> 15
   <211> 87
   <212> DNA
   <213> Bacillus subtilis
<400> 15
   gtgagaagca aaaaattgtg gatcagcttg ttgtttgcgt taacgttaat ctttacgatg      60
   gcgttcagca acatgagcgc gcaggct                                          87
<210> 16
   <211> 29
   <212> PRT
   <213> Bacillus subtilis
<400> 16
   Met Arg Ser Lys Lys Leu Trp Ile Ser Leu Leu Phe Ala Leu Thr Leu
   1               5                   10                  15
   Ile Phe Thr Met Ala Phe Ser Asn Met Ser Ala Gln Ala
               20                  25
<210> 17
   <211> 4
   <212> PRT
   <213> Artificial Sequence
<220>
   <223> pNA-AAPF Amino Acid Sequence
<400> 17
   Ala Ala Pro Phe
   1